# EUROPEAN PATENT APPLICATION

(11) **EP 4 624 576 A1**
(43) Date of publication of application: **01.10.2025**
(21) Application number: 23894673.5
(22) Date of filing: 24.11.2023
(51) Int. Cl.: C12N 15/115, A61K 31/712, A61K 31/713, A61P 25/00, A61P 27/00, A61P 29/00, A61P 35/00, A61P 37/00, C07D 405/04, C07D 409/02, C07D 409/14, C07D 471/04

(54) **NUCLEIC ACID APTAMER**

(30) Priority: 25.11.2022 SG 10202260204R; 25.11.2022 SG 10202260206Y; 23.03.2023 SG 10202300790X
(71) Applicant: Xenolis Pte. Ltd., Singapore 118259 (SG)
(72) Inventor: HIRAO, Michiko, Singapore 118259 (SG); MATSUNAGA, Ken-ichiro, Singapore 118259 (SG); TAN, Hui Pen, Singapore 118259 (SG); HIRAO, Ichiro, Singapore 118259 (SG)
(74) Representative: D Young & Co LLP
(86) International application number: PCT/JP2023/042275
(87) International publication number: WO 2024/111671

(57) **Abstract**

An object of the present invention is to provide a method for enhancing the affinity of a nucleic acid aptamer comprising 7-(2-thienyl)imidazo[4,5-b]pyridine (Ds base) to a target protein. Provided is a nucleic acid aptamer comprising an unnatural base(s) represented by the general formula (I) and/or the general formula (II).

## Description

### Technical Field

The present invention relates to: an unnatural base; a nucleoside and a nucleotide that each comprise the unnatural base; a nucleic acid aptamer comprising the unnatural base; a pharmaceutical composition; and the like.

### Background Art

A nucleic acid aptamer is a nucleic acid strand that binds specifically to a target, such as a small molecule, a protein, or a cell. A nucleic acid aptamer is usually generated from a nucleic acid library having a randomized sequence, through repetitions of selection and amplification, using SELEX (systematic evolution of ligands by exponential enrichment).

A nucleic acid aptamer generated using SELEX undergoes base sequencing, and then is chemically synthesized for mass production and modification. The nucleic acid aptamer can be provided at high purity, and thus, can be used as antibody substitute with excellent quality control.

In general, however, a nucleic acid is more hydrophilic than a protein-based antibody. Because of this, the hydrophobic interaction of a nucleic acid aptamer with a target protein can be weaker than the hydrophobic interaction of an antibody. Accordingly, a conventional nucleic acid aptamer has difficulty in providing a stronger binding force than an antibody, and has been considered to be insufficient for practical use.

To solve the above-described problem of a nucleic acid aptamer, the present inventors have developed ExSELEX (genetic alphabet Expansion for SELEX) as a new aptamer-generating method that enables the improvement of the hydrophobic interaction with a target protein by introducing the hydrophobic unnatural base 7-(2-thienyl)imidazo[4,5-b]pyridine (herein referred to as "Ds" or a "Ds base") as a fifth base into a nucleic acid aptamer (Non-Patent Literature 1). The Ds base is base-paired with 2-nitro-4-propynylpyrrole modified with a diol (the latter base is herein referred to as "Px" or a "Px base"). Accordingly, a Ds-Px base pair formed by base pairing of a Ds base and a Px base functions with high fidelity as a third base pair in PCR amplification (Non-Patent Literature 1 to 6). A nucleic acid aptamer obtained using ExSELEX can achieve high affinity with sub-nanomolar K_{D} value on the basis of comprising a Ds base (K_{D} value represents affinity to a target protein).

### Citation List

### Non-Patent Literature

Non-Patent Literature 1: Kimoto, M., et al., Nat. Biotechnol., 2013, 31: 453-457.
Non-Patent Literature 2: Kimoto, M., et al., Nucleic Acids Res., 2009, 37: e14.
Non-Patent Literature 3: Yamashige, R., et al., Nucleic Acids Res., 2012, 40:2793-2806.
Non-Patent Literature 4: Matsunaga, K., et al., J. Am. Chem. Soc., 2017, 139: 324-334.
Non-Patent Literature 5: Matsunaga, K., et al., Nucleic Acids Res., 2021, 49: 11407-11424.
Non-Patent Literature 6: Kimoto, M. and Hirao, I., Chem. Soc, Rev., 2020, 49: 7602-7626.

### Summary of Invention

### Technical Problem

An object of the present invention is to provide a method for enhancing the affinity of a Ds-containing nucleic acid aptamer to a target protein.

### Solution to Problem

In an ExSELEX method developed by the present inventors in the past, a Ds base is base-paired with a Px base, and a Ds-Px base pair functions with high fidelity as a third base pair in PCR amplification. ExSELEX is a method based on this Ds-Px base pair, and thus, modification of a Ds base, having a possibility of affecting a base pair with a Px base, is not considered hitherto.

However, there is a possibility that, except in a step in ExSELEX, the Ds base is not the best hydrophobic artificial base that affords high affinity. For example, there are possibilities of a hydrophobic artificial base that further reinforces hydrophobic interaction , from the viewpoints of the van der Waals force, the stacking of a base, polarity, and the like. Accordingly, there is a possibility that the affinity of a nucleic acid aptamer comprising a Ds base is improved by replacing the Ds base with a new artificial base (hereinafter often referred to as a "Ds alternative").

To solve the above-described problem, the present inventors have created a group of new artificial bases (herein collectively referred to as "Ds alternatives") by substituting a 1-deazapurine portion out of two modules-the 1-deazapurine(imidazo[4,5-*b*]pyridine) portion and a thienyl side chain that constitute a Ds base-with pyrrolo[2,3-*b*]pyridine, purine, indole, benzo[*d*]imidazole, or pyrrolo[2,3-*d*]pyrimidine, and substituting the thienyl side chain with furanyl, imidazolyl, thiazolyl, pyridazinyl, or a modified structure thereof (for example, a methylthienyl or bithiophenyl portion). The present inventors have come to complete the present invention through the discovery that substituting, with a Ds alternative, the Ds base of a nucleic acid aptamer developed using ExSELEX in the past allows for generation of a new nucleic acid aptamer that is improved in affinity.

The present invention is based on the new discovery, and provides the following.
[1] A nucleic acid aptamer comprising an unnatural base(s) represented by the following general formula(s) (I) and/or (II): 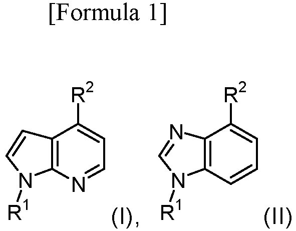 wherein R¹ represents a sugar portion in a nucleoside or a C¹ or C² alkyl group, and R² represents any of the following formulas (III) to (IX): 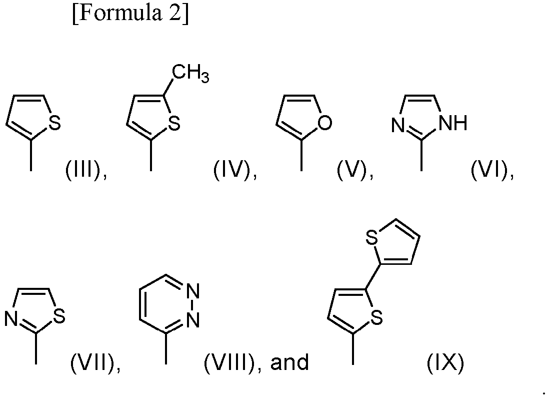
[2] The nucleic acid aptamer of [1], comprising two said unnatural bases.
[3] The nucleic acid aptamer of [1] or [2], comprising the unnatural base(s) represented by said general formulas (I) and (II).
[4] The nucleic acid aptamer of any one of [1] to [3], wherein said unnatural base is any of the unnatural bases represented by the following formulas (X) to (XVII) and formulas (XXI) to (XXII): 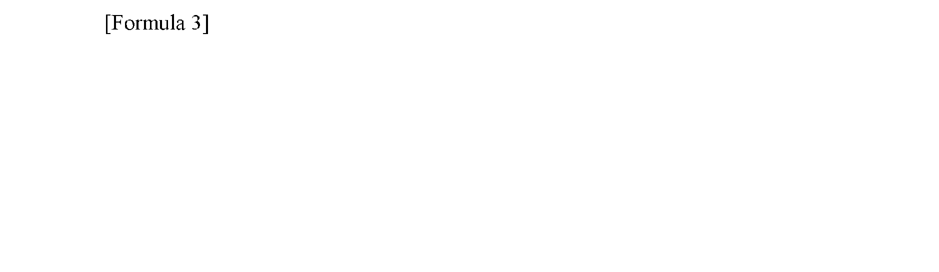 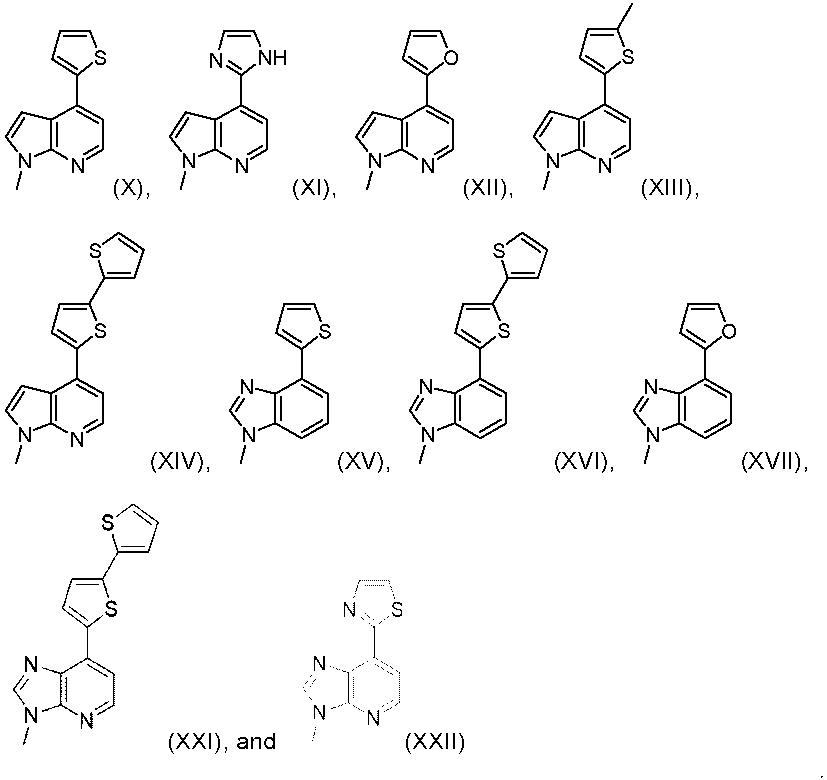
[5] The nucleic acid aptamer of any one of [1] to [4], comprising an unnatural base represented by the following formula (XVIII): 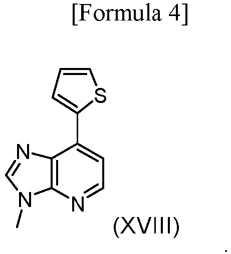
[6] The nucleic acid aptamer of any one of [1] to [5], which is a DNA aptamer.
[7] The nucleic acid aptamer of [1],
   (1) comprising the base sequence of SEQ ID NO: 1 or 2, wherein the nucleic acid aptamer binds to von Willebrand factor (vWF protein),
   (2) comprising the base sequence of SEQ ID NO: 15 or 16, wherein the nucleic acid aptamer binds to Interferon gamma (IFNγ),
   (3) comprising the base sequence of SEQ ID NO: 26, wherein the nucleic acid aptamer binds to vascular endothelial growth factor (VEGF),
   (4) comprising the base sequence of SEQ ID NO: 27 or 28, wherein the nucleic acid aptamer binds to Dengue Virus NS1 Protein Serotype 1 (DEN1 protein), or
   (5) comprising the base sequence of SEQ ID NO: 38 or 39, wherein the nucleic acid aptamer binds to Dengue Virus NS1 Protein Serotype 3 (DEN3 protein), and
   wherein, in said base sequence, at least one of the bases represented by n is an unnatural base represented by said general formula(s) (I) and/or (II).
[8] The nucleic acid aptamer of [7], comprising two said unnatural bases as the bases represented by n in said base sequence.
[9] The nucleic acid aptamer of [7] or [8], comprising the unnatural base(s) represented by said general formulas (I) and (II).
[10] The nucleic acid aptamer of [7], comprising 7-(2-thienyl)-3*H*-imidazo[4,5-*b*]pyridine-3-yl as a base represented by n in said base sequence.
[11] The nucleic acid aptamer of [7], wherein said unnatural base is any of the bases represented by the following formulas (X) to (XVII) and formulas (XXI) to (XXII): 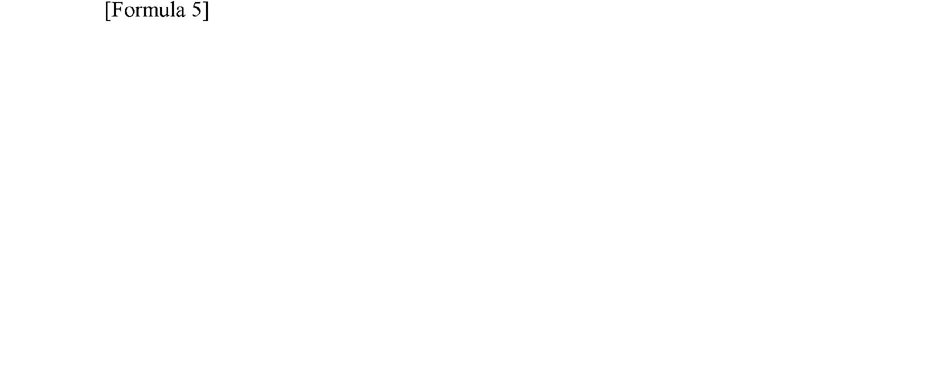 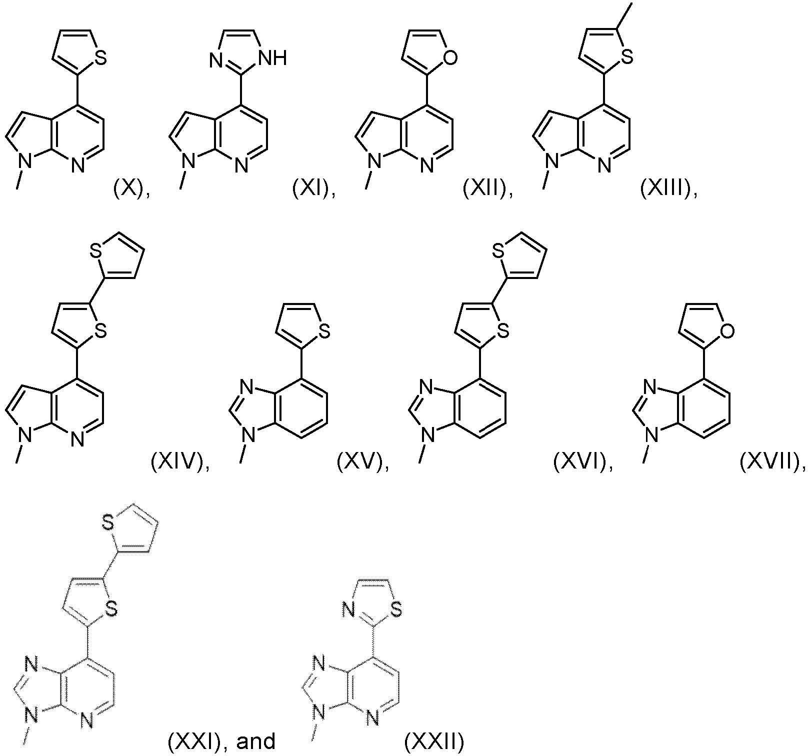
[12] The nucleic acid aptamer of [7], comprising an unnatural base represented by the following formula (XVIII): 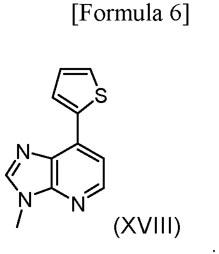
[13] The nucleic acid aptamer of [7], which is a DNA aptamer.
[14] A pharmaceutical composition comprising the nucleic acid aptamer of [7].
[15] The pharmaceutical composition of [14], comprising the nucleic acid aptamer of said (1) as an active ingredient, wherein said pharmaceutical composition is for treating and/or preventing a disease selected from the group consisting of thrombosis, thrombotic thrombocytopenic purpura, intracranial embolism, brain embolism, carotid artery stenosis, thrombotic microangiopathy, and acute myocardial infarction.
[16] The pharmaceutical composition of [14], comprising the nucleic acid aptamer of said (2) as an active ingredient, wherein said pharmaceutical composition is for inhibiting the function of Interferon gamma.
[17] The pharmaceutical composition of [14], comprising the nucleic acid aptamer of said (3) as an active ingredient, wherein said pharmaceutical composition is for inhibiting the function of vascular endothelial growth factor.
[18] The pharmaceutical composition of [14], comprising the nucleic acid aptamer of said (4) and/or the nucleic acid aptamer of said (5) as an active ingredient(s), wherein said pharmaceutical composition is for treating and/or preventing dengue fever.
[19] An unnatural base represented by any of the following formulas (XI) to (XIII) and formulas (XV) to (XVII): 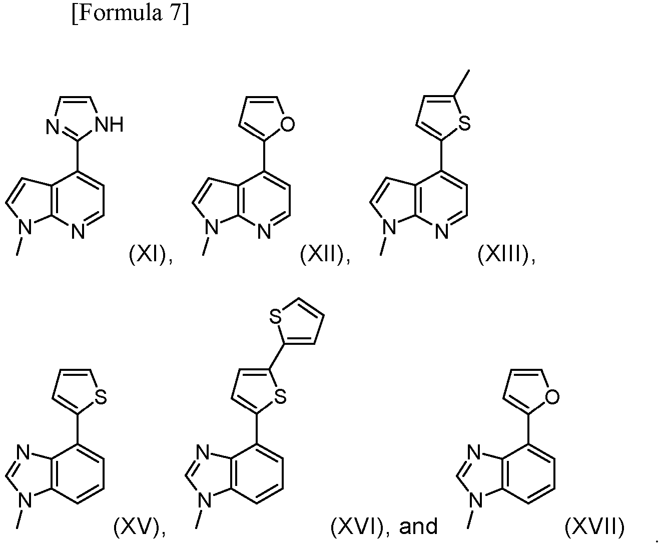
[20] A nucleoside comprising the unnatural base of [19].
[21] A nucleotide comprising the unnatural base of [19].

The present specification encompasses the disclosure of Singapore Patent Application No. 10202260206Y, Singapore Patent Application No. 10202260204R, and Singapore Patent Application No. 10202300790X that serve as the basis of the priority of the present application. Note that SEQ ID NOs: 1 to 54 herein are the same as SEQ ID NOs: 1 to 54 in Singapore Patent Application No. 10202260206Y and Singapore Patent Application No. 10202260204R. In addition, SEQ ID NOs: 55 to 141 herein are the same as SEQ ID NOs: 1 to 87 in Singapore Patent Application No. 10202300790X.

### Advantageous Effects of Invention

The present invention provides a method for enhancing the affinity of a nucleic acid aptamer comprising a Ds base to a target protein.

### Brief Description of Drawing

[Figure 1] Figure 1 shows: a Ds base that is a hydrophobic unnatural base; a Px base to base-pair with a Ds base; and four kinds of natural DNA bases.
[Figure 2] Figure 2 shows various Ds alternatives resulting from substituting the 1-deazapurine portion with pyrrolo[2,3-*b*]pyridine, purine, indole, benzo[*d*]imidazole, or pyrrolo[2,3-*d*]pyrimidine, and substituting the thienyl side chain with furanyl, imidazolyl, thiazolyl, pyridazinyl, and a modified structure thereof (for example, a methylthienyl or bithiophenyl portion).
[Figure 3] Figure 3 shows the examples of an anti-VEGF aptamer, anti-IFNγ aptamer, and anti-vWF aptamer.
[Figure 4] Figure 4 shows the examples of an anti-DEN1-NS1 aptamer, anti-DEN2-NS1 aptamer, anti-DEN3-NS1 aptamer, and anti-DEN4-NS1 aptamer.
[Figure 5] Figure 5 shows the results obtained by analyzing the affinity of the Ds aptamer (AptIFNγ-DsDs) and 11 kinds of UB aptamers (AptIFNγ-BsBs, AptIFNγ-IsIs, AptIFNγ-YsYs, AptIFNγ-YiYi, AptIFNγ-DoDo, AptIFNγ-DpDp, AptIFNγ-DssDss, AptIFNγ-DtDt, AptIFNγ-YoYo, AptIFNγ-PsPs, and AptIFNγ-EsEs) to IFNγ, using EMSA. Figure 5A shows gels stained with SYBR Gold. Figure 5B shows the relative binding ratios (%) of the nucleic acid aptamers bound to the target.
[Figure 6] Figure 6 shows the results obtained by analyzing the affinity of the Ds aptamer (AptvWF-DsDs) and 11 kinds of UB aptamers (AptvWF-BsBs, AptvWF-IsIs, AptvWF-YsYs, AptvWF-YiYi, AptvWF-DoDo, AptvWF-DpDp, AptvWF-DssDss, AptvWF-DtDt, AptvWF-YoYo, AptvWF-PsPs, and AptvWF-EsEs) to vWF, using EMSA. Figure 6A shows gels stained with SYBR Gold. Figure 6B shows the relative binding ratios (%) of the nucleic acid aptamers bound to the target.
[Figure 7] Figure 7 shows the results obtained by analyzing the affinity of the Ds aptamer (AptvWF-DsDs) and 6 kinds of UB aptamers (AptvWF-YsYs, AptvWF-BsYs, AptvWF-BsYo, AptvWF-YsBs, AptvWF-BsBs, and AptvWF-YoYo) to vWF, using EMSA. Figure 7A shows gel stained with SYBR Gold. Figure 7B shows the relative binding ratios (%) of the nucleic acid aptamers bound to the target.
[Figure 8] Figure 8 shows the results obtained by analyzing the affinity of the Ds aptamer (AptD1-DsDs) and 11 kinds of UB aptamers (AptD1-BsBs, AptD1-IsIs, AptD1-YsYs, AptD1-YiYi, AptD1-DoDo, AptD1-DpDp, AptD1-DssDss, AptD1-DtDt, AptD1-YoYo, AptD1-PsPs, and AptD1-EsEs) to DEN1-NS1, using EMSA. Figure 8A shows gels stained with SYBR Gold. Figure 8B shows the relative binding ratios (%) of the nucleic acid aptamers bound to the target.
[Figure 9] Figure 9 shows the results obtained by analyzing the affinity of the Ds aptamer (AptD1-DsDs) and 9 kinds of UB aptamers (AptD1-DsYs, AptD1-YsDs, AptD1-YsYs, AptD1-DsBs, AptD1-BsDs, AptD1-BsBs, AptD1-DsDt, AptD1-DtDs, and AptD1-DtDt) to DEN1-NS1, using EMSA. Figure 9A shows gel stained with SYBR Gold. Figure 9B shows the relative binding ratios (%) of the nucleic acid aptamers bound to the target.
[Figure 10] Figure 10 shows the results obtained by analyzing the affinity of the Ds aptamer (AptD1-DsDs) and 7 kinds of UB aptamers (AptD1-BsBs, AptD1-YsYs, AptD1-YoYo, AptD1-YsBs, AptD1-YoBs, AptD1-YsmBs, and AptD1-BsYs) to DEN1-NS1, using EMSA. Figure 10A shows gel stained with SYBR Gold. Figure 10B shows the relative binding ratios (%) of the nucleic acid aptamers bound to the target.
[Figure 11] Figure 11 shows the results obtained by analyzing the affinity of the Ds aptamer (AptD3-DsDs) and 11 kinds of UB aptamers (AptD3-BsBs, AptD3-IsIs, AptD3-YsYs, AptD3-YiYi, AptD3-DoDo, AptD3-DpDp, AptD3-DssDss, AptD3-DtDt, AptD3-YoYo, AptD3-PsPs, and AptD3-EsEs) to DEN3-NS1, using EMSA. Figure 11A shows gels stained with SYBR Gold. Figure 11B shows the relative binding ratios (%) of the nucleic acid aptamers bound to the target.
[Figure 12] Figure 12 shows the results obtained by analyzing the affinity of the Ds aptamer (AptD3-DsDs) and 7 kinds of UB aptamers (AptD3-YsYs, AptD3-YsDss, AptD3-DssYs, AptD3-DssDss, AptD3-YoYo, AptD3-YssYss, and AptD3-YsmYsm) to DEN3-NS1, using EMSA. Figure 12A shows gel stained with SYBR Gold. Figure 12B shows the relative binding ratios (%) of the nucleic acid aptamers bound to the target.
[Figure 13] Figure 13 shows the results obtained by analyzing the affinity of the Ds aptamer (AptD3-DsDs) and 8 kinds of UB aptamers (AptD3-YssYss, AptD3-YssYs, AptD3-YssYo, AptD3-YssYsm, AptD3-YsmYss, AptD3-YsmYsm, AptD3-YsYs, and AptD3-YoYo) to DEN3-NS1, using EMSA. Figure 13A shows gel stained with SYBR Gold. Figure 13B shows the relative binding ratios (%) of the nucleic acid aptamers bound to the target.
[Figure 14] Figure 14 schematically shows a method for measuring the detection sensitivity of an anti-IFNγ antibody and an IFNγ-targeting nucleic acid aptamer to IFNγ, using ELISA.
Figure 14A shows ELISA intended for an anti-IFNγ antibody. Figure 14B shows ELISA intended for an IFNγ-targeting nucleic acid aptamer.
[Figure 15] Figure 15 shows the results obtained by measuring the detection sensitivity of an anti-IFNγ antibody and IFNγ-targeting nucleic acid aptamers to IFNγ, using ELISA. Figure 15A shows the results obtained by measuring the detection sensitivity of an anti-IFNγ antibody, AptIFNγ-DsDs, AptIFNγ-YsYs, and AptIFNγ-YoYo aptamer to IFNγ. The abscissa shows the concentration of an antibody used to fix IFNγ. Figure 15B shows the signal intensities obtained by using an antibody and a nucleic acid aptamer at various concentrations in ELISA.
[Figure 16] Figure 16 shows the results obtained by analyzing the affinity of 7 kinds of nucleic acid aptamers (AptIFNγ-1, AptIFNγ-2, AptIFNγ-3, AptIFNγ-4, AptIFNγ-5, AptIFNγ-6, and Bio-AptIFNγ-3) to human IFNγ, using EMSA.
[Figure 17] Figure 17 shows the results obtained by analyzing the affinity of 5 kinds of nucleic acid aptamers (AptIFNγ-1, AptIFNγ-3, and Bio-AptIFNγ-3; AptDs-IFNγ and Bio-AptDs-IFNγ as comparative controls) to non-glycosylated IFNγ and glycosylated IFNγ, using EMSA.
[Figure 18] Figure 18 shows the results obtained by analyzing the affinity of 10 kinds of nucleic acid aptamers (XL2-01a, XL2-01b, XL2-01c, XL2-01d, XL2-01e, XL2-01h, XL2-01i, XL2-01j, XL2-01k, and XL2-01l) to non-glycosylated IFNγ and glycosylated IFNγ, using EMSA.
[Figure 19] Figure 19 shows the results obtained by analyzing the affinity of 3 kinds of nucleic acid aptamers (Bio-XL2-01a, Bio-XL2-01i, and Bio-AptDs-IFNγ) to glycosylated IFNγ, using ELONA.
[Figure 20] Figure 20 shows the results obtained by analyzing the affinity of 3 kinds of nucleic acid aptamers (Bio-XL2-01a, Bio-XL2-01i, and Bio-AptDs-IFNγ) to glycosylated IFNγ, using ELONA.
[Figure 21] Figure 21 shows the results obtained by analyzing the affinity of 9 kinds of nucleic acid aptamers (Thr101, Thr102, Thr104, Thr106, Thr201, Thr202, Thr203, Thr204, and Thr205) to human Thrombin, using EMSA.
[Figure 22] Figure 22 shows the results obtained by analyzing the affinity of 7 kinds of nucleic acid aptamers (Thr104, Bio-Thr104b, Thr204, Bio-Thr204, Thr205, Bio-Thr205, and BioMH-RE31) to human Thrombin using EMSA.
[Figure 23] Figure 23 shows the results obtained by analyzing the affinity of 4 kinds of nucleic acid aptamers (Bio-Thr104b, Bio-Thr204, Bio-Thr205, and BioMH-RE31) to human Thrombin using ELONA.
[Figure 24] Figure 24 shows the results obtained by analyzing the affinity of 6 kinds of nucleic acid aptamers (Thr204-DD, Thr204-BB, Thr204-BY, Thr204-YB, Thr204-YY, and Thr204-AA) to human Thrombin, using EMSA.
[Figure 25] Figure 25 shows the results obtained by analyzing the affinity of 6 kinds of nucleic acid aptamers (Bio-HMGB1-301-DD, Bio-HMGB1-301-BB, Bio-HMGB1-301-BY, Bio-HMGB1-301-YB, Bio-HMGB1-301-YY, and Bio-HMGB1-301-AA) to human HMGB1 protein, using EMSA.
[Figure 26] Figure 26 shows the results obtained by analyzing the affinity of 6 kinds of nucleic acid aptamers (IFNg-201AAD, IFNg-201AAY, IFNg-201AAB, IFNg-201ADD, IFNg-201AYD, and IFNg-201ABD) to human IFNγ, using EMSA.
[Figure 27] Figure 27 shows the results obtained by analyzing the detection sensitivity of 4 kinds of nucleic acid aptamers (B-I-Apt1-DD, B-I-Apt1-YY, B-IFNg-201AAD, B-IFNg-201AAB, and B-IFNg-201AYD) to human IFNγ, using ELISA. A nucleic acid aptamer was used for capturing IFNγ, and a B133.5 antibody was used as a detecting antibody. The results obtained by using the controls-a 2G1 antibody as a capture antibody and a Bio-B133.5 antibody as a detecting antibody-are shown in the right column.
[Figure 28] Figure 28 shows the results obtained by analyzing the detection sensitivity of the same 4 kinds of nucleic acid aptamers as those analyzed in Figure 19 to human IFNγ, using ELISA. A B133.5 antibody was used as an antibody for capturing IFNγ, and a nucleic acid aptamer was used for detection. The results obtained by using the controls-a B133.5 antibody as a capture antibody and a Bio-2G1 antibody as a detecting antibody-are shown in the right column.
[Figure 29] Figure 29 shows the results obtained by analyzing the detection sensitivity of 2 kinds of nucleic acid aptamers (B-IFNg-201AAD and B-IFNg-201AAB) to human IFNγ, using ELISA. A B133.5 antibody was used as an antibody for capturing IFNγ, and a nucleic acid aptamer shown in this figure was used for detection. The results obtained by using the controls-a 2G1 antibody as a capture antibody and a Bio-B133.5 antibody as a detecting antibody-are shown in the right column.
[Figure 30] Figure 30 shows the structure of an AptD1c(DsDs) aptamer that binds to DEN1-NS1 protein as a target.
[Figure 31] Figure 31 shows the results obtained by analyzing the affinity of 8 kinds of nucleic acid aptamers (AptD1c(DsDs), AptD1c2(DsDs), AptD1c2(AA), AptD1c2a(YsYs), AptD1c2b(YsBs), AptD1c2c(BsYs), and AptD1c2d(BsBs)) to DEN1-NS1 protein, using EMSA.
[Figure 32] Figure 32 shows the results obtained by analyzing the affinity of 6 kinds of nucleic acid aptamers (AptD2-b1, AptD2-b2, AptD2-b3, AptD2-b4, AptD2-bb(Ys), and AptD2-bc(Bs)) to DEN2-NS1 protein, DEN1-NS1 protein, ZIKA NS1 protein, and the like, using EMSA.
[Figure 33] Figure 33 shows the results obtained by analyzing the affinity of 5 kinds of nucleic acid aptamers (AptD4-1(DsDs), AptD4-2(YsYs), AptD4-3(YsBs), AptD4-4(BsYs), and AptD4-5(BsBs)) to DENV4 NS1 protein, using EMSA.
[Figure 34] Figure 34 shows the results obtained by analyzing the affinity of 7 kinds of nucleic acid aptamers (V-Apt1(DsDs), V-Apt4(AA), b58(YsYs), c58(YsBs), d58(BsYs), e58(BsBs), and f58(YoYo)) to human VEGF₁₆₅ protein, using EMSA.
[Figure 35] Figure 35 shows the results obtained by analyzing the affinity of 6 kinds of nucleic acid aptamers (MB45-Ds, MB45-Ys, MB45-Yo, MB45-Bs, MB45-Bo, and MB45-A) to human Transferrin receptor 1 (TrfR1), using EMSA.

### Description of Embodiments

### <Definitions of terms>

The definitions of the general terms used herein will be described below.

As used herein, a "nucleic acid" or a "nucleic acid molecule" refers to a polymer in which the constituent units are nucleosides in principle, and linked via an internucleoside bond.

As used herein, a "natural nucleoside" refers to a nucleoside present in nature. Examples include: a ribonucleoside consisting of a ribose and a base, such as adenine, cytosine, guanine, or uracil; and a deoxyribonucleoside consisting of a deoxyribose and said base, such as adenine, cytosine, guanine, or thymine.

As used herein, an "unnatural nucleoside" refers to an arbitrary nucleoside other than a natural nucleoside, and comprises a modified nucleoside and a nucleoside mimic.

As used herein, a "modified nucleoside" means a nucleoside having a modified portion, such as a modified base and/or a modified sugar portion.

As used herein, a "nucleotide" refers to a molecule having a phosphate group covalently bound to the sugar portion of a nucleoside. In a nucleotide comprising pentofuranosyl sugar, a phosphate group is usually linked to the hydroxyl group at position 2', position 3', or position 5' of the sugar. Amidite or phosphoramidite to be used as a raw material for nucleic acid synthesis is encompassed in a nucleotide.

As used herein, an "oligonucleotide" refers to a linear oligomer formed by several to tens of nucleotides linked with each other, in which the hydroxyl group of the sugar portion and the phosphate group are linked via covalent bonding between any nucleotides flanked with each other. In addition, a "polynucleotide" refers to a linear polymer formed by linking, via said covalent bonding, more nucleotides-tens or more of, preferably hundreds or more of, nucleotides-than in an oligonucleotide. Inside a natural oligonucleotide or polynucleotide structure, the phosphate group generally forms an internucleoside bond.

As used herein, a "natural base" refers to any of adenine, cytosine, guanine, thymine, uracil, and a modified base thereof that are present in nature. Note that, in the base sequences herein, a purine base selected from adenine and guanine is represented by "R", and a pyrimidine base selected from cytosine, thymine, and uracil is represented by "Y".

As used herein, an "unnatural base" or an "artificial base" refers to an arbitrary nucleobase other than a natural base. An unnatural base or an artificial base can substitute for a natural base constituting a natural nucleoside. In this regard, an unnatural base or an artificial base herein may be a base that is capable of forming an artificial base pair, or that is not capable of forming an artificial base pair.

As used herein, an "artificial base pair" refers to a pair of artificial bases capable of forming base pairing, such as adenine and thymine, adenine and uracil, or guanine and cytosine, which are natural bases. Base pairing formed by an artificial base pair can comprise a hydrogen bond found in base pairing between natural bases, a stacking effect via hydrophobic interaction, and the like. In some cases, a base-pairable artificial base pair can be accurately replicated and transcribed owing to the complementarity between the artificial bases. A nucleic acid strand comprising an artificial base can also be amplified using a nucleic acid amplification method, such as PCR.

Specific examples of the above-described artificial base include: unnatural bases represented by the below-described general formula (I) and/or general formula (II); and Ds (7-(2-thienyl)-3*H*-imidazo[4,5-*b*]pyridine-3-yl; herein referred to as "Ds"); Pn (2-nitropyrrole-1-yl; herein referred to as "Pn"); Pa (2-formyl-1*H*-pyrrole-1-yl; herein referred to as "Pa"); P (2-amino-imidazo[1,2-*a*]-1,3,5-triazin-4(8*H*)-one; herein referred to as "P"); Z (6-amino-5-nitro-2(1*H*)-pyridone; herein referred to as "Z"); 5SICS (6-methylisoquinoline-1(2*H*)-thione; herein referred to as "5SICS"); NaM (3-methoxynaphthalen-2-yl; herein referred to as "NaM"); and MMO2 (2-methoxy-4-methylphenyl; herein referred to as "MMO2"). Among these artificial bases, examples of a complementary artificial base of Ds include Pn and Pa. Example of a complementary artificial base of P include Z. Examples of a complementary artificial base of 5SICS include NaM and MMO2.

When the substrate for replication or transcription does not contain an unnatural nucleoside having a complementary artificial base for an artificial base, the artificial base is base-paired alternatively with a natural base close to the complementary artificial base in structure and/or nature, in some cases. In this case, the unnatural nucleoside in a nucleic acid molecule used as a template result in being substituted with the natural nucleoside after replication or transcription. For example, it is known that the above-described Ds is substituted with A or T.

As used herein, a "modified base" means a chemically-modified base. Examples of the modified base include modified pyrimidines (for example, 5-hydroxycytosine, 5-fluorouracil, 4-thiouracil, 5-(3-indole-2-ethyl)uracil, and 5-(4-hydroxyphenyl-2-ethyl)uracil), modified purines (for example, 6-methyladenine and 6-thioguanosine), and other heterocyclic bases.

As used herein, a "stem structure" or a "stem region" means a double-stranded structure formed by part of the constituent bases, in which part, for example, consecutive two or more bases are completely or partly base-paired with each other. The length of the stem structure is, for example, 1 bp or more, 2 bp or more, 3 bp or more, 4 bp or more, or 5 bp or more, and/or 30 bp or less, 25 bp or less, 20 bp or less, 15 bp or less, 10 bp or less, or 8 bp or less. Examples of the stem structure include a structure formed by base-pairing of two base sequences of SEQ ID NOs: 105 and 106, SEQ ID NOs: 107 and 108, SEQ ID NOs: 109 and 110, SEQ ID NOs: 111 and 112, SEQ ID NOs: 113 and 114, SEQ ID NOs: 115 and 116, SEQ ID NOs: 117 and 118, SEQ ID NOs: 119 and 120, SEQ ID NOs: 121 and 122, SEQ ID NOs: 123 and 124, SEQ ID NOs: 125 and 126, SEQ ID NOs: 127 and 128, SEQ ID NOs: 129 and 130, SEQ ID NOs: 131 and 132, SEQ ID NOs: 133 and 134, SEQ ID NOs: 135 and 136, or SEQ ID NOs: 138 and 139.

As used herein, a "mini-hairpin structure" or a "mini-hairpin" means a structure in which the below-described three DNA nucleic acid regions-the first nucleic acid region, the second nucleic acid region, and the third nucleic acid region-are linked in this order from the 5' end side to the 3' end side. The "first nucleic acid region" is a nucleic acid region consisting of two to five arbitrary nucleotides. A base in this nucleic acid region is preferably, but not limited to, guanine or cytosine. The "second nucleic acid region" is a nucleic acid region consisting of the base sequence 5'-gna-3' or 5'-gnna-3'. Each n in the sequence independently consists of any of a natural base, said base analog, and said modified base. The "third nucleic acid region" is a nucleic acid region having a base sequence complementary to the first nucleic acid region. Accordingly, the base sequence of the third nucleic acid region depends on the base sequence of the first nucleic acid region. In addition, the first nucleic acid region and the third nucleic acid region form a base pair in a molecule. As a result, the first nucleic acid region and the third nucleic acid region are completely base-paired with each other to constitute a stem portion, and in addition, the second nucleic acid region present between the first nucleic acid region and the third nucleic acid region constitutes a loop portion. One example of the mini-hairpin sequence includes 5'-CGCGTAGCG-3' (SEQ ID NO: 214; the base T may be biotinylated). The mini-hairpin structure enhances degradation resistance to nuclease, and/or can result in an increased thermal stability of a DNA aptamer through increasing the Tm value of the DNA aptamer.

As used herein, an "internal loop structure" refers to a loop structure that is within a stem structure, and generated in a case where one or more bases not forming a base pair are present at the positions corresponding between both of two strands forming the stem structure.

As used herein, a "bulge structure" refers to a protrusion structure within a stem structure, in which the protrusion structure is generated only at one of the positions corresponding between two strands forming the stem structure, at which position one or more bases not forming a base pair are present.

As used herein, the "loop structure" means a loop-shaped structure not base-paired inside a nucleic acid, in which the structure is generated owing to the formation of a stem structure, and located between two strands constituting the stem structure.

As used herein, a "hairpin structure" or a "stem-loop structure" means a structure consisting of one stem structure and one loop structure (one set of a stem structure and a loop structure).

As used herein, a "target molecule" refers to a substance that can be an object to which a nucleic acid aptamer binds. The target molecule is not particularly limited to any kind, subject to being a biological material to which a nucleic acid aptamer can bind. Examples include: proteins, such as peptides (oligopeptides and polypeptides); nucleic acids; lipids; sugars (comprising a sugar chain); and low-molecular-weight compounds. A molecule as a target to which a nucleic acid aptamer of the present invention binds is a protein in principle, and thus, a target molecule is herein often referred to as a "target protein".

As used herein, the term "complementary" means the relationship which enables nucleobases to form what is called a Watson-Crick base pair (natural base pair) or a non-Watson-Crick base pair (Hoogsteen base pair or the like) via hydrogen bonding. In the present invention, a base sequence is acceptable, subject to having a complementarity of at least 80%, preferably at least 90% (for example, 95%, 96%, 97%, 98%, or 99% or more). The complementarity of a base sequence can be determined using the BLAST program or the like.

As used herein, the term "multiple" refers to, for example, 2 to 10, 2 to 9, 2 to 8, 2 to 7, 2 to 6, 2 to 5, 2 to 4, 2 to 3, or 2.

### <Nucleic acid aptamer>

In one aspect of the present invention, a nucleic acid aptamer is provided.

As used herein, a "nucleic acid aptamer" is an aptamer composed of a nucleic acid molecule, and refers to a ligand molecule that binds firmly and specifically to a target molecule owing to the conformation formed on the basis of a secondary structure, or furthermore a tertiary structure, formed by single-stranded nucleic acid molecules via hydrogen bonding or the like. In a case where a nucleic acid aptamer has the capability to specifically inhibit or suppress the function, for example, the physiological activity, of a target molecule, the nucleic acid aptamer can be an agent for inhibiting the function of the target molecule. As used herein, "inhibiting the function of a target molecule" refers to inhibiting or suppressing the function, for example, the catalytic function or the gene expression regulating function, of a target molecule. As used herein, a "target molecule" refers to a substance that can be an object to which a nucleic acid aptamer binds. As used herein, a nucleic acid constituting a nucleic acid aptamer is not particularly limited to any kind, and may be, for example, a DNA aptamer composed of DNA alone, an RNA aptamer composed of RNA alone, or a nucleic acid aptamer composed of a combination of DNA and RNA.

A nucleic acid aptamer of the present invention comprises at least one unnatural base represented by the following general formula(s) (I) and/or (II), 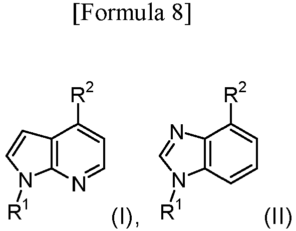 wherein R¹ represents a sugar portion in a nucleoside or a C¹ or C² alkyl group, and R² represents any of the following formulas (III) to (IX):

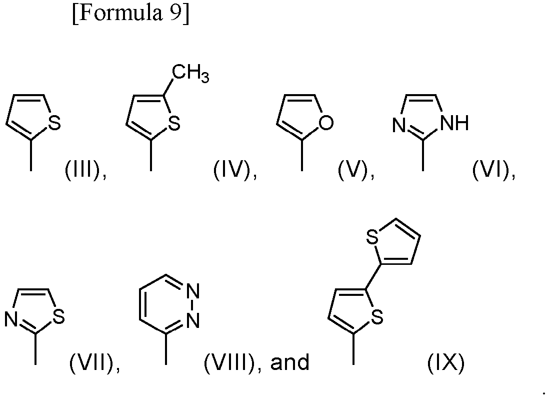

For a nucleic acid aptamer of the present invention, R¹ in the above-described general formula (I) and/or general formula (II) represents a sugar portion in a nucleoside, or represents a C¹ or C² alkyl group. In a case where the nucleic acid aptamer is a DNA aptamer, the sugar portion in a nucleoside is a deoxyribose portion, and the unnatural base binds to the carbon atom at position 1' in the deoxyribose. In a case where the nucleic acid aptamer is an RNA aptamer, the sugar portion in a nucleoside is a ribose portion, and the unnatural base binds to the carbon atom at position 1' in the ribose.

As used herein, an "alkyl" or "alkyl group" refers to a saturated hydrocarbon having one or more carbon atoms. Specific examples of the alkyl group include methyl, ethyl, propyl, and butyl. The number of carbon atoms in a "C¹ or C² alkyl group" is 1 or 2, and the alkyl group in this case is a methyl group or an ethyl group.

Examples of substitution of a hydrogen group in an alkyl group include substitution with hydroxyl, carboxyl, cyano, nitro, halogen (for example, Cl, F, Br, or I), thiol, alkoxyl group, ester, thioether, thio ester, amine, or the like. The number of substitutions of a hydrogen group in an alkyl group is not limited, and may be, for example, 1 or 2 or more.

In a nucleic acid aptamer of the present invention, the number of unnatural bases represented by the above-described general formula (I) and/or general formula (II) is not limited, subject to being 1 or more, and may be, for example, 1 or more, 2 or more, 3 or more, or 4 or more, and/or may be 20 or less, 10 or less, 8 or less, 7 or less, 6 or less, or 5 or less. For example, a nucleic acid aptamer of the present invention comprises one to nine, one to six, or one to three, for example, one, two, three, or four unnatural bases represented by the above-described general formula (I) and/or general formula (II).

In addition, in a nucleic acid aptamer of the present invention, an unnatural base represented by the above-described general formula (I) and/or general formula (II) is not limited to any position, and may be located, for example, on the 5' end and/or 3' end of a nucleic acid strand constituting the nucleic acid aptamer, and/or inside the nucleic acid strand.

In one embodiment, a nucleic acid aptamer of the present invention comprises an unnatural base represented by the above-described general formulas (I) and (II). In the present embodiment, the number of unnatural bases represented by each of the above-described general formula (I) and general formula (II) is not limited, subject to being 1 or more, and may be, for example, 1 or more and 20 or less, more specifically 1, 2, 3, or 4.

In one embodiment, an unnatural base that is in a nucleic acid aptamer of the present invention, and represented by the above-described general formula (I) and general formula (II) is an arbitrary unnatural base other than an unnatural base represented by the below-described formula (XVIII) (herein referred to as "Ds").

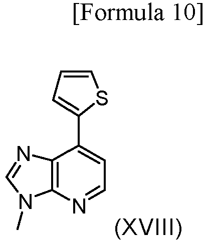

In one embodiment, an unnatural base that is in a nucleic acid aptamer of the present invention, and represented by the above-described general formula (I) and/or general formula (II) may be any unnatural base represented by the below-described formula (X) to formula (XVII) and formula (XXI) to formula (XXII). In this regard, herein, an unnatural base represented by the following formula (X) is referred to as "Ys", an unnatural base represented by the following formula (XI) is referred to as "Yi", an unnatural base represented by the following formula (XII) is referred to as "Yo", an unnatural base represented by the following formula (XIII) is referred to as "Ysm", an unnatural base represented by the following formula (XIV) is referred to as "Yss", an unnatural base represented by the following formula (XV) is referred to as "Bs", an unnatural base represented by the following formula (XVI) is referred to as "Bss", an unnatural base represented by the following formula (XVII) is referred to as "Bo", an unnatural base represented by the following formula (XXI) is referred to as "Dss", and an unnatural base represented by the following formula (XXII) is referred to as "Dt".

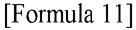

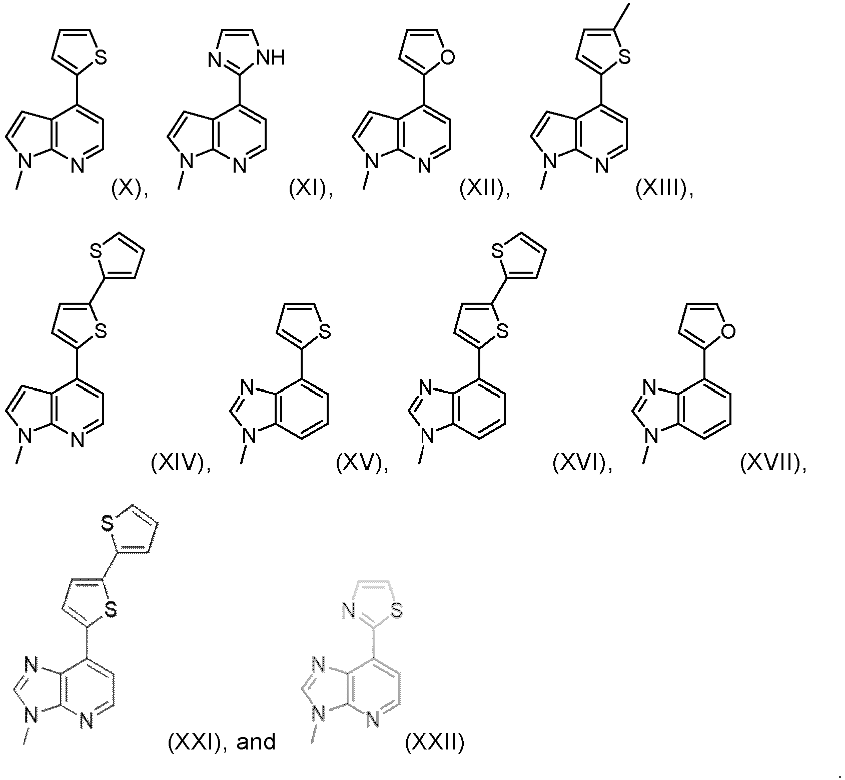

In one embodiment, a nucleic acid aptamer of the present invention comprises an unnatural base (Ds) represented by the above-described formula (XVIII) in addition to an unnatural base represented by the above-described formula(s) (I) and/or (II). In the present embodiment, the number of Ds's is not limited, subject to being 1 or more, and may be, for example, 1 or more and 20 or less, more specifically 1, 2, 3, or 4.

In one embodiment, a nucleic acid aptamer of the present invention is composed of a 5' region, a central region, and a 3' region. As used herein, the "5' region" and the "3' region" are regions that are located on the 5' end side and the 3' end side respectively in the nucleic acid aptamer, and each comprises a pair of base sequences complementary with each other so as to form a stem structure. As used herein, the "central region" is a region that is located between said 5' region and said 3' region, and mainly has the role of binding to a target molecule. In principle, a nucleic acid aptamer of the present invention can comprise, in the central region, an unnatural base represented by the above-described general formula (I) and/or general formula (II).

In one embodiment, a nucleic acid aptamer of the present invention comprises a mini-hairpin sequence on the 5' end side, on the 3' end side, and/or inside the sequence, for example, the 3' end side. The mini-hairpin sequence may have a modifying group, such as biotin, bound thereto. For example, a nucleic acid aptamer of the present invention comprises the above-described 5' region, central region, and 3' region, and mini-hairpin sequence in this order from the 5' side, or comprises the mini-hairpin sequence and the above-described 5' region, central region, and 3' region in this order from the 5' side.

In addition, in one embodiment, a nucleic acid aptamer of the present invention comprises an internal loop structure, bulge structure, loop structure, hairpin structure, and/or stem-loop structure at a site not influential to the capacity of the nucleic acid aptamer to bind to a target protein.

The length of a nucleic acid aptamer of the present invention is, for example, 10 mer or more, 15 mer or more, 20 mer or more, 30 mer or more, 40 mer or more, 50 mer or more, 60 mer or more, 70 mer or more, 80 mer or more, 90 mer or more, or 100 mer or more, and/or 300 mer or less, 200 mer or less, 150 mer or less, 140 mer or less, 130 mer or less, 120 mer or less, 110 mer or less, 100 mer or less, 90 mer or less, 80 mer or less, 70 mer or less, 60 mer or less, or 50 mer or less.

A nucleic acid aptamer of the present invention optionally comprises a base analog, another artificial base, another modified base, or the like in addition to the above-described unnatural base and/or Ds.

A nucleic acid aptamer of the present invention may be modified by addition of another substance, for example, polyethylene glycol (PEG) (for example, an approximately 20 to approximately 60 kDa PEG polymer), an amino acid, peptide, inverted dT, lipid, pigment, fluorescent substance, enzyme, radioactive substance, or biotin. If desired, such a substance may be linked via a known linker. Examples of the linker herein include a nucleotide linker, peptide linker, and linker comprising a disulfide bond. It is known that linking PEG generally enables the half-life period of a DNA aptamer to be extended. In addition, the position of modification of a nucleic acid aptamer of the present invention is not limited, and may be, for example, the 5' end portion, the 3' end portion, or inside the aptamer sequence. For example, the mini-hairpin sequence may be modified.

A method for producing a nucleic acid aptamer of the present invention is not particularly limited. A method known in the art may be used. For example, a nucleic acid aptamer of the present invention may be chemically synthesized in accordance with a known solid phase synthesis method on the basis of the above-described sequence. For a method for chemically synthesizing a nucleic acid, see, for example, Current Protocols in Nucleic Acid Chemistry, Volume 1, Section 3. In addition, the nucleic acid aptamer may be produced, for example, by synthesizing some fragments on the basis of the sequence of the nucleic acid aptamer, followed by linking the fragments by intramolecular annealing, ligation with ligase, or the like. A nucleic acid aptamer of the present invention, after chemical synthesis, is preferably purified using a method known in the art, before use. Examples of a purifying method include a gel purification method, affinity column purification method, and HPLC method.

Furthermore, the present invention also provides: a nucleoside or nucleotide comprising an unnatural base represented by the above-described general formula (I) or general formula (II); and a nucleic acid (for example, DNA or RNA) comprising an unnatural base represented by the above-described general formula (I) and/or general formula (II). For example, the nucleic acid aptamer also provides: a nucleoside or nucleotide (for example, amidite) comprising an unnatural base represented by any of the above-described formula (XI) to formula (XIII) and the above-described formula (XV) to formula (XVII); and a nucleic acid (for example, an oligonucleotide or polynucleotide, such as DNA or RNA) comprising an unnatural base represented by the above-described formula (XI) to formula (XIII) and the above-described formula (XV) to formula (XVII).

In (1) to (10) below, the constitution of each binding target for a nucleic acid aptamer of the present invention will be described specifically.

### <(1) Nucleic acid aptamer that binds to vWF>

In one further aspect of a nucleic acid aptamer of the present invention, a nucleic acid aptamer that binds to vWF protein is provided.

As used herein, "vWF" refers to von Willebrand factor protein (herein referred to as "vWF protein"). vWF is one of the blood coagulation factors present in blood. It is known that the gene mutation thereof involves various diseases, such as von Willebrand disease, and that the production of an autoantibody to vWF induces acquired thrombotic thrombocytopenic purpura or the like. In the present invention, the origin of vWF is not limited to any biological species. Examples include: mammals, for example, primates, such as humans and chimpanzees: laboratory animals, such as rats and mice; livestock animals, such as pigs, bovines, horses, sheep, and goats; and pet animals, such as dogs and cats. vWF from a human is preferable.

The nucleic acid aptamer of the present aspect comprises the base sequence of SEQ ID NO: 205, 206, or 187, or the same base sequence as SEQ ID NO: 205, 206, or 187 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

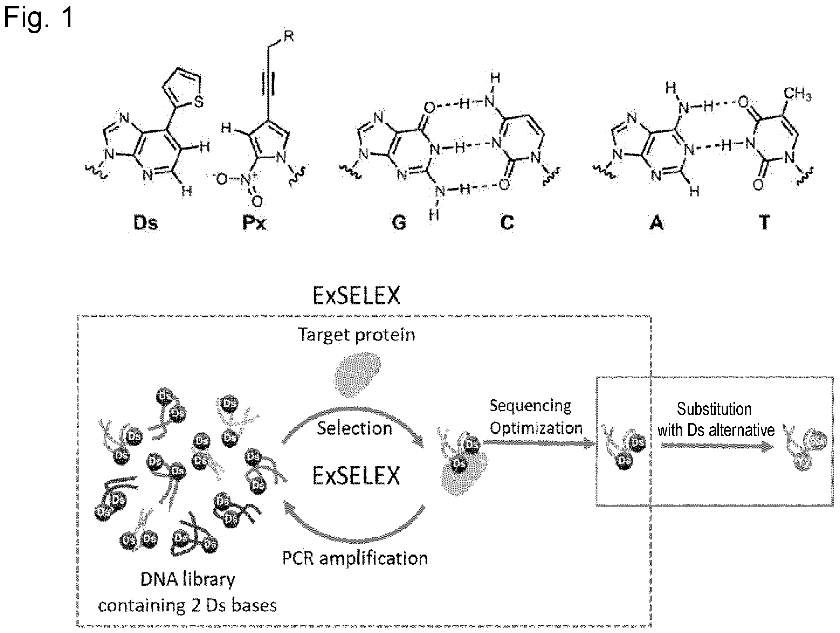

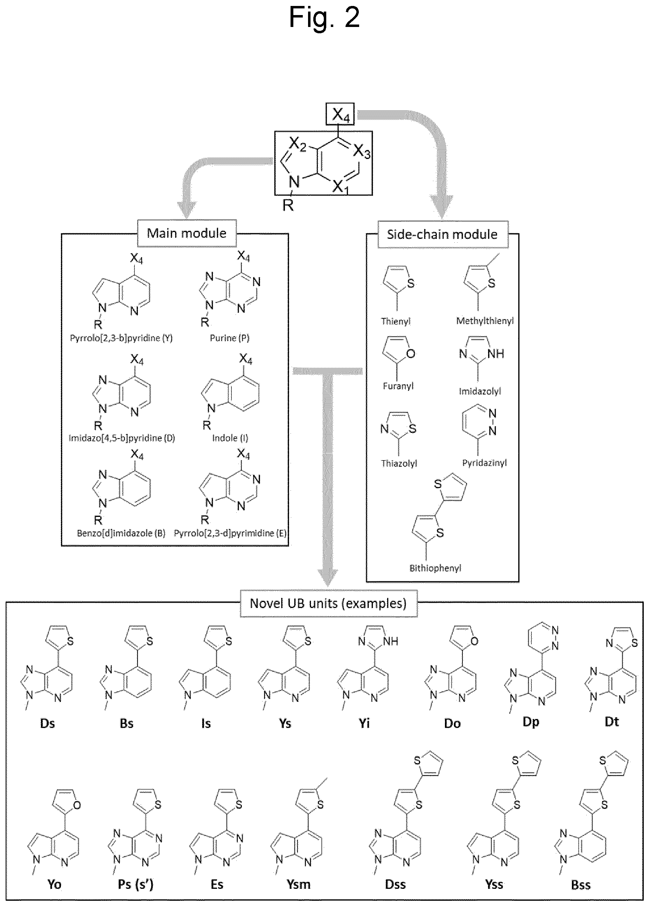

The base sequence of SEQ ID NO: 206 (5'-GAnGAGYACCTAACnGGTCTCRRYRnYGGA-3') encompasses a sequence resulting from removing a stem region consisting of the respective 7 bases of the 5' end and the 3' end from the base sequence of "Anti-vWF" in Figure 1(b) in the literature (Kimoto M., et al., bioRxiv 2022.06.27. 497860) and the base sequences (SEQ ID NOs: 9 to 14) comprising the substitutions (C14T, A29G, A30G, C31T, G32A, and T34C) shown to be free from impairing the binding activity in Figure 2(c) in the same literature. The base sequence of SEQ ID NO: 205 (5'-GAnGAGYACCGAAGGTCTCRRYRnY-3') encompasses a sequence resulting from removing a stem region consisting of the respective 7 bases of the 5' end and the 3' end from the base sequence of the nucleic acid aptamer vWF2-DsDsDs-2mhGC of SEQ ID NO: 21 in WO2017/073536 and a sequence resulting from extending, to acceptable bases, the positions corresponding to the above-described acceptable substitutions (C14T, A29G, A30G, C31T, G32A, and T34C) in the base sequence. The base sequence of SEQ ID NO: 187 (5'-GAnGAGCACCGAAGGTCTCAACGnTGGA-3') corresponds to the central region resulting from removing a stem region and a mini-hairpin sequence from the base sequence of a nucleic acid aptamer such as AptvWF-BsBs in (2) in Example 3, is encompassed in the base sequence of SEQ ID NO: 205, and is a sequence resulting from adding the base sequence GGA to the 3' end side of a base sequence having purine bases that are, and pyrimidines bases that are, specified as specific bases.

A nucleic acid aptamer that binds to a vWF protein comprises, as a base represented by n in the above-described base sequence, at least one, for example, two, three, or four unnatural bases represented by the above-described general formula (I) and/or general formula (II).

In a further embodiment, a nucleic acid aptamer that binds to vWF protein may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to vWF protein is selected from the group consisting of (1-a) to (1-d) below:
(1-a) a nucleic acid aptamer comprising the base sequence of SEQ ID NO: 1 or 3, wherein, in said base sequence, the bases represented by n at position 10 and position 31 respectively are (I) base Bs and base Bs, (II) base Ys and base Ys, (III) base Yo and base Yo, (IV) base Bs and base Ys, (V) base Bs and base Yo, or (VI) base Ys and base Bs;
(1-b) a nucleic acid aptamer comprising the same base sequence as SEQ ID NO: 1 or 3 except that one or multiple bases are deleted, substituted, and/or added except at position 10 and position 31, wherein, in said base sequence, the positions corresponding to the bases represented by n at position 10 and position 31 respectively in SEQ ID NO: 1 or 3 are (I) base Bs and base Bs, (II) base Ys and base Ys, (III) base Yo and base Yo, (IV) base Bs and base Ys, (V) base Bs and base Yo, or (VI) base Ys and base Bs;
(1-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 205 or 187, and the bases represented by n at position 3 and position 24 respectively in said base sequence of SEQ ID NO: 205 or 187 are (I) base Bs and base Bs, (II) base Ys and base Ys, (III) base Yo and base Yo, (IV) base Bs and base Ys, (V) base Bs and base Yo, or (VI) base Ys and base Bs; and
(1-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 205 or 187 except that one or multiple bases are deleted, substituted, and/or added except at position 3 and position 24, wherein, in said base sequence in said central region, the positions corresponding to the bases represented by n at position 3 and position 24 respectively in SEQ ID NO: 205 or 187 are (I) base Bs and base Bs, (II) base Ys and base Ys, (III) base Yo and base Yo, (IV) base Bs and base Ys, (V) base Bs and base Yo, or (VI) base Ys and base Bs.

In this regard, the above-described SEQ ID NOs: 205 and 187 are sequences resulting from adding, to the 5' end side and 3' end side of SEQ ID NOs: 1 and 3 respectively, base sequences complementary with each other, and capable of forming a stem structure. In addition, the base sequence of SEQ ID NO: 3 is encompassed in the base sequence of SEQ ID NO: 1, and has purine bases that are, and pyrimidine bases that are, specified as specific bases.

### <(2) Nucleic acid aptamer that binds to IFNγ>

In one further aspect of a nucleic acid aptamer of the present invention, a nucleic acid aptamer that binds to an IFNγ protein is provided.

As used herein, "IFNγ (Interferon gamma)" is a cytokine that is secreted from an immune cell, such as a T cell or an NK cell, and is produced in an immune response to infection by a virus, bacterium, or the like. In the present invention, the origin of IFNγ is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. IFNγ from a human is preferable.

The nucleic acid aptamer of the present aspect comprises the base sequence of SEQ ID NO: 207, 208, 188, 56, 59, 61, 65, 67, 189, or 213, or the same base sequence as SEQ ID NO: 207, 208, 188, 56, 59, 61, 65, 67, 189, or 213 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 188 (5'-CGGGTCCGCGAAGCGGTAGGTnTGGGCTAGGCnGCT-3') corresponds to the central region resulting from removing a stem region and a mini-hairpin sequence from the base sequence of a nucleic acid aptamer such as AptIFNγ-YoYo in the result (1) in Example 3. The base sequence of SEQ ID NO: 207 (5'-CGGGTCCGCGAAGCGGTAGGTnYGGGCTAGGCnGYY-3') encompasses a sequence resulting from removing a stem region consisting of the respective six bases of the 5' end and the 3' end from the base sequence of "Anti-IFNγ" in Figure 1(b) in the literature (Kimoto M., et al., bioRxiv 2022.06.27. 497860) and the base sequences (SEQ ID NOs: 23 to 25) comprising the substitutions (T30C, C42T, and T43C) shown to be free from impairing the binding activity in Figure 2(b) in the same literature. The base sequence of SEQ ID NO: 208 (5'-CGGGTCATTTAnTAATGTAGGTnYGGGCTAGGCnGYY-3') encompasses the base sequence of "Anti-IFNγ" in Figure 1(b) in the literature (Kimoto M., *et al.*, *bioRxiv* 2022.06.27. 497860) and base sequence resulting from extending, to acceptable bases, the positions corresponding to the above-described acceptable substitutions. The base sequence of SEQ ID NO: 56 (5'-TGTGGTGGGACnGGGCTnATGTAnGGGATCT-3'), the base sequence of SEQ ID NO: 59 (5'-TGTGGTGGGACTGGGCTnATGTAnGGGATCT-3'), and the base sequence of SEQ ID NO: 61 (5'-TGTGGTGGGACnGGGCTAATGTAnGGGATCT-3') correspond to the central region resulting from removing the sequences of the end portions comprising a stem region from the base sequence of a nucleic acid aptamer such as AptIFNγ-1 in (b) in Example 4. The base sequence of SEQ ID NO: 65 (5'-CCGTCATnATTGTTnTTTTGATCCTTGnAGTGGGGA-3') and the base sequence of SEQ ID NO: 67 (5'-CCGTCATAnTTGTTnTTTTGATCCTTGnAGTGGGGA-3') correspond to the central region resulting from removing the sequences of the end portions comprising a stem region from the base sequence of a nucleic acid aptamer such as XL2-01a in (c) in Example 4. The base sequence of SEQ ID NO: 189 (5'-CCGTCATAATTGTTnTTTTGATCCTTGnAGTGGGGA-3') and the base sequence of SEQ ID NO: 213 (5'-CCGTCATAATTGTTATTTTGATCCTTGnAGTGGGGA-3') correspond to the central region resulting from removing a stem region from the base sequence of a nucleic acid aptamer such as IFNg-201AAY in Example 7.

A nucleic acid aptamer that binds to IFNγ comprises at least one unnatural base, for example, comprises two, three, or four unnatural bases represented by the above-described general formula (I) and/or general formula (II) as a base represented by n in the above-described base sequence.

In a further embodiment, a nucleic acid aptamer that binds to IFNγ may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to IFNγ is selected from the group consisting of (2-i-a) to (2-iii-d) below:
(2-i-a) a nucleic acid aptamer comprising the base sequence of SEQ ID NO: 15 or 17 wherein, in said base sequence, the bases represented by n at position 28 and position 39 respectively are (I) base Ys and base Ys, or (II) base Yo and base Yo;
(2-i-b) a nucleic acid aptamer comprising the same base sequence as SEQ ID NO: 15 or 17 except that one or multiple bases are deleted, substituted, and/or added except at position 28 and position 39, wherein, in said base sequence, the positions corresponding to the bases represented by n at position 28 and position 39 respectively in SEQ ID NO: 15 or 17 are (I) base Ys and base Ys or (II) base Yo and base Yo;
(2-i-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 207 or 188, and the bases represented by n at position 22 and position 33 respectively in said base sequence of SEQ ID NO: 207 or 188 are (I) base Ys and base Ys or (II) base Yo and base Yo;
(2-i-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 207 or 188 except that one or multiple bases are deleted, substituted, and/or added except at position 22 and position 33, wherein, in said base sequence in said central region, the positions corresponding to the bases represented by n at position 22 and position 33 respectively in SEQ ID NO: 207 or 188 are (I) base Ys and base Ys or (II) base Yo and base Yo;
(2-ii-a) a nucleic acid aptamer comprising the base sequence of SEQ ID NO: 62 wherein, in said base sequence, the bases represented by n at position 24 and position 37 respectively are (I) base Ys and base Ds or (II) base Bs and base Ds;
(2-ii-b) a nucleic acid aptamer comprising the same base sequence as SEQ ID NO: 62 except that one or multiple bases are deleted, substituted, and/or added except at position 24 and position 37, wherein, in said base sequence, the positions corresponding to the bases represented by n at position 24 and position 37 respectively in SEQ ID NO: 62 are (I) base Ys and base Ds or (II) base Bs and base Ds;
(2-ii-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 189, and the bases represented by n at position 15 and position 28 respectively in said base sequence of SEQ ID NO: 189 are (I) base Ys and base Ds or (II) base Bs and base Ds;
(2-ii-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 189 except that one or multiple bases are deleted, substituted, and/or added except at position 15 and position 28, wherein, in said base sequence in said central region, the positions corresponding to the bases represented by n at position 15 and position 28 respectively in SEQ ID NO: 189 are (I) base Ys and base Ds or (II) base Bs and base Ds;
(2-iii-a) a nucleic acid aptamer comprising the base sequence of SEQ ID NO: 212 wherein, in said base sequence, the base represented by n at position 37 is base Ys or base Bs;
(2-iii-b) a nucleic acid aptamer comprising the base sequence as SEQ ID NO: 212 except that one or multiple bases are deleted, substituted, and/or added except at position 37, wherein, in said base sequence, the position corresponding to the base represented by n at position 37 in SEQ ID NO: 212 is base Ys or base Bs;
(2-iii-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 213, and the base represented by n at position 28 in said base sequence of SEQ ID NO: 213 is base Ys or base Bs; and
(2-iii-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 213 except that one or multiple bases are deleted, substituted, and/or added except at position 28, wherein, in said base sequence in said central region, the position corresponding to the base represented by n at position 28 in SEQ ID NO: 213 is base Ys or base Bs.

In this regard, the above-described SEQ ID NOs: 15, 17, 62 and 212 are sequence resulting from adding, to the 5' end side and 3' end side of SEQ ID NOs: 207, 188, 189 and 213 respectively, sequences complementary with each other, and capable of forming a stem structure. In addition, the base sequence of SEQ ID NO: 17 is encompassed in the base sequence of SEQ ID NO: 15, and has purine bases that are, and pyrimidine bases that are, specified as specific bases.

### <(3) Nucleic acid aptamer that binds to Thrombin>

In one further aspect of a nucleic acid aptamer of the present invention, a nucleic acid aptamer that binds to Thrombin is provided.

As used herein, "Thrombin" is a serine protease that plays a leading role in hemostasis through converting fibrinogen into fibrin. In the present invention, the origin of Thrombin is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. Thrombin from a human is preferable.

The nucleic acid aptamer of the present aspect comprises the base sequence of SEQ ID NO: 190, or the same base sequence as SEQ ID NO: 190 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 190 (5'-TGTGGTnGTTTTnTTGGGGGGGTGGTTAAG-3') corresponds to the central region resulting from removing a stem region from the base sequence of a nucleic acid aptamer such as Thr204-BB in Example 5.

A nucleic acid aptamer that binds to Thrombin comprises, as a base represented by n in the above-described base sequence, at least one, for example, two, three, or four unnatural bases represented by the above-described general formula (I) and/or general formula (II).

In a further embodiment, a nucleic acid aptamer that binds to Thrombin may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to Thrombin is selected from the group consisting of (3-a) to (3-d) below:
(3-a) a nucleic acid aptamer comprising the base sequence of SEQ ID NO: 81 wherein, in said base sequence, the bases represented by n at position 16 and position 22 respectively are (I) base Bs and base Bs, (II) base Bs and base Ys, (III) base Ys and base Bs, or (IV) base Ys and base Ys;
(3-b) a nucleic acid aptamer comprising the same base sequence as SEQ ID NO: 81 except that one or multiple bases are deleted, substituted, and/or added except at position 16 and position 22, wherein, in said base sequence, the positions corresponding to the bases represented by n at position 16 and position 22 respectively in SEQ ID NO: 81 are (I) base Bs and base Bs, (II) base Bs and base Ys, (III) base Ys and base Bs, or (IV) base Ys and base Ys;
(3-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 190, and the bases represented by n at position 7 and position 13 respectively in said base sequence of SEQ ID NO: 190 are (I) base Bs and base Bs, (II) base Bs and base Ys, (III) base Ys and base Bs, or (IV) base Ys and base Ys; and
(3-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 190 except that one or multiple bases are deleted, substituted, and/or added except position 7 and position 13, wherein, in said base sequence in said central region, the positions corresponding to the bases represented by n at position 7 and position 13 respectively in SEQ ID NO: 190 are (I) base Bs and base Bs, (II) base Bs and base Ys, (III) base Ys and base Bs, or (IV) base Ys and base Ys.

In this regard, the above-described SEQ ID NO: 81 is a sequence resulting from adding, to the 5' end side and 3' end side of SEQ ID NO: 190, the respective sequences complementary with each other, and capable of forming a stem structure.

### <(4) Nucleic acid aptamer that binds to VEGF>

In one further aspect of a nucleic acid aptamer of the present invention, a nucleic acid aptamer that binds to VEGF is provided.

As used herein, "VEGF (vascular endothelial growth factor)" is a growth factor that functions as an angiogenic growth factor, and is known to be one of the causative factors of age-related macular degeneration (AMD). Age-related macular degeneration is a progressive retinal disease that causes a serious symptom in an adult, such as decreased visual performance or acquired blindness. It has been proved that the pathology of the disease is worsened with the progress of angiogenesis in the retina, and thus becomes severe.

In the present invention, the origin of VEGF is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. VEGF from a human is preferable.

The nucleic acid aptamer of the present aspect comprises the base sequence of SEQ ID NO: 209 or 192, or the same base sequence as SEQ ID NO: 209 or 192 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 192 (5'-TAAGCCGCGTCCGAAGGGGCnTGCGGCGAnCCCGAATGGGT-3') corresponds to the central region resulting from removing a stem region and a mini-hairpin sequence from the base sequence of a nucleic acid aptamer such as b58(YsYs) in Example 11. The base sequence of SEQ ID NO: 209 (5'-TAAACTGCGTCCGAAGGGGCnTGCAGTGAnCCCGAATGGGT-3') corresponds to a sequence resulting from removing a stem region consisting of the respective three bases of the 5' end and the 3' end from the base sequence of "Anti-VEGF₁₆₅" in Figure 1(b) in the literature (Kimoto M., et al., bioRxiv 2022.06.27. 497860).

A nucleic acid aptamer that binds to VEGF comprises, as a base represented by n in the above-described base sequence, at least one, for example, two, three, or four unnatural bases represented by the above-described general formula (I) and/or general formula (II).

In a further embodiment, a nucleic acid aptamer that binds to VEGF may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to VEGF is selected from the group consisting of (4-a) to (4-d) below:
(4-a) a nucleic acid aptamer comprising the base sequence of SEQ ID NO: 191 wherein, in said base sequence, the bases represented by n at position 25 and position 34 respectively are (I) base Ys and base Ys, (II) base Ys and base Bs, (III) base Bs and base Ys, (IV) base Bs and base Bs, or (V) base Yo and base Yo;
(4-b) a nucleic acid aptamer comprising the same base sequence as SEQ ID NO: 191 except that one or multiple bases are deleted, substituted, and/or added except at position 25 and position 34, wherein, in said base sequence, the positions corresponding to the bases represented by n at position 25 and position 34 respectively in SEQ ID NO: 191 are (I) base Ys and base Ys, (II) base Ys and base Bs, (III) base Bs and base Ys, (IV) base Bs and base Bs, or (V) base Yo and base Yo;
(4-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 192, and the bases represented by n at position 21 and position 30 respectively in said base sequence of SEQ ID NO: 192 are (I) base Ys and base Ys, (II) base Ys and base Bs, (III) base Bs and base Ys, (IV) base Bs and base Bs, or (V) base Yo and base Yo; and
(4-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 192 except that one or multiple bases are deleted, substituted, and/or added except at position 21 and position 30, wherein, in said base sequence in said central region, the positions corresponding to the bases represented by n at position 21 and position 30 respectively in SEQ ID NO: 192 are (I) base Ys and base Ys, (II) base Ys and base Bs, (III) base Bs and base Ys, (IV) base Bs and base Bs, or (V) base Yo and base Yo.

In this regard, the above-described SEQ ID NO: 191 is a sequence resulting from adding, to the 5' end side and 3' end side of SEQ ID NO: 192, the respective sequences complementary with each other, and capable of forming a stem structure.

### <(5) Nucleic acid aptamer that binds to HMGB1>

In one further aspect of a nucleic acid aptamer of the present invention, a nucleic acid aptamer that binds to HMGB1 is provided.

"HMGB1" (High Mobility Group Box 1) herein functions as a chromatin protein, and in addition, is secreted from an immune cell, such as a macrophage or a monocyte, to function also as a cytokine mediator of inflammation. In the present invention, the origin of HMGB1 is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. HMGB1 from a human is preferable.

The nucleic acid aptamer of the present aspect comprises the base sequence of SEQ ID NO: 194, or the same base sequence as SEQ ID NO: 194 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 194 (5'-GCCACCGTATGnACGCTCACAGnAGTTGCCGA-3') corresponds to the central region resulting from removing a stem region and a mini-hairpin sequence from the base sequence of a nucleic acid aptamer such as Bio-HMGB1-301-BB in Example 6.

A nucleic acid aptamer that binds to HMGB1 comprises, as a base represented by n in the above-described base sequence, at least one, for example, two, three, or four unnatural bases represented by the above-described general formula (I) and/or general formula (II).

In a further embodiment, a nucleic acid aptamer that binds to HMGB1 may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to HMGB1 protein is selected from the group consisting of (5-a) to (5-d) below:
(5-a) a nucleic acid aptamer comprising the base sequence of SEQ ID NO: 193 wherein, in said base sequence, the bases represented by n at position 22 and position 33 respectively are (I) base Bs and base Bs, (II) base Bs and base Ys, (III) base Ys and base Bs, or (IV) base Ys and base Ys;
(5-b) a nucleic acid aptamer comprising the same base sequence as SEQ ID NO: 193 except that one or multiple bases are deleted, substituted, and/or added except at position 22 and position 33, wherein, in said base sequence, the positions corresponding to the bases represented by n at position 22 and position 33 respectively in SEQ ID NO: 193 are (I) base Bs and base Bs, (II) base Bs and base Ys, (III) base Ys and base Bs, or (IV) base Ys and base Ys;
(5-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 194, and the bases represented by n at position 12 and position 23 respectively in said base sequence of SEQ ID NO: 194 are (I) base Bs and base Bs, (II) base Bs and base Ys, (III) base Ys and base Bs, or (IV) base Ys and base Ys; and
(5-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 194 except that one or multiple bases are deleted, substituted, and/or added except at position 12 and position 23, wherein, in said base sequence in said central region, the positions corresponding to the bases represented by n at position 12 and position 23 respectively in SEQ ID NO: 194 are (I) base Bs and base Bs, (II) base Bs and base Ys, (III) base Ys and base Bs, or (IV) base Ys and base Ys.

In this regard, the above-described SEQ ID NO: 193 is a sequence resulting from adding, to the 5' end side and 3' end side of SEQ ID NO: 194, the respective sequences complementary with each other, and capable of forming a stem structure.

### <(6) Nucleic acid aptamer that binds to Transferrin receptor 1>

In one further aspect of a nucleic acid aptamer of the present invention, a nucleic acid aptamer that binds to Transferrin receptor 1 is provided.

As used herein, "Transferrin receptor 1" (TrfR1) is one kind of Transferrin receptor that functions as a receptor for transferrin that binds to an iron ion, and has the role of transporting. Transferrin receptor 1 takes in a transferrin/iron complex to transport the iron into a cell. In the present invention, the origin of Transferrin receptor 1 is not limited to any biological species. Examples include: mammals, for example, primates, such as humans and chimpanzees; laboratory animals, such as rats and mice; livestock animals, such as pigs, bovines, horses, sheep, and goats; and pet animals, such as dogs and cats. Transferrin receptor 1 from a human is preferable.

The nucleic acid aptamer of the present aspect comprises the base sequence of SEQ ID NO: 196, or the same base sequence as SEQ ID NO: 196 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 196 (5'-GGGGTGTTTGTGCCGTGAGnTG-3') corresponds to the central region resulting from removing a stem region and a mini-hairpin sequence from the base sequence of a nucleic acid aptamer such as MB45-Bs in Example 12.

A nucleic acid aptamer that binds to Transferrin receptor 1 comprises, as a base represented by n in the above-described base sequence, at least one, for example, two, three, or four unnatural bases represented by the above-described general formula (I) and/or general formula (II).

In a further embodiment, a nucleic acid aptamer that binds to Transferrin receptor 1 may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to Transferrin receptor 1 protein is selected from the group consisting of (6-a) to (6-d) below:
(6-a) a nucleic acid aptamer comprising the base sequence of SEQ ID NO: 195 wherein, in said base sequence, the base represented by n at position 27 is base Bs;
(6-b) a nucleic acid aptamer comprising the same base sequence as SEQ ID NO: 195 except that one or multiple bases are deleted, substituted, and/or added except at position 27, wherein, in said base sequence, the positions corresponding to the base represented by n at position 27 in SEQ ID NO: 195 is base Bs;
(6-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 196, and the base represented by n at position 20 in said base sequence of SEQ ID NO: 196 is base Bs; and
(6-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 196 except that one or multiple bases are deleted, substituted, and/or added except at position 20, wherein, in said base sequence in said central region, the position corresponding to the base represented by n at position 20 in SEQ ID NO: 196 is base Bs.

In this regard, the above-described SEQ ID NO: 195 is a sequence resulting from adding, to the 5' end side and 3' end side of SEQ ID NO: 196, the respective sequences complementary with each other, and capable of forming a stem structure.

### <(7) Nucleic acid aptamer that binds to DEN1 protein>

In one further aspect of a nucleic acid aptamer of the present invention, a nucleic acid aptamer that binds to Dengue Virus NS1 Protein Serotype 1 (DEN1 protein) is provided.

As used herein, "NS1 protein" refers to Nonstructural protein 1 derived from a dengue virus. Among NS1 proteins, Dengue Virus NS1 Protein Serotype 1 is referred to as "DEN1 protein", Dengue Virus NS1 Protein Serotype 2 is referred to as "DEN2 protein", Dengue Virus NS1 Protein Serotype 3 is referred to as "DEN3 protein", and Dengue Virus NS1 Protein Serotype 4 is referred to as "DEN4 protein".

The nucleic acid aptamer of the present aspect comprises the base sequence of SEQ ID NO: 210, 197 or 199, or the same base sequence as SEQ ID NO: 210, 197 or 199 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 197 (5'-ACTGGTGTnCTCGGnATGG-3') corresponds to the central region resulting from removing a stem region and a mini-hairpin sequence from the base sequence of a nucleic acid aptamer such as AptD1-YsDs in the result (3) in Example 3. The base sequence of SEQ ID NO: 210 (5'-RCTGGTGYnCTCGGnATGR-3') encompasses a sequence resulting from removing a stem region consisting of the respective 4 bases of the 5' end and the 3' end from the base sequences (SEQ ID NOs: 35 to 37) comprising the substitutions (A7G, T14C, and G25A) shown to be free from impairing the binding activity in Figure 2(d) in the literature (Kimoto M., et al., bioRxiv 2022.06.27. 497860). In addition, the base sequence of SEQ ID NO: 199 (5'-GTAnTCAGACGTATACnCATCA-3') corresponds to the central region resulting from removing a stem region and a mini-hairpin sequence from the base sequence of a nucleic acid aptamer such as AptD1c2a(YsYs) in Example 8.

A nucleic acid aptamer that binds to a DEN1 protein comprises, as a base represented by n in the above-described base sequence, at least one, for example, two, three, or four unnatural bases represented by the above-described general formula (I) and/or general formula (II).

In a further embodiment, a nucleic acid aptamer that binds to DEN1 protein may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to DEN1 protein is selected from the group consisting of the following (7-i-a) to (7-ii-d):
(7-i-a) a nucleic acid aptamer comprising the base sequence of SEQ ID NO: 27 or 29 wherein, in said base sequence, the bases represented by n at position 19 and position 25 respectively are (I) base Yss and base Ds, (II) base Ds and base Dt, (III) base Ys and base Bs, (IV) base Yo and base Bs, or (V) base Ysm and base Bs;
(7-i-b) a nucleic acid aptamer comprising the same base sequence as SEQ ID NO: 27 or 29 except that one or multiple bases are deleted, substituted, and/or added except at position 19 and position 25, wherein, in said base sequence, the positions corresponding to the bases represented by n at position 19 and position 25 respectively in SEQ ID NO: 27 or 29 are (I) base Yss and base Ds, (II) base Ds and base Dt, (III) base Ys and base Bs, (IV) base Yo and base Bs, or (V) base Ysm and base Bs;
(7-i-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 210 or 197, and the bases represented by n at position 9 and position 15 respectively in said base sequence of SEQ ID NO: 210 or 197 are (I) base Yss and base Ds, (II) base Ds and base Dt, (III) base Ys and base Bs, (IV) base Yo and base Bs, or (V) base Ysm and base Bs;
(7-i-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 210 or 197 except that one or multiple bases are deleted, substituted, and/or added except at position 9 and position 15, wherein, in said base sequence in said central region, the positions corresponding to the bases represented by n at position 9 and position 15 respectively in SEQ ID NO: 210 or 197 are (I) base Yss and base Ds, (II) base Ds and base Dt, (III) base Ys and base Bs, (IV) base Yo and base Bs, or (V) base Ysm and base Bs;
(7-ii-a) a nucleic acid aptamer comprising the base sequence of SEQ ID NO: 198 wherein, in said base sequence, the bases represented by n at position 14 and position 27 respectively are (I) base Ys and base Ys, (II) base Ys and base Bs, (III) base Bs and base Ys, or (IV) base Bs and base Bs;
(7-ii-b) a nucleic acid aptamer comprising the same base sequence as SEQ ID NO: 198 except that one or multiple bases are deleted, substituted, and/or added except at position 14 and position 27, wherein, in said base sequence, the positions corresponding to the bases represented by n at position 14 and position 27 respectively in SEQ ID NO: 198 are (I) base Ys and base Ys, (II) base Ys and base Bs, (III) base Bs and base Ys, or (IV) base Bs and base Bs;
(7-ii-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 199, and the bases represented by n at position 4 and position 17 respectively in said base sequence of SEQ ID NO: 199 are (I) base Ys and base Ys, (II) base Ys and base Bs, (III) base Bs and base Ys, or (IV) base Bs and base Bs; and
(7-ii-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 199 except that one or multiple bases are deleted, substituted, and/or added except at position 4 and position 17, wherein, in said base sequence in said central region, the positions corresponding to the bases represented by n at position 4 and position 17 respectively in SEQ ID NO: 199 are (I) base Ys and base Ys, (II) base Ys and base Bs, (III) base Bs and base Ys, or (IV) base Bs and base Bs.

In this regard, the above-described SEQ ID NOs: 27, 29 and 198 are sequences resulting from adding, to the 5' end side and 3' end side of SEQ ID NOs: 210, 197 and 199 respectively, sequences complementary with each other, and capable of forming a stem structure. In addition, the base sequence of SEQ ID NO: 29 is encompassed in the base sequence of SEQ ID NO: 27, and has purine bases that are, and pyrimidine bases that are, specified as specific bases.

### <(8) Nucleic acid aptamer that binds to DEN2 protein>

In one further aspect of a nucleic acid aptamer of the present invention, a nucleic acid aptamer that binds to Dengue Virus NS1 Protein Serotype 2 (DEN2 protein) is provided.

The nucleic acid aptamer of the present aspect comprises the base sequence of SEQ ID NO: 201, or the same base sequence as SEQ ID NO: 201 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 201 (5'-CGnCCAACCTCCACCAATCAAAGC-3') corresponds to the central region resulting from removing a stem region and a mini-hairpin sequence from the base sequence of a nucleic acid aptamer such as AptD2bb (Ys) in Example 9.

A nucleic acid aptamer that binds to a DEN2 protein comprises, as a base represented by n in the above-described base sequence, at least one, for example, one to three, for example, one, two, three, or four unnatural bases represented by the above-described general formula (I) and/or general formula (II).

In a further embodiment, a nucleic acid aptamer that binds to DEN2 protein may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to DEN2 protein is selected from the group consisting of (8-a) to (8-d) below:
(8-a) a nucleic acid aptamer comprising the base sequence of SEQ ID NO: 200 wherein, in said base sequence, the base represented by n at position 13 is base Bs;
(8-b) a nucleic acid aptamer comprising the same base sequence as SEQ ID NO: 200 except that one or multiple bases are deleted, substituted, and/or added except at position 13, wherein, in said base sequence, the positions corresponding to the base represented by n at position 13 in SEQ ID NO: 200 is base Ys or base Bs;
(8-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 201, and the base represented by n at position 3 in said base sequence of SEQ ID NO: 201 is base Ys or base Bs; and
(8-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 201 except that one or multiple bases are deleted, substituted, and/or added except at position 3, wherein, in said base sequence in said central region, the position corresponding to the base represented by n at position 3 of SEQ ID NO: 201 is base Ys or base Bs.

In this regard, the above-described SEQ ID NO: 200 is a sequence resulting from adding, to the 5' end side and 3' end side of SEQ ID NO: 201, the respective sequences that can form a stem structure, and are complementary with each other.

### <(9) Nucleic acid aptamer that binds to DEN3 protein>

In one further aspect of a nucleic acid aptamer of the present invention, a nucleic acid aptamer that binds to Dengue Virus NS1 Protein Serotype 3 (DEN3 protein) is provided.

The nucleic acid aptamer of the present aspect comprises the base sequence of SEQ ID NO: 39 or 202, or the same base sequence as SEQ ID NO: 39 or 202 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 202 (5'-TCTAnCCTGGCCnTGTGGTACTGTAACGGC-3') corresponds to the central region resulting from removing a stem region from the base sequence of a nucleic acid aptamer such as AptD3-YsDss in the result (3) in Example 3. The base sequence of SEQ ID NO: 39 (5'-TCYAnCCYGGCCnYGTGGYRYYGTARCGGY-3') is a sequence extended so as to further encompass the base sequences (SEQ ID NO: 46 to 54) having, introduced therein, the substitutions corresponding to the substitutions (T9C, T14C, T20C, T25C, A26G, C27T, T28C, A32G, and C36T) shown to be free from impairing the binding activity in Figure 2 (e) in the literature (Kimoto M., *et al., bioRxiv* 2022.06.27.497860).

A nucleic acid aptamer that binds to a DEN3 protein comprises, as a base represented by n in the above-described base sequence, at least one, for example, one to three, for example, one, two, three, or four unnatural bases represented by the above-described general formula (I) and/or general formula (II).

In a further embodiment, a nucleic acid aptamer that binds to DEN3 protein may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to DEN3 protein is selected from the group consisting of (9-a) to (9-d) below:
(9-a) a nucleic acid aptamer comprising the base sequence of SEQ ID NO: 38 or 40 wherein, in said base sequence, the bases represented by n at position 15 and position 23 respectively are (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(9-b) a nucleic acid aptamer comprising the same base sequence as SEQ ID NO: 38 or 40 except that one or multiple bases are deleted, substituted, and/or added except at position 15 and position 23, wherein, in said base sequence, the positions corresponding to the bases represented by n at position 15 and position 23 respectively in SEQ ID NO: 38 or 40 are (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(9-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 39 or 202, and the bases represented by n at position 5 and position 13 respectively in said base sequence of SEQ ID NO: 39 or 202 are (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm; and
(9-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 39 or 202 except that one or multiple bases are deleted, substituted, and/or added except at position 5 and position 13, wherein, in said base sequence in said central region, the positions corresponding to the bases represented by n at position 5 and position 13 respectively in SEQ ID NO: 39 or 202 are (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm.

In this regard, the above-described SEQ ID NOs: 38 and 40 are sequence resulting from adding, to the 5' end side and 3' end side of SEQ ID NOs: 39 and 202 respectively, sequences that can form a stem structure, and are complementary with each other. In addition, the base sequence of SEQ ID NO: 40 is encompassed in the base sequence of SEQ ID NO: 38, and has purine bases that are, and pyrimidine bases that are, specified as specific bases.

### <(10) Nucleic acid aptamer that binds to DEN4 protein>

In one further aspect of a nucleic acid aptamer of the present invention, a nucleic acid aptamer that binds to Dengue Virus NS1 Protein Serotype 4 (DEN4 protein) is provided.

The nucleic acid aptamer of the present aspect comprises the base sequence of SEQ ID NO: 204, or the same base sequence as SEQ ID NO: 204 except that a pair of base sequences complementary to each other, and capable of forming a stem structure are added to the 5' end side and 3' end side, wherein, in said base sequence, at least one of the bases represented by n is an unnatural base represented by the above-described general formula (I) and/or general formula (II). For example, at least one of the bases represented by n in said base sequence is any unnatural base represented by the formula (X) to the formula (XVII) and the formula (XXI) to the formula (XXII). In a case where two or more of the bases represented by n in said base sequence are any of said unnatural bases, each of the unnatural bases can be independently selected from the unnatural bases represented by the above-described general formula (I) and/or general formula (II), for example, the unnatural bases represented by the above-described general formula (X) to formula (XVII) and the above-described formula (XXI) to formula (XXII).

The base sequence of SEQ ID NO: 204 (5'-GACGTAACGCnTATCAAATCnAAACAGCTTAGGG-3') corresponds to the central region resulting from removing a stem region and a mini-hairpin sequence from the base sequence of a nucleic acid aptamer such as AptD4-2(YsYs) in Example 10.

A nucleic acid aptamer that binds to a DEN4 protein comprises, as a base represented by n in the above-described base sequence, at least one, for example, two, three, or four unnatural bases represented by the above-described general formula (I) and/or general formula (II).

In a further embodiment, a nucleic acid aptamer that binds to DEN4 protein may comprise Ds as at least one of the bases represented by n in said base sequence.

In one embodiment of the present aspect, an unnatural base represented by the above-described general formula (I) and/or general formula (II) is any of the bases represented by the above-described formula (X) to formula (XVII) and formula (XXI) to formula (XXII).

In a further embodiment of the present aspect, a nucleic acid aptamer that binds to DEN4 protein is selected from the group consisting of (10-a) to (10-d) below:
(10-a) a nucleic acid aptamer comprising the base sequence of SEQ ID NO: 203 wherein, in said base sequence, the bases represented by n at position 18 and position 28 respectively are (I) base Ys and base Ys, (II) base Ys and base Bs, (III) base Bs and base Ys, or (IV) base Bs and base Bs;
(10-b) a nucleic acid aptamer comprising the same base sequence as SEQ ID NO: 203 except that one or multiple bases are deleted, substituted, and/or added except at position 18 and position 28, wherein, in said base sequence, the positions corresponding to the bases represented by n at position 18 and position 28 respectively in SEQ ID NO: 203 are (I) base Ys and base Ys, (II) base Ys and base Bs, (III) base Bs and base Ys, or (IV) base Bs and base Bs;
(10-c) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the base sequence of SEQ ID NO: 204, and the bases represented by n at positions 11 and 21 respectively in said base sequence of SEQ ID NO: 204 are (I) base Ys and base Ys, (II) base Ys and base Bs, (III) base Bs and base Ys, or (IV) base Bs and base Bs; and
(10-d) a nucleic acid aptamer comprising a 5' region, a central region, and a 3' region in this order from the 5' side, wherein said 5' region and 3' region comprise a pair of base sequences complementary to each other, and capable of forming a stem structure, said central region comprises the same base sequence as SEQ ID NO: 204 except that one or multiple bases are deleted, substituted, and/or added except at positions 11 and 21, wherein, in said base sequence in said central region, the positions corresponding to the bases represented by n at positions 11 and 21 respectively in SEQ ID NO: 204 are (I) base Ys and base Ys, (II) base Ys and base Bs, (III) base Bs and base Ys, or (IV) base Bs and base Bs.

In this regard, the above-described SEQ ID NO: 203 is a sequence resulting from adding, to the 5' end side and 3' end side of SEQ ID NO: 204, the respective sequences complementary with each other, and capable of forming a stem structure.

### <Pharmaceutical composition>

In one aspect of the present invention, a pharmaceutical composition comprising any of the above-described nucleic acid aptamers is provided. A pharmaceutical composition of the invention comprises any one or more of the above-described nucleic acid aptamers. A pharmaceutical composition of the invention may comprise a combination of any two or more of the above-described nucleic acid aptamers. In addition, the pharmaceutical composition may comprise one or more other drugs to the extent of not losing the binding capacity of a nucleic acid aptamer of the present invention.

A disease to be prevented and/or treated with a pharmaceutical composition comprising the above-described (1) a nucleic acid aptamer that binds to vWF is a disease that can be caused by: the gene mutation, overexpression, or the like of vWF; the production of an autoantibody to vWF; or the like (the disease is hereinafter also referred to as a "vWF-associated disease"). Examples of the vWF-associated disease include thrombosis, thrombotic thrombocytopenic purpura, intracranial embolism, brain embolism, carotid artery stenosis, thrombotic microangiopathy, and acute myocardial infarction.

A disease to be prevented and/or treated with a pharmaceutical composition comprising the above-described (2) a nucleic acid aptamer that binds to IFNγ is a disease that needs the inhibition of the function of IFNγ (the disease is hereinafter also referred to as a "IFNγ-associated disease"). Examples of the IFNγ-associated disease include an autoimmune disease and an inflammatory disorder. The autoimmune disease or the inflammatory disorder may be, for example, Crohn's disease, systemic lupus erythematosus, psoriasis, rheumatoid arthritis, vasculitis, uveitis, atopic dermatitis, type I diabetes mellitus, multiple sclerosis, schizophrenia, cornea transplant rejection, alopecia areata, plaque psoriasis, vitiligo, pemphigus vulgaris, epidermolysis bullosa, acne vulgaris, herpes simplex virus type 1, or Hunner's interstitial cystitis.

A disease to be prevented and/or treated with a pharmaceutical composition comprising the above-described (3) a nucleic acid aptamer that binds to Thrombin is a disease that needs the inhibition of the function of Thrombin (the disease is hereinafter also referred to as a "Thrombin-associated disease"). Examples of the Thrombin-associated disease include thrombotic disorder. Examples of the thrombotic disorder include venous thrombosis (for example, deep venous thrombosis), pulmonary embolism, atrial fibrillation, myocardial infarction, arterial thrombosis, stroke (for example, thrombosis-induced stroke), atherosclerosis, and diffuse intravascular coagulation.

A disease to be prevented and/or treated with a pharmaceutical composition comprising the above-described (4) a nucleic acid aptamer that binds to VEGF is a disease that needs the inhibition of the function of VEGF (the disease is hereinafter also referred to as a "VEGF-associated disease"). Examples of the VEGF-associated disease include age-related macular degeneration, diabetic retinopathy, rheumatoid arthritis, heart disease preceded by myocardial infarction, wound healing, and cancers.

A disease to be prevented and/or treated with a pharmaceutical composition comprising the above-described (5) a nucleic acid aptamer that binds to HMGB1 is a disease that needs the inhibition of the function of HMGB1 (the disease is hereinafter also referred to as an "HMGB1-associated disease"). Examples of the HMGB1-associated disease include autoimmune disease and cardiovascular disease. Further specific examples include rheumatoid arthritis, inflammatory bowel disease, sepsis, cancer, lupus, Sjogren's syndrome, myocardial infarction, arteriosclerosis, stroke, cerebral infarction, cerebral edema, cerebral vasospasm, traumatic brain injury, atherosclerosis, neuropathic pain, arthritis, acute pulmonary trauma, cerebral ischemia, renal ischemia, and hepatic ischemia.

A disease to be prevented and/or treated with a pharmaceutical composition comprising the above-described (6) a nucleic acid aptamer that binds to Transferrin receptor 1 is a disease that needs the inhibition of the function of Transferrin receptor 1 (the disease is hereinafter also referred to as a "Transferrin receptor 1-associated disease"). Examples of Transferrin receptor 1-associated disease include neuropathy, iron deficiency anemia, iron overload disorder, cancers, neurodegenerative disease, and infectious diseases. Further specific examples include eye disease, seizure disorder, lysosomal storage disease, amyloidosis, viral infection, microbial infection, ischemia, conduct disorder, and CNS inflammation.

A disease to be prevented and/or treated using a pharmaceutical composition comprising the above-described (7) a nucleic acid aptamer that binds to DEN1 protein, (8) a nucleic acid aptamer that binds to DEN2 protein, (9) a nucleic acid aptamer that binds to DEN3 protein, and/or (10) a nucleic acid aptamer that binds to DEN4 protein is dengue virus infectious disease. Dengue virus infectious disease encompasses dengue fever and severe dengue (for example, dengue hemorrhagic fever and dengue shock syndrome).

A pharmaceutical composition of the present invention may comprise a pharmaceutically acceptable carrier. A "pharmaceutically acceptable carrier" refers to a substance that facilitates formulation of a pharmaceutical composition to be usually used in the technical field of formulation, and the application of the pharmaceutical composition to an organism, and is added to the extent of not inhibiting or suppressing the action of the pharmaceutical composition. Examples of carriers include an excipient, binder, disintegrant, filler, emulsifier, lubricant, an agent for lubricating, and stabilizer.

Examples of the "excipient" include; sugars, such as monosaccharides, disaccharides, cyclodextrin, and polysaccharides (specifically comprising, but not limited to, glucose, sucrose, lactose, raffinose, mannitol, sorbitol, inositol, dextrin, maltodextrin, starch, and cellulose), metal salts (for example, sodium phosphate, calcium phosphate, calcium sulfate, and magnesium sulfate); citric acid; tartaric acid; glycine; low-, middle-, and high-molecular-weight polyethylene glycols (PEG); Pluronic; and combinations thereof.

Examples of the "binder" include corn, wheat, rice, starch paste produced using potato starch, gelatin, tragacanth, methylcellulose, hydroxypropylmethylcellulose, carboxymethylcellulose sodium, and/or polyvinylpyrrolidone.

Examples of the "disintegrant" include said starch, carboxymethyl starch, cross-linked polyvinylpyrrolidone, agar, alginic acid, alginic acid sodium, and salts thereof.

Examples of the "filler" include said sugar and/or calcium phosphate (for example, tricalcium phosphate or calcium hydrogenphosphate).

Examples of the "emulsifier" include a sorbitan fatty acid ester, glycerin fatty acid ester, sucrose fatty acid ester, and propylene glycol fatty acid ester.

Examples of the "lubricant" and the "agent for lubricating" include a silicic acid salt, talc, stearic acid salt, and polyethylene glycol.

Examples of the "stabilizer" include; agents for preventing oxidation, such as ascorbic acid and sulfite; and sugars, such as trehalose and dextrose.

Such a carrier may be suitably used, if desired. If desired, a pharmaceutical composition of the present invention may comprise, besides the above-described additives, flavoring agents, dissolution aids (solubilizers), suspending agents, diluents, surfactants, absorption promoters (e.g., quaternary ammonium salts, sodium lauryl sulfate, etc.), bulking agents, wetting agents, moisturizing agents (e.g., glycerin, starch, etc.), adsorbents (e.g., starch, lactose, kaolin, bentonite, colloidal silicic acid, etc.), disintegration inhibitors (e.g., sucrose, stearin, cocoa butter, hydrogenated oil, etc.), coating agents, coloring agents, preservatives, fragrances, flavorings, sweeteners, buffers, isotonicity agents, soothing agents, dissolution aids.

Examples of the "surfactant" include: an alkali metal salt, alkaline earth metal salt, and ammonium salt each of lignosulfonic acid, naphthalenesulfonic acid, phenolsulfonic acid, or dibutylnaphthalenesulfonic acid; alkylarylsulfonate; alkylsulfate; alkylsulfonate; fatty alcohol sulfate; fatty acid; and sulfated fatty alcohol glycol ether; and furthermore, a condensate of sulfonated naphthalene or a naphthalene derivative and formaldehyde; a condensate of naphthalene or naphthalenesulfonic acid and phenol or formaldehyde; polyoxyethyleneoctylphenyl ether; ethoxylated isooctylphenol; octylphenol; nonylphenol; alkylphenyl polyglycol ether; tributylphenyl polyglycol ether; tristearylphenyl polyglycol ether; alkylaryl polyether alcohol; a condensate of alcohol and fatty alcohol or ethylene oxide; ethoxylated castor oil; polyoxyethylene alkyl ether; ethoxylated polyoxypropylene; lauryl alcohol polyglycol ether acetal; sorbitol ester; lignosulfite waste liquor; and methylcellulose.

A pharmaceutical composition of the present embodiment may encompass one or more of the above-described carriers per pharmaceutical composition.

The dosage form of a pharmaceutical composition of the present invention is not particularly limited, subject to being a form that does not allow inactivation of an active ingredient, and allows achieving the pharmacological effect in an organism after administration. The dosage form usually depends on an administration method and/or a prescribing condition.

Examples of a dosage form suitable for oral administration include solid preparations (comprising tablets, pills, lingusorbs, capsules, drops, and troches), granules, dusting powder, powder, and liquid preparations. Furthermore, if desired, the solid preparation can take a coated dosage form known in the art, for example, a sugar-coated tablet, gelatin-coated tablet, enteric-coated tablet, film-coated tablet, bilayered tablet, or multilayered tablet.

The parenteral administration is subclassified into systemic administration and topical administration, and the topical administration is further subclassified into interstitial administration, transepidermal administration, transmucosal administration, and transrectal administration. The pharmaceutical composition can take a dosage form suitable for each administration method. Examples of a dosage form suitable for systemic or interstitial administration include an injection that is a liquid preparation. Examples of a dosage form suitable for transepidermal administration or transmucosal administration include liquid preparations (comprising liniments, eyedrops, nasal drops, and inhalants), agents for suspending (comprising emulsions and creams), dusting powders (comprising nasal drops and inhalants), pastes, gels, ointments, and plasters. Examples of a dosage form suitable for transrectal administration include suppositories.

In this regard, the specific shape and size of each of the above-described dosage forms are not particularly limited. Any of the dosage forms may be in the scope of a dosage form known in the art.

To produce a pharmaceutical composition of the present invention, a formulation method known in the art may be applied, in principle. For example, the method described in Remington's Pharmaceutical Sciences (Merck Publishing Co., Easton, Pa.) can be used as a reference.

For example, in the case of an injection, a nucleic acid aptamer of the present invention is dissolved in a pharmaceutically acceptable solvent, a pharmaceutically acceptable carrier is added, if desired, and a method commonly used in the art can be used to produce an injection.

Examples of the "pharmaceutically acceptable solvent" include water, ethanol, propylene glycol, ethoxylated isostearyl alcohol, polyoxylated isostearyl alcohol, and polyoxyethylene sorbitan fatty acid esters. If desired, these are preferably adjusted to be isotonic with blood.

A pharmaceutical composition of the present invention can be administered in a pharmaceutically effective amount to an organism for the purpose of treating or preventing an object disease. An organism as an object of administration is a vertebrate, preferably a mammal, more preferably a human.

A pharmaceutical composition of the present invention may be administered through any of systemic administration and topical administration. Any of these can be suitably selected in accordance with the kind of a disease, the site of onset, the degree of progression, or the like. For a disease at the site of onset of which is local, it is preferable to use topical administration in which direct administration is performed, through injection or the like, on the site of onset and an area around the site. This is because topical administration enables a nucleic acid aptamer of the present invention to be administered in a sufficient amount to a site (tissue or organ) to be treated, and in addition, is less prone to affect another tissue. On the other hand, in a case where a site to be treated is unidentifiable, or in a case where the onset of a disease is systemic, systemic administration through intravenous injection or the like is preferable, without limitation. This is because systemic administration allows a nucleic acid aptamer of the present invention to spread systemically via a blood flow, thus enabling the nucleic acid aptamer to be administered even to a lesion site undetectable by diagnosis.

A pharmaceutical composition of the present invention can be administered using a suitable method which does not deactivate an active ingredient. The administration may be, for example, parenteral (for example, with injection, aerosol, application, eye drops, or nasal drops) or oral. Injection is preferable.

In the case of administration by injection, the site of injection is not particularly limited. The site may be any site, subject to enabling a DNA aptamer as an active ingredient to bind to a target substance. The site is, for example, an intravenous, intraarterial, intrahepatic, intramuscular, intraarticular, intramedullary, intrathecal, intraventricular, pulmonary, percutaneous, subcutaneous, intracutaneous, or intraperitoneal site.

### <Method for treating and/or preventing>

In one aspect, a method for treating and/or preventing a disease, comprising administering a nucleic acid aptamer of the present invention or the above-described pharmaceutical composition to a test subject, is provided.

Herein, an animal species that can be a "test subject" is, for example: a mammal, for example, a primate, such as a human or a chimpanzee: a laboratory animal, such as a rat or a mouse; a livestock animal, such as a pig, bovine, horse, sheep, or goat; or a pet animal, such as a dog or a cat. The test subject is preferably a human.

### <Agent for detecting>

In one aspect of the present invention, an agent for detecting is also provided. An agent for detecting according to the present invention is a drug for detecting a target protein *in vivo* or *in vitro* by utilizing the binding capacity of a nucleic acid aptamer of the present invention to the target protein. The target protein is vWF, IFNγ, Thrombin, VEGF, HMGB1, Transferrin receptor 1, DEN1 protein, DEN2 protein, DEN3 protein, or DEN4 protein. For example, preliminarily labeling a nucleic acid aptamer with a fluorescent reagent or the like, followed by administering the resulting aptamer, makes it possible to determine the intensity of expression of any of the above-described target proteins in an organism, and in addition, to examine the localization of the target protein. This makes it possible to assist in diagnosing the above-described diseases. A nucleic acid aptamer of the present invention is useful also in imaging, tissue stain, and the like.

In one embodiment, the present invention relates to a composition for detecting a target protein, in which the composition comprises a nucleic acid aptamer of the present invention. The constitution of the composition is the same as the constitution of the above-described pharmaceutical composition, and thus, the description is omitted here.

In one aspect, the present invention relates to a kit for detecting a target protein, in which the kit comprises a nucleic acid aptamer of the present invention. A kit of the present invention may comprise a buffer, labeling reagent, and/or manual, in addition to a nucleic acid aptamer of the present invention.

### <Detecting method>

In one aspect, the present invention relates to a method for detecting any of the above-described target proteins. The present method comprises: a step of bringing a sample obtained from a test subject, into contact with a nucleic acid aptamer of the present invention; and a step of detecting any of the above-described target proteins on the basis of binding between the sample and the nucleic acid aptamer. The present method makes it possible to assist in diagnosing diseases associated with the above-described target proteins.

Examples of a sample to be used in the present method include tissues and biological samples. Examples of the tissue include a lesion site, brain, heart, liver, pancreas, lung, bone marrow, lymph node, and spleen. For example, a biopsy sample of such a tissue can be used. Examples of the biological sample include: bodily fluids (for example, blood, blood plasma, lymph liquid, tissue liquid, or urine); and cells, for example, peripheral blood cells, hair matrix cells, oral-cavity cells, nasal-cavity cells, intestinal cells, intravaginal cells, mucosal cells, and sputa (that may comprise alveolar cells, tracheal cells, or the like).

A detecting step in a detecting method of the present invention is not particularly limited, subject to utilizing binding between a sample and a nucleic acid aptamer. A known method may be used. For example, an SPR method, quartz crystal microbalance method, nephelometric method, colorimetric method, or fluorescence method can be utilized.

SPR (surface plasmon resonance) refers to a phenomenon in which, on a thin metal film irradiated with a laser beam, the intensity of reflected light is markedly attenuated at a specific incidence angle (resonance angle). The SPR method is a measuring method to be performed utilizing this phenomenon. An adsorbate on a sensor portion, i.e., the surface of a thin metal film, can be measured with high sensitivity. In the present invention, for example, a nucleic acid aptamer of the present invention is preliminarily fixed on the surface of a thin metal film, a sample is allowed to pass on the surface of the thin metal film, a difference in an adsorbate on the surface of the metal between before and after the passing of the sample is generated by binding between the nucleic acid molecule and a target substance, and the difference is detected, whereby the target substance in the sample can be detected. As the SPR method, a substitution method, an indirect competition method, and the like are known. Any of these may be used.

The QCM (Quartz Crystal Microbalance) method is a method to be performed utilizing a phenomenon in which a substance is adsorbed in the surface of an electrode attached to a quartz oscillator, with the result that the resonance frequency of the quartz oscillator is decreased in accordance with the mass of the substance. A QCM sensor based on using this method can quantitatively seize a trace amount of adsorbate in accordance with the quantity of change in the quartz resonance frequency. In the present invention, a DNA aptamer is preliminarily fixed on the surface of an electrode in the same manner as in said SPR method, and a sample was brought into contact with the surface of the electrode, whereby a target substance in the sample can be quantitatively detected in accordance with the quantity of change caused in the quartz resonance frequency by binding between the DNA aptamer and the target substance. This technology is well known in the art. For example, Christopher J., et al. (2005) Self-Assembled Monolayers of a Form of Nanotechnology, Chemical Review, 105: 1103-1169 may be used as a reference.

A nephelometric method is a method in which a solution is irradiated with light, the attenuation of the light scattered by a substance floating in the solution is optically measured, or the light transmitted by the solution is optically measured, using a colorimeter or the like to measure the amount of the substance in the solution. In the present invention, measuring the absorbance before and after adding a nucleic acid aptamer of the present invention to a sample makes it possible to quantitatively detect the target substance in the sample.

In addition, an antibody to a target substance is used together to enable the target substance to be detected. For example, a method in which the sandwich method of ELISA is applied may be used. In this method, a nucleic acid aptamer of the present invention is first fixed on a solid-phase carrier, and next, a sample is added so that a target substance present in the sample can be bound to said nucleic acid aptamer. Subsequently, the sample was rinsed, and then, an anti-target-substance antibody is added to bind to the target substance. The resulting sample is washed. Then, using a secondary antibody suitably labeled, the anti-target-substance antibody is detected, whereby the target substance in the sample can be detected. Examples of the solid-phase carrier that can be used include an insoluble carrier in the shape of beads, a microplate, test tube, stick, test piece, or the like consisting of a material, such as polystyrene, polycarbonate, polyvinyl toluene, polypropylene, polyethylene, polyvinyl chloride, nylon, polymethacrylate, latex, gelatin, agarose, cellulose, Sepharose, glass, metal, ceramics, or magnetic material.

In addition, an antibody to a target substance is used together to enable the target substance to be detected. For example, a method in which the sandwich method of ELISA is applied may be used.

In a case where a target protein is detected using the detecting step of the present aspect, a test subject from which a sample is derived is revealed to have a disease associated with the target protein. For example, in a case where DEN1 to DEN4 proteins are detected using nucleic acid aptamers that bind to the DEN1 to DEN4 proteins in the present invention, a test subject is revealed to have dengue viruses serotype 1 to serotype 4 respectively.

In one further aspect of the present invention, any of the above-described nucleic acid aptamers of the present invention is also provided for use in the treatment and/or prevention of a disease in a test subject such as a human. Furthermore, the present invention provides any of the above-described nucleic acid aptamers of the present invention in the production of a drug for the treatment and/or prevention of a disease.

In one further aspect, the present invention relates to a method for assisting in diagnosing any or no contraction of a disease in a test subject. The present method comprises: a step of administering a nucleic acid aptamer of the present invention to a test subject; and a step of detecting the nucleic acid aptamer. For example, in a case where a nucleic acid aptamer at a high concentration has been detected at a specific site in an organism, it can be judged that a disease has been generated at the site. As the detecting step, a known method may be used. For example, the above-described fluorescence method may be used.

### Examples

### <Example 1: Chemical synthesis of Ds alternative>

### (Purpose)

The purpose was to modify the structure of a highly hydrophobic artificial base 7-(2-thienyl)imidazo[4,5-b]pyridine (hereinafter referred to as "Ds") to produce a new artificial base (hereinafter referred to as a "Ds alternative"). The highly hydrophobic Ds base was composed of two modules: a 1-deazapurine(imidazo[4,5-b]pyridine) portion and a thienyl side chain. Here, various Ds alternatives (Bs, Is, Ys, Yi, Do, Dp, Dt, Yo, Ps, Es, Ysm, Dss, Yss, Bss, and Bo) shown in Figure 2 were synthesized by substituting the 1-deazapurine portion with pyrrolo[2,3-*b*]pyridine, purine, indole, benzo[*d*]imidazole, or pyrrolo[2,3-*d*]pyrimidine, and substituting the thienyl side chain with furanyl, imidazolyl, thiazolyl, pyridazinyl, and a modified structure thereof (for example, a methylthienyl or bithiophenyl portion).

### (Method and results)

Among Ds and the Ds alternatives shown in Figure 2, unnatural amidites dDs, dDss, and dPs (also referred to as "s'") were produced using the method described in the past literature (Hirao, I., et al., Nat. Methods, 2006, 3: 729-735.; Kimoto, M., et al., J. Am. Chem. Soc., 2010, 132:4988-4989.; and Fujiwara, T., et al., Bioorg. Med. Chem. Lett., 2001, 11: 2221-2223). The unnatural amidites dDt, dYi, dDp, and dDo were synthesized using the same method. A method for chemically synthesizing other unnatural amidites are described in the sections on chemical synthesis. The Ds alternatives other than the above were synthesized using the following method.

### (1) Chemical synthesis of dBs amidite

The compounds 2 to 6 corresponding to (2) to (6) in the reaction path scheme shown below were synthesized using the following method.

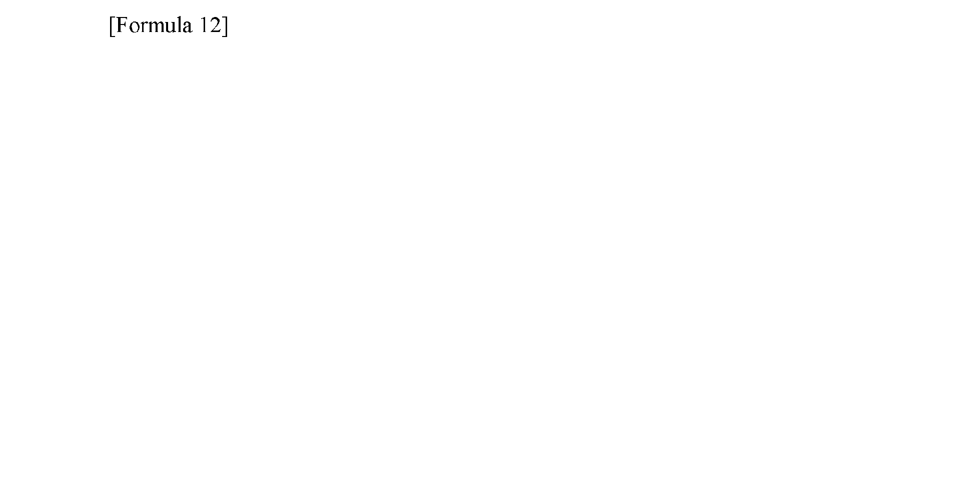

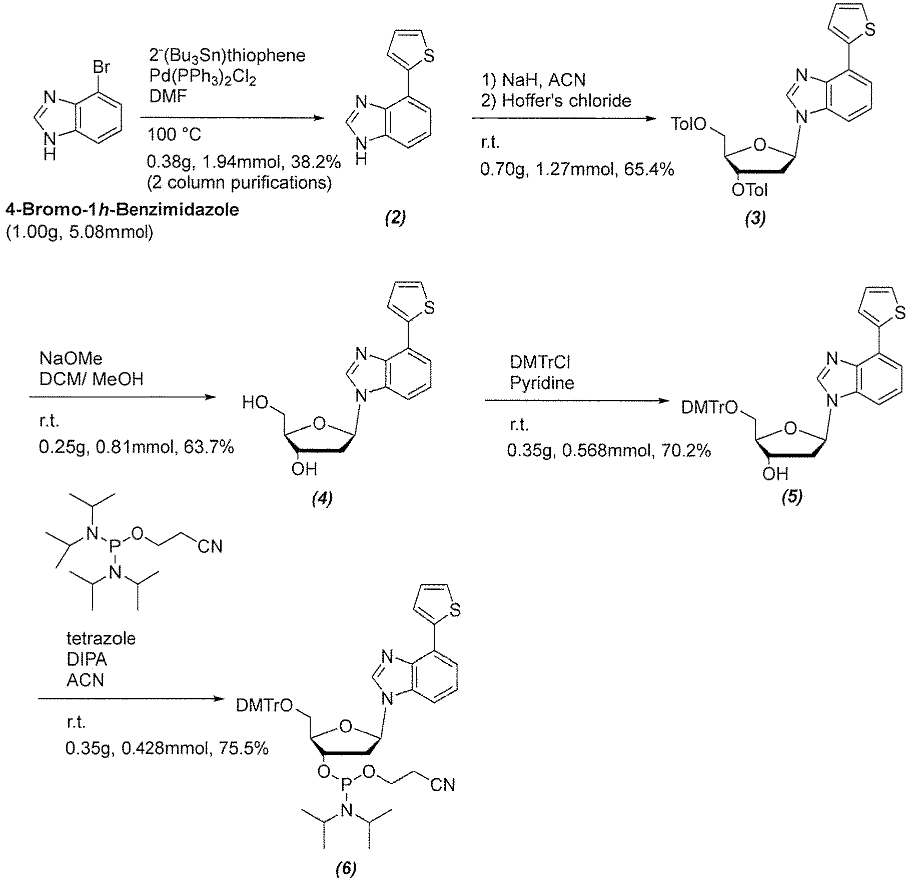

### Compound 2: 4-(thiophene-2-yl)-1H-benzo[d]imidazole

A mixture of 4-bromo-1h-benzimidazole (1.00 g, 5.08 mmol) and Pd(PPh₃)₂Cl₂ (0.357 g, 0.508 mmol) in DMF (25 mL) was degassed in vacuo, and the gas phase was replaced with argon. After 10 minutes, 2-(tributylstannyl)thiophene (1.97 mL, 6.10 mmol) was added. Then, the resulting mixture was further degassed under argon for 10 minutes, and stirred at 100°C for 6 hours. The reaction mixture was subjected to liquid extraction using brine and ethyl acetate. The organic layer was concentrated in vacuo to obtain a crude product. The crude product was subjected to two steps of silica gel column chromatography, using a hexane/EtOAc solvent (20 to 50% EtOAc in hexane). Then, a compound 2 as a mixture with an unreacted starting material was obtained in the form of an off-white solid (0.39 g, 1.94 mmol, 38.2%).

### Compound 3: methyl ((2R,3S,5R)-3-((4-methylbenzoyl)oxy)-5-(4-(thiophene-2-yl)-1H-benzo[d]imidazole-1-yl)tetrahydrofuran-2-yl)4-methylbenzoate

The compound 2 (0.38 g, 1.94 mmol) was dissolved in ACN (25 mL), and sodium hydride (60%) (0.093 g, 2.33 mmol) was added. The resulting mixture was stirred at ambient temperature for 20 minutes. Then, Hoffer chloride (0.91 g, 2.33 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography performed using hexane/EtOAc (20% to 30% EtOAc in hexane) as a solvent, whereby a compound 3 as a product of interest was obtained in the form of a colorless oil (0.70 g, 1.27 mmol, 65.4%). ¹H NMR (DMSO-d₆, 400 MHz) δ, ppm: 8.58 (s, 1H), 8.07 (dd, 1H, J = 1.2, 3.6 Hz), 8.00 (d, 2H, J = 8.2 Hz), 7.84 (d, 2H, J = 8.2 Hz), 7.66 (dd, 1H, J = 0.8, 8.1 Hz), 7.60 - 7.58 (m, 2H), 7.39 (d, 2H, J = 8.0 Hz), 7.31 (d, 2H, J = 8.0 Hz), 7.19 - 7.15 (m, 2H), 6.64 - 6.60 (m, 1H), 5.76 - 5.74 (m, 1H), 4.66 - 4.54 (m, 3H), 2.84 - 2.78 (m, 1H), 2.42, 2.36 (2s, 6H), 2.33 - 2.32 (m, 1H).

### Compound 4: (2R,3S,5R)-2-(hydroxymethyl)-5-(4-(thiophene-2-yl)-1H-benzo[d]imidazole-1-yl)tetrahydrofuran-3-ol

To a solution of the compound 3 (0.70 g, 1.27 mmol) in DCM/MeOH (1:1) (9.6 mL), sodium methoxide (5 M in MeOH) (0.660 mL, 3.30 mmol) was added, and the resulting solution was stirred at ambient temperature for 30 minutes. The solvent was removed in vacuo to obtain a crude product. The crude product was subjected to silica gel column chromatography (0 to 6% MeOH in DCM) and then C18 RP-HPLC (isocratic elution with 35% CH₃CN in H2O). Then, a compound 4 was isolated in the form of a white solid (0.31 g, 0.97 mmol, 76.9%). ¹H NMR (DMSO-d₆, 400 MHz) δ, ppm: 8.57 (s, 1H), 8.09 (dd, 1H, J = 1.2, 3.6 Hz), 7.65 - 7.58 (m, 3H), 7.29 (t, 1H, J = 7.8 Hz), 7.18 (dd, 1H, J = 3.6, 5.1 Hz), 6.41 - 6.37 (m, 1H), 5.36 (d, 1H, J = 4.2 Hz), 4.98 (t, 1H, J = 5.3 Hz), 4.43 - 4.39 (m, 1H), 3.90 - 3.87 (m, 1H), 3.61 - 3.52 (m, 2H), 2.66 -2.59 (m, 1H), 2.37 - 2.31 (m, 1H).

### Compound 5: (2R,3S,5R)-2-((bis(4-methoxyphenyl)(phenyl)methoxy)methyl)-5-(4-(thiophene-2-yl)-1H-benzo[d]imidazole-1-yl)tetrahydrofuran-3-ol

The compound 4 (0.25 g, 0.81 mmol) was evaporated together with pyridine (three times) before pyridine (1.0 mL) and DMTrCl (0.27 g, 0.81 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 100% EtOAc in hexane) (1% TEA), whereby a compound 5 as a product of interest was obtained in the form of a colorless oil (0.35 g, 0.57 mmol, 70.2%). ¹H NMR (DMSO-d₆, 400 MHz) δ, ppm: 8.50 (s, 1H), 8.10 (dd, 1H, J = 1.1, 3.6 Hz), 7.66 - 7.58 (m, 3H), 7.31 - 7.13 (m, 11H), 6.77 - 6.71 (m, 4H), 6.44 (t, 1H, J = 6.2 Hz), 5.43 (d, 1H, J = 4.8 Hz), 4.48 - 4.43 (m, 1H), 4.00 - 3.99 (m, 1H), 3.67, 3.65 (2s, 6H), 3.17 - 3.08 (m, 2H), 2.84 - 2.77 (m, 1H), 2.45 - 2.39 (m, 1H).

### Compound 6: (2R,3S,5R)-2-((bis(4-methoxyphenyl)(phenyl)methoxy)methyl)-5-(4-(thiophene-2-yl)-1H-benzo[d]imidazole-1-yl)tetrahydrofuran-3-yl(2-cyanoethyl)diisopropylphosphoramidite

The compound 5 (0.35 g, 0.57 mmol) and 1*H*-tetrazole in ACN (0.4 M) (0.653 mL, 0.26 mmol) were evaporated (three times) together with ACN before ACN (14.5 mL), DIPA (0.037 mL, 0.26 mmol), and 2-cyanoethyl *N,N,N',N'*-tetraisopropylphosphorodiamidite (0.288 mL, 0.91 mmol) were added. The reaction mixture was stirred at ambient temperature for 3 hours. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 40% EtOAc in hexane) (1% TEA). Then, a compound 6 was obtained in the form of a foamy white solid (0.35 g, 0.43 mmol, 75.5%). ¹H NMR (DMSO-d₆, 400 MHz) δ, ppm: 8.49 (d, 1H, J = 2.6 Hz), 8.10 - 8.09 (m, 1H), 7.67 - 7.58 (m, 3H), 7.33 - 7.15 (m, 11H), 6.80 - 6.74 (m, 4H), 6.49 - 6.45 (m, 1H), 4.74 - 4.68 (m, 1H), 4.18 - 4.10 (m, 1H), 3.79 - 3.50 (m, 10H), 3.28 - 3.14 (m, 2H), 2.95 - 2.89 (m, 1H), 2.78 (t, 1H, J = 5.9 Hz), 2.66 (t, 1H, J = 5.9 Hz), 1.15 - 1.11 (m, 10H), 1.01 - 1.00 (m, 2H). ³¹P NMR (DMSO-d₆, 162 MHz) δ, ppm: 148.02, 147.32 (diastereoisomer).

### (2) Chemical synthesis of dIs amidite

The compounds 2 to 6 corresponding to (2) to (6) in the reaction path scheme shown below were synthesized using the following method.

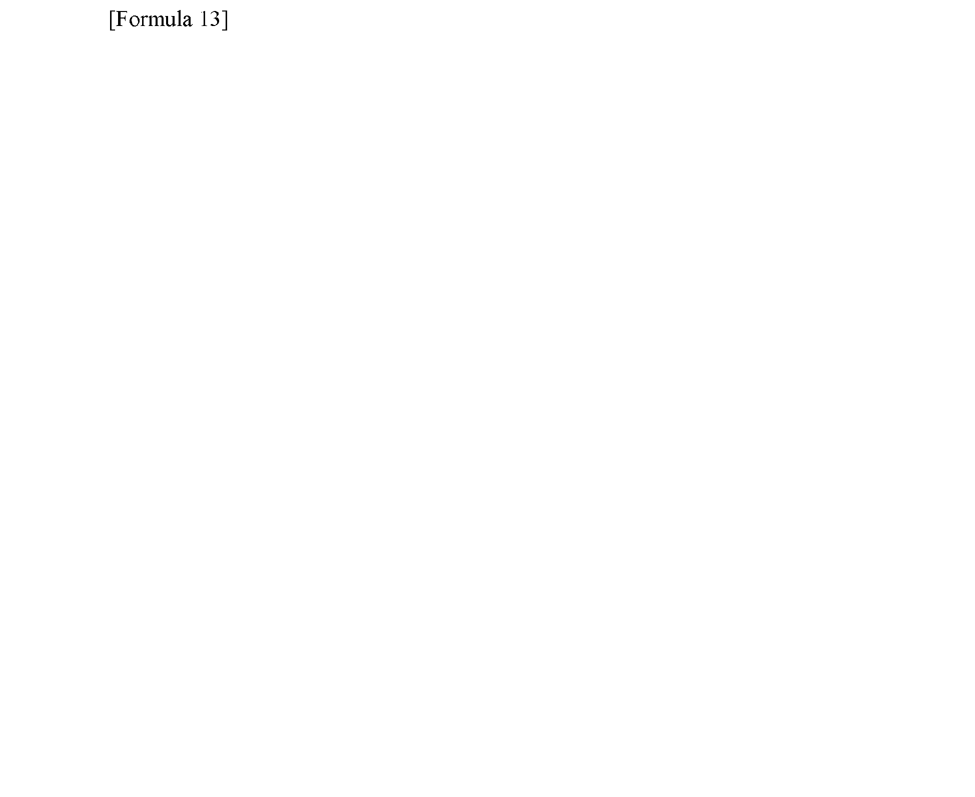

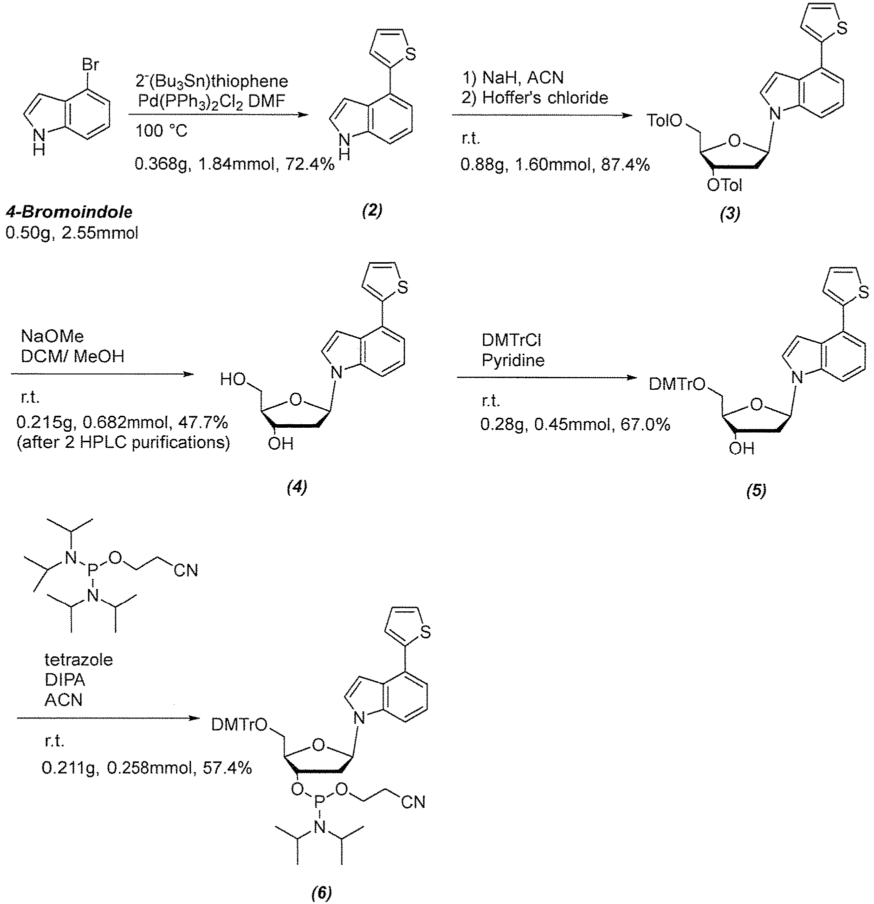

### Compound 2: 4-(thiophene-2-yl)-1H-indole

A mixture of 4-bromoindole (0.50 g, 2.55 mmol) and Pd(PPh₃)₂Cl₂ (0.179 g, 0.255 mmol) in DMF (13 mL) was degassed in vacuo, and the gas phase was replaced with argon for 10 minutes. After 2-(tributylstannyl)thiophene (0.989 mL, 3.06 mmol) was added, the resulting mixture was further degassed under argon for 10 minutes, and stirred at 100°C for 3 hours. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was subjected to silica gel column chromatography using a hexane/EtOAc solvent (5 to 100% EtOAc in hexane). Then, a compound 2 was obtained in the form of a brown oil (0.36 g, 1.84 mmol, 72.4%). ¹H NMR (DMSO-d₆, 400 MHz) δ, ppm: 11.47 (bs, 1H), 7.57 (dd, 1H, J = 1.2, 5.1 Hz), 7.52 (dd, 1H, J = 1.2, 3.6 Hz), 7.46 (t, 1H, J = 2.8 Hz), 7.39 (dt, 1H, J = 0.9, 8.1 Hz), 7.26 (dd, 1H, J = 0.9, 7.3 Hz), 7.19 (dd, 1H, J = 3.6, 5.1 Hz), 7.16 - 7.12 (m, 1H), 6.80 - 6.79 (m, 1H).

### Compound 3: methyl ((2R,3S,5R)-3-((4-methylbenzoyl)oxy)-5-(4-(thiophene-2-yl)-1H-indole-1-yl)tetrahydrofuran-2-yl)4-methylbenzoate

To a mixture of the compound 2 (0.36 g, 1.84 mmol) dissolved in ACN (24 mL), sodium hydride (60%) (0.088 g, 2.21 mmol) was added. The resulting mixture was stirred at ambient temperature for 20 minutes. Then, Hoffer chloride (0.86 g, 2.21 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (5 to 10% EtOAc in hexane), whereby a compound 3 as a product of interest was obtained in the form of a light yellow oil (0.89 g, 1.60 mmol, 87.4%). ¹H NMR (DMSO-d₆, 400 MHz) δ, ppm: 8.02 -7.98 (m, 2H), 7.89 - 7.86 (m, 3H), 7.73 - 7.68 (m, 2H), 7.60 (dd, 1H, J = 1.1, 5.1 Hz), 7.51 (dd, 1H, J = 1.1, 3.6 Hz), 7.40 - 7.31 (m, 6H), 7.20 (dd, 1H, J = 3.6, 5.1 Hz), 7.15 (t, 1H, J = 7.8 Hz), 6.88 (d, 1H, J = 3.4 Hz), 6.67 - 6.59 (m, 1H), 5.73 - 5.70 (m, 1H), 4.63 - 4.50 (m, 3H), 3.04 - 2.97 (m, 1H), 2.74 - 2.69 (m, 1H), 2.42, 2.38 (2s, 6H).

### Compound 4: (2R,3S,5R)-2-(hydroxymethyl)-5-(4-(thiophene-2-yl)-1H-indole-1-yl)tetrahydrofuran-3-ol

To a stirred solution of the compound 3 (0.88 g, 1.60 mmol) in DCM/MeOH (1:1) (12.2 mL), sodium methoxide (5 M in MeOH) (0.832 mL, 4.16 mmol) was added, and the resulting solution was stirred at ambient temperature for 30 minutes. The solvent was removed in vacuo to obtain a crude product. The crude product was subjected to silica gel column chromatography (0 to 5% MeOH in DCM), subsequently C18 RP-HPLC (isocratic elution with 40% CH₃CN in H₂O), and repurification by C18 RP-HPLC (isocratic elution with 45% CH₃CN in H₂O). Then, a compound 4 was isolated in the form of a white solid (0.22 g, 0.682 mmol, 47.7%). ¹H NMR (DMSO-d₆, 400 MHz) δ, ppm: 7.71 (d, 1H, J = 3.5 Hz), 7.60 - 7.58 (m, 2H), 7.51 (dd, 1H, J = 1.1, 3.6 Hz), 7.31 (dd, 1H, J = 0.8, 7.4 Hz), 7.22 - 7.18 (m, 2H), 6.86 (d, 1H, J = 3.4 Hz), 6.44 - 6.40 (m, 1H), 5.30 (d, 1H, J = 4.3 Hz), 4.90 (t, 1H, J = 5.4 Hz), 4.38 - 4.34 (m, 1H), 3.85 - 3.82 (m, 1H), 3.58 - 3.47 (m, 2H), 2.55 - 2.52 (m, 1H), 2.28 - 2.23 (m, 1H).

### Compound 5: (2R,3S,5R)-2-((bis(4-methoxyphenyl)(phenyl)methoxy)methyl)-5-(4-(thiophene-2-yl)-1H-indole-1-yl)tetrahydrofuran-3-ol

The compound 4 (0.21 g, 0.68 mmol) was evaporated together with pyridine (three times) before pyridine (1.0 mL) and DMTrCl (0.23 g, 0.68 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 50% EtOAc in hexane) (1% TEA), whereby a compound 5 as a product of interest was obtained in the form of a light yellow oil (0.28 g, 0.45 mmol, 67.0%). ¹H NMR (DMSO-d₆, 400 MHz) δ, ppm: 7.64 (d, 1H, J = 8.3 Hz), 7.60 - 7.58 (m, 2H), 7.51 (dd, 1H, J = 1.1, 3.6 Hz), 7.35 - 7.32 (m, 3H), 7.25 - 7.16 (m, 9H), 6.83 (d, 1H, J = 3.1 Hz), 6.80 - 6.76 (m, 4H), 6.46 (t, 1H, J = 6.4 Hz), 5.38 (d, 1H, J = 4.7 Hz), 4.43 - 4.38 (m, 1H), 3.96 - 3.95 (m, 1H), 3.69, 3.68 (2s, 6H), 3.13 - 3.11 (m, 2H), 2.63 - 2.59 (m, 1H), 2.37 - 2.30 (m, 1H).

### Compound 6: (2R,3S,5R)-2-((bis(4-methoxyphenyl)(phenyl)methoxy)methyl)-5-(4-(thiophene-2-yl)-1H-indole-1-yl)tetrahydrofuran-3-yl(2-cyanoethyl)diisopropylphosphoramidite

The compound 5 (0.28 g, 0.45 mmol) and 1*H*-tetrazole in acetonitrile (ACN) (0.4 M) (0.518 mL, 0.21 mmol) were evaporated (three times) together with ACN before ACN (11.5 mL), DIPA (0.029 mL, 0.21 mmol), and 2-cyanoethyl *N,N,N',N'-*tetraisopropylphosphorodiamidite (0.228 mL, 0.72 mmol) were added. The reaction mixture was stirred at ambient temperature for 3 hours. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 20% EtOAc in hexane) (1% TEA), whereby a compound 6 was obtained in the form of a foamy white solid (0.21 g, 0.26 mmol, 57.4%). ¹H NMR (DMSO-d₆, 400 MHz) δ, ppm: 7.65 (dd, 1H, J = 3.1, 8.3 Hz), 7.60 - 7.59 (m, 2H), 7.51 (dd, 1H, J = 1.0, 3.6 Hz), 7.37 - 7.32 (m, 3H), 7.26 - 7.16 (m, 9H), 6.86 - 6.77 (m, 5H), 6.51 - 6.46 (m, 1H), 4.71 - 4.65 (m, 1H), 4.13 - 4.05 (m, 1H), 3.72 - 3.52 (m, 10H), 3.28 - 3.13 (m, 2H), 2.79 - 2.75 (m, 2H), 2.67 (t, 1H, J = 5.9 Hz), 1.16 - 1.11 (m, 10H), 1.02 - 1.01 (m, 2H). ³¹P NMR (DMSO-d₆, 162 MHz) δ, ppm: 147.81, 147.16 (diastereoisomer).

### (3) Chemical synthesis of dYs amidite

The compounds 2 to 6 corresponding to (2) to (6) in the reaction path scheme shown below were synthesized using the following method.

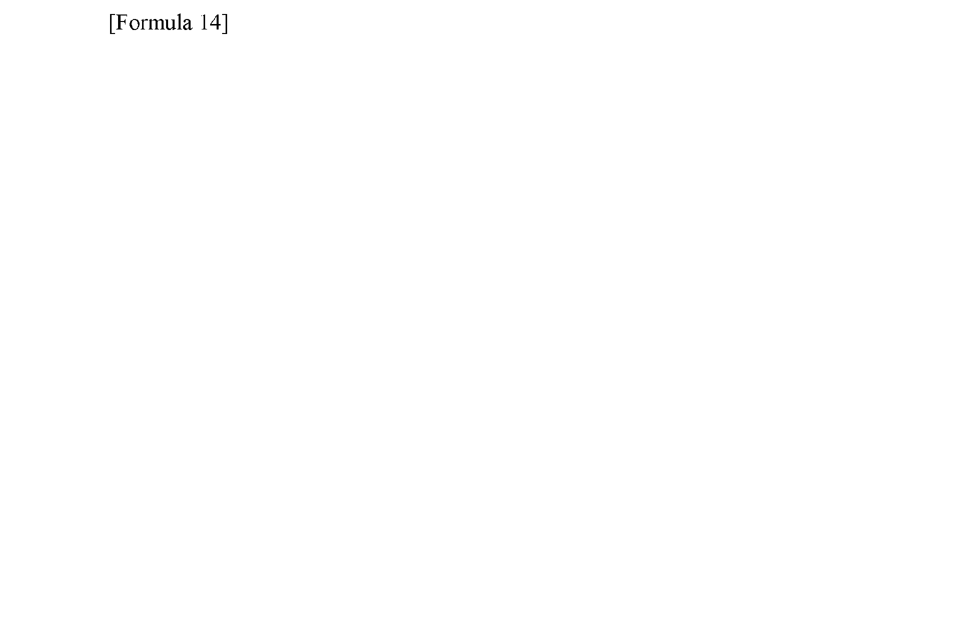

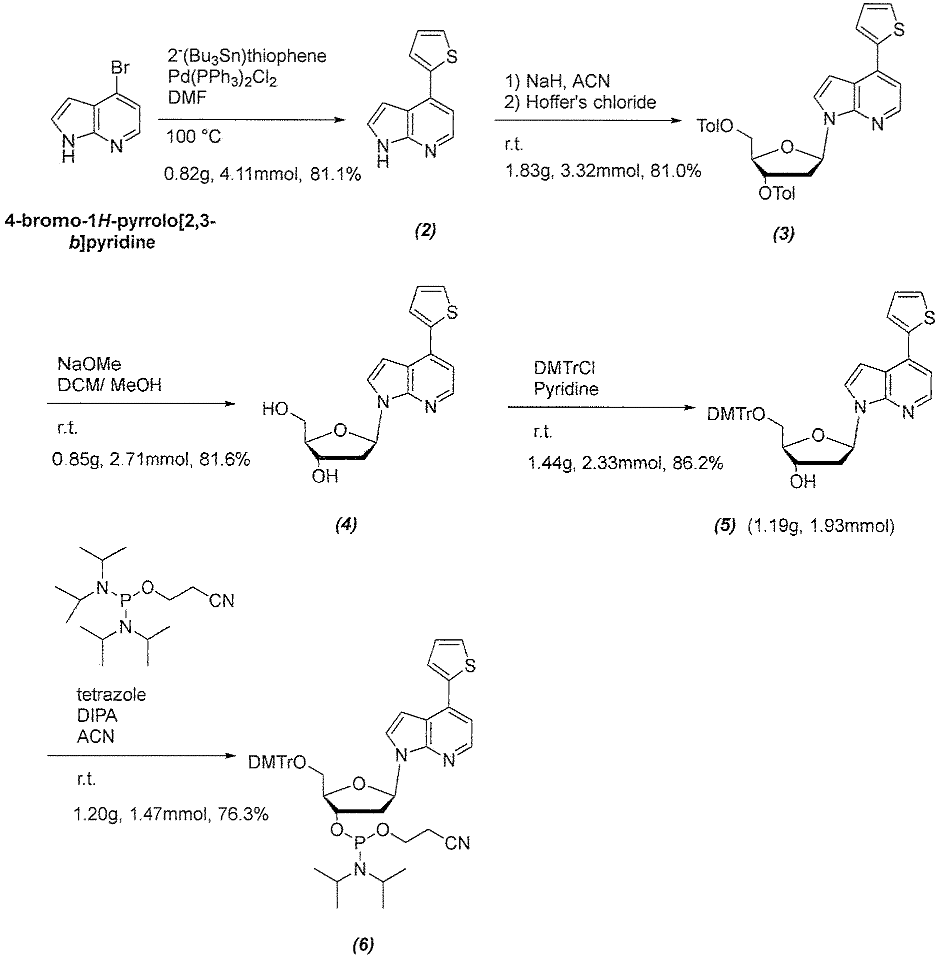

### Compound 2: 4-(thiophene-2-yl)-1H-pyrrolo[2,3-b]pyridine

A mixture of 4-bromo-1*H*-pyrrolo[2,3-*b*]pyridine (1.00 g, 5.07 mmol) and Pd(PPh₃)₂Cl₂ (0.180 g, 0.253 mmol) in DMF (25 mL) was degassed in vacuo, and the gas phase was replaced with argon for 10 minutes. After 2-(tributylstannyl)thiophene (2.46 mL, 7.61 mmol) was added, the resulting mixture was further degassed under argon for 10 minutes, and stirred at 100°C for 4 hours. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was subjected to a precipitating operation twice, using a solvent mixture of ethyl acetate and dichloromethane, to obtain a compound 2 in the form of a light yellow solid (0.82 g, 4.11 mmol, 81.1%). ¹H NMR (DMSO-d₆, 400 MHz) δ, ppm: 11.85 (s, 1H), 8.23 (d, 1H, J = 5.0 Hz), 7.81 (dd, 1H, J = 1.2, 3.6 Hz), 7.76 (dd, 1H, J = 1.1, 5.0 Hz), 7.58 (dd, 1H, J = 2.6, 3.4 Hz), 7.34 (d, 1H, J = 5.0 Hz), 7.26 (dd, 1H, J = 3.7, 5.1 Hz), 6.83 (dd, 1H, J = 1.9, 3.6 Hz).

### Compound 3: methyl ((2R,3S,5R)-3-((4-methylbenzoyl)oxy)-5-(4-(thiophene-2-yl)-1H-pyrrolo[2,3-b]pyridine-1-yl)tetrahydrofuran-2-yl)4-methylbenzoate

To a mixture of the compound 2 (0.82 g, 4.11 mmol) dissolved in ACN (52 mL), sodium hydride (60%) (0.20 g, 4.93 mmol) was added. The resulting mixture was stirred at ambient temperature for 20 minutes. Then, Hoffer chloride (1.92 g, 4.93 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane), whereby a compound 3 as a product of interest was obtained in the form of a yellow oil (1.83 g, 3.32 mmol, 81.0%). ¹H NMR (DMSO-d₆, 400 MHz) δ, ppm: 8.29 (d, 1H, J = 5.0 Hz), 7.99 - 7.97 (m, 2H), 7.90 - 7.88 (m, 2H), 7.86 (d, 1H, J = 3.9 Hz), 7.83 (dd, 1H, J = 1.1, 3.7 Hz), 7.80 (dd, 1H, J = 1.1, 5.1 Hz), 7.44 (d, 1H, J = 5.0 Hz), 7.40 - 7.38 (m, 2H), 7.34 - 7.32 (m, 2H), 7.28 (dd, 1H, J = 3.7, 5.1 Hz), 6.96 (d, 1H, J = 3.8 Hz), 6.91 - 6.88 (m, 1H), 5.76 - 5.74 (m, 1H), 4.64 - 4.53 (m, 3H), 3.18 - 3.11 (m, 1H), 2.75 - 2.69 (m, 1H), 2.42, 2.38 (2s, 6H).

### Compound 4: (2R,3S,5R)-2-(hydroxymethyl)-5-(4-(thiophene-2-yl)-1H-pyrrolo[2,3-b]pyridine-1-yl)tetrahydrofuran-3-ol

To a solution of the compound 3 (1.83 g, 3.32 mmol) in DCM/MeOH (1:1) (25.2 mL), sodium methoxide (5 M in MeOH) (1.726 mL, 8.63 mmol) was added, and the resulting solution was stirred at ambient temperature for 30 minutes. The solvent was removed in vacuo to obtain a crude product. The crude product was subjected to silica gel column chromatography (0 to 5% MeOH in DCM), and is thereby freeze-dried. Then, a compound 4 was isolated in the form of a foamy white solid (0.85 g, 2.71 mmol, 81.6%). ¹H NMR (DMSO-d₆, 400 MHz) δ, ppm: 8.27 (d, 1H, J = 5.0 Hz), 7.87 (d, 1H, J = 3.8 Hz), 7.83 (dd, 1H, J = 1.1, 3.6 Hz), 7.79 (dd, 1H, J = 1.1, 5.1 Hz), 7.41 (d, 1H, J = 5.0 Hz), 7.28 (dd, 1H, J = 3.7, 5.1 Hz), 6.93 (d, 1H, J = 3.8 Hz), 6.75 (dd, 1H, J = 6.0, 8.2 Hz), 5.29 (d, 1H, J = 4.1 Hz), 5.04 (t, 1H, J = 5.6 Hz), 4.40 - 4.37 (m, 1H), 3.86 - 3.84 (m, 1H), 3.62 - 3.50 (m, 2H), 2.60 - 2.53 (m, 1H), 2.26 - 2.21 (m, 1H).

### Compound 5: (2R,3S,5R)-2-((bis(4-methoxyphenyl)(phenyl)methoxy)methyl)-5-(4-(thiophene-2-yl)-1H-pyrrolo[2,3-b]pyridine-1-yl)tetrahydrofuran-3-ol

The compound 4 (0.85 g, 2.71 mmol) was evaporated together with pyridine (three times) before pyridine (2.7 mL) and DMTrCl (0.92 g, 2.71 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a DCM/MeOH solvent (0 to 1% MeOH in DCM) (1% TEA), whereby a compound 5 as a desired product was obtained in the form of a foamy light green solid (1.44 g, 2.33 mmol, 86.2%). ¹H NMR (DMSO-d₆, 400 MHz) δ, ppm: 8.27 (d, 1H, J = 5.0 Hz), 7.83 (dd, 1H, J = 1.1, 3.7 Hz), 7.79 (dd, 1H, J = 1.1, 5.1 Hz), 7.70 (d, 1H, J = 3.8 Hz), 7.43 (d, 1H, J = 5.0 Hz), 7.38 - 7.36 (m, 2H), 7.29 - 7.19 (m, 8H), 6.90 (d, 1H, J = 3.8 Hz), 6.85 - 6.81 (m, 4H), 6.77 (t, 1H, J = 6.7 Hz), 5.36 (d, 1H, J = 4.5 Hz), 4.42 - 4.38 (m, 1H), 3.98 - 3.95 (m, 1H), 3.71, 3.70 (2s, 6H), 3.17 - 3.16 (m, 2H), 2.65 - 2.59 (m, 1H), 2.34 - 2.28 (m, 1H).

### Compound 6: (2R,3S,5R)-2-((bis(4-methoxyphenyl)(phenyl)methoxy)methyl)-5-(4-(thiophene-2-yl)-1H-pyrrolo[2,3-b]pyridine-1-yl)tetrahydrofuran-3-yl(2-cyanoethyl)diisopropylphosphoramidite

The compound 5 (1.19 g, 1.93 mmol) and 1*H*-tetrazole in ACN (0.4 M) (2.22 mL, 0.88 mmol) were evaporated (three times) together with ACN before ACN (49 mL), DIPA (0.124 mL, 0.88 mmol), and 2-cyanoethyl *N,N,N',N'*-tetraisopropylphosphorodiamidite (0.98 mL, 3.09 mmol) were added. The reaction mixture was stirred at ambient temperature for 3.5 hours. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 40% EtOAc in hexane) (1% TEA), whereby a compound 6 was obtained in the form of a foamy white solid (1.20 g, 1.47 mmol, 76.3%). ¹H NMR (DMSO-d₆, 400 MHz) δ, ppm: 8.25 (dd, 1H, J = 0.9, 5.1 Hz), 7.83 (dd, 1H, J = 1.1, 3.7 Hz), 7.79 (dd, 1H, J = 1.1, 5.1 Hz), 7.73 (d, 1H, J = 3.8 Hz), 7.43 (dd, 1H, J = 0.6, 5.0 Hz), 7.39 - 7.35 (m, 2H), 7.28 - 7.19 (m, 8H), 6.93 - 6.92 (m, 1H), 6.85 - 6.79 (m, 4H), 6.75 (t, 1H, J = 6.8 Hz), 4.72 - 4.66 (m, 1H), 4.13 - 4.06 (m, 1H), 3.78 - 3.52 (m, 10H), 3.30 - 3.14 (m, 2H), 2.86 - 2.81 (m, 1H), 2.78 (t, 1H, J = 5.9 Hz), 2.68 (t, 1H, J = 5.9 Hz), 2.55 - 2.41 (m, 1H), 1.16 - 1.12 (m, 9H), 1.06 - 1.04 (m, 3H). ³¹P NMR (DMSO-d₆, 162 MHz) δ, ppm: 147.57, 146.96 (diastereoisomer).

### (4) Chemical synthesis of dYo amidite

The compounds 2 to 6 corresponding to (2) to (6) in the reaction path scheme shown below were synthesized using the following method.

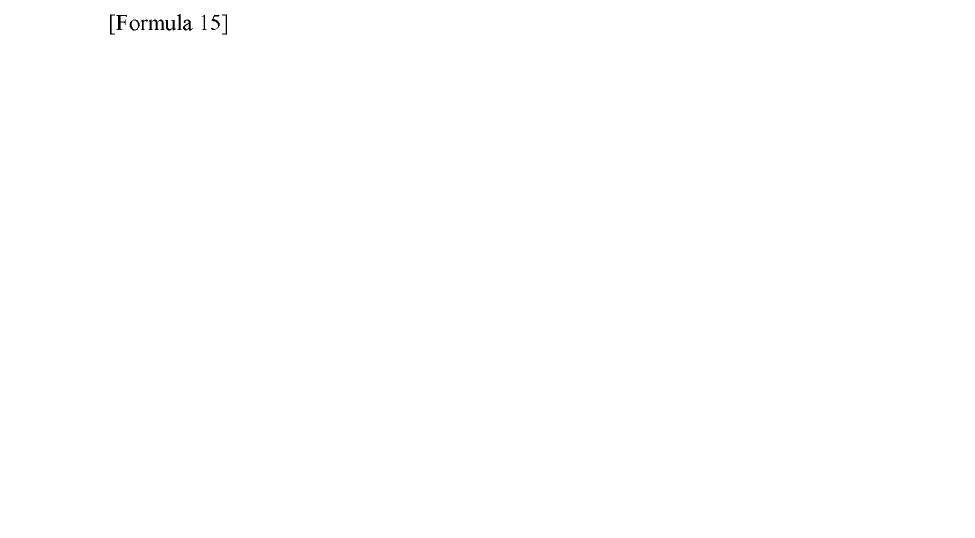

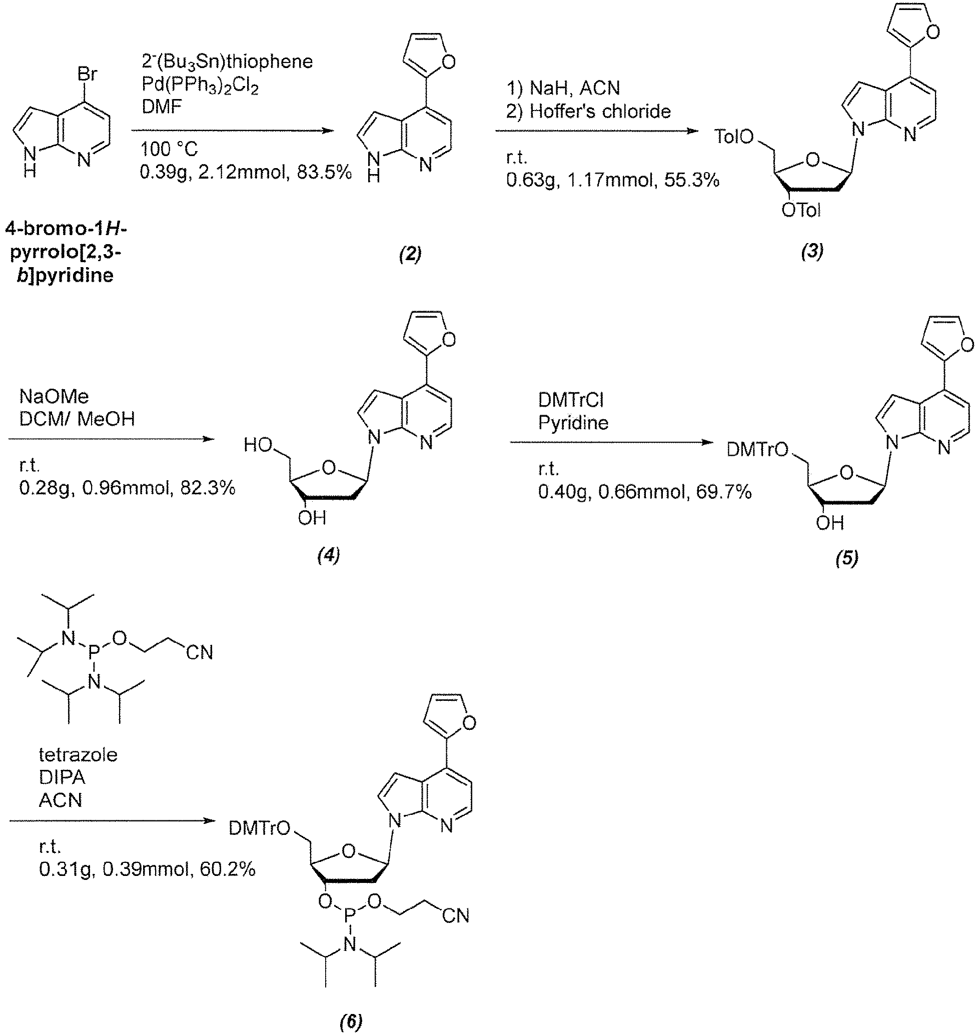

### Compound 2: 4-(furan-2-yl)-1H-pyrrolo[2,3-b]pyridine

A mixture of 4-bromo-1*H*-pyrrolo[2,3-*b*]pyridine (0.50 g, 2.54 mmol) and Pd(PPh₃)₂Cl₂ (0.089 g, 0.1273 mmol) in DMF (12.5 mL) was degassed in vacuo, and the gas phase was replaced with argon for 10 minutes. After 2-(tributylstannyl)furan (1.20 mL, 3.81 mmol) was added, the resulting mixture was further degassed under argon for 10 minutes, and stirred at 100°C for 3 hours. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was washed with dichloromethane twice to obtain a compound 2 in the form of a light yellow solid (0.39 g, 2.12 mmol, 83.5%). ¹H NMR (DMSO-d₆, 400 MHz) δ, ppm: 11.78 (bs, 1H), 8.23 (d, 1H, J = 5.1 Hz), 7.92 (dd, 1H, J = 0.6, 1.8 Hz), 7.55 (dd, 1H, J = 2.6, 3.3 Hz), 7.38 (d, 1H, J = 5.0 Hz), 7.29 (dd, 1H, J = 0.6, 3.5 Hz), 6.87 (dd, 1H, J = 1.9, 3.5 Hz), 6.72 (dd, 1H, J = 1.8, 3.4 Hz).

### Compound 3: (2R,3S,5R)-5-(4-(furan-2-yl)-1H-pyrrolo[2,3-b]pyridine-1-yl)-2-(((4-methylbenzoyl)oxy)methyl)tetrahydrofuran-3-yl 4-methylbenzoate.

To a mixture of the compound 2 (0.39 g, 2.12 mmol) dissolved in ACN (27 mL), sodium hydride (60%) (0.10 g, 2.54 mmol) was added. The resulting mixture was stirred at ambient temperature for 20 minutes. Then, Hoffer chloride (0.99 g, 2.54 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane), whereby a compound 3 as a product of interest was obtained in the form of a colorless oil (0.63 g, 1.17 mmol, 55.3%). ¹H NMR (DMSO-d₆, 400 MHz) δ, ppm: 8.29 (d, 1H, J = 5.1 Hz), 7.99 - 7.96 (m, 2H), 7.95 (dd, 1H, J = 0.5, 1.7 Hz), 7.90 - 7.88 (m, 2H), 7.83 (d, 1H, J = 3.8 Hz), 7.49 (d, 1H, J = 5.0 Hz), 7.40 - 7.32 (m, 5H), 7.02 (d, 1H, J = 3.8 Hz), 6.89 (dd, 1H, J = 5.9, 8.7 Hz), 6.73 (dd, 1H, J = 1.8, 3.4 Hz), 5.76 - 5.74 (m, 1H), 4.65 - 4.60 (m, 1H), 4.56 - 4.51 (m, 2H), 3.18 - 3.10 (m, 1H), 2.74 - 2.68 (m, 1H), 2.41, 2.38 (2s, 6H).

### Compound 4:(2R,3S,5R)-5-(4-(furan-2-yl)-1H-pyrrolo[2,3-b]pyridine-1-yl)-2-(hydroxymethyl)tetrahydrofuran-3-ol

To a solution of the compound 3 (0.63 g, 1.17 mmol) in DCM/MeOH (1:1) (9.0 mL), sodium methoxide (5 M in MeOH) (0.608 mL, 3.04 mmol) was added, and the resulting solution was stirred at ambient temperature for 30 minutes. The solvent was removed in vacuo to obtain a crude product. The crude product was subjected to silica gel column chromatography (0 to 5% MeOH in DCM), whereby a compound 4 was isolated in the form of a white solid (0.29 g, 0.96 mmol, 82.3%). ¹H NMR (DMSO-d₆, 400 MHz) δ, ppm: 8.27 (d, 1H, J = 5.1 Hz), 7.95 (dd, 1H, J = 0.6, 1.8 Hz), 7.84 (d, 1H, J = 3.8 Hz), 7.45 (d, 1H, J = 5.1 Hz), 7.34 (dd, 1H, J = 0.6, 3.5 Hz), 6.98 (d, 1H, J = 3.7 Hz), 6.76 - 6.72 (m, 2H), 5.28 (d, 1H, J = 4.1 Hz), 5.04 (t, 1H, J = 5.6 Hz), 4.40 - 4.36 (m, 1H), 3.86 - 3.83 (m, 1H), 3.62 - 3.49 (m, 2H), 2.60 - 2.49 (m, 1H), 2.25 - 2.19 (m, 1H).

### Compound 5: (2R,3S,5R)-2-((bis(4-methoxyphenyl)(phenyl)methoxy)methyl)-5-(4-(furan-2-yl)-1H-pyrrolo[2,3-b]pyridine-1-yl)tetrahydrofuran-3-ol

The compound 4 (0.29 g, 0.96 mmol) was evaporated together with pyridine (three times) before pyridine (1.0 mL) and DMTrCl (0.33 g, 0.96 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a DCM/MeOH solvent (0 to 1% MeOH in DCM) (1% TEA), whereby a compound 5 as a desired product was obtained in the form of a white solid (0.40 g, 0.67 mmol, 69.7%). ¹H NMR (DMSO-d₆, 400 MHz) δ, ppm: 8.27 (d, 1H, J = 5.1 Hz), 7.94 (dd, 1H, J = 0.6, 1.7 Hz), 7.67 (d, 1H, J = 3.8 Hz), 7.47 (d, 1H, J = 5.0 Hz), 7.38 - 7.34 (m, 3H), 7.28 - 7.19 (m, 7H), 6.96 (d, 1H, J = 3.8 Hz), 6.84 - 6.80 (m, 5H), 6.74 (dd, 1H, J = 1.8, 3.5 Hz), 5.35 (d, 1H, J = 4.5 Hz), 4.42 - 4.37 (m, 1H), 3.97 - 3.94 (m, 1H), 3.71, 3.70 (2s, 6H), 3.16 - 3.15 (m, 2H), 2.66 - 2.59 (m, 1H), 2.33 - 2.27 (m, 1H).

### Compound 6: (2R,3S,5R)-2-((bis(4-methoxyphenyl)(phenyl)methoxy)methyl)-5-(4-(furan-2-yl)-1H-pyrrolo[2,3-b]pyridine-1-yl)tetrahydrofuran-3-yl(2-cyanoethyl)diisopropylphosphoramidite

The compound 5 (0.40 g, 0.66 mmol) and 1*H*-tetrazole in ACN (0.4 M) (0.77 mL, 0.31 mmol) were evaporated (three times) together with ACN before ACN (17 mL), DIPA (0.043 mL, 0.31 mmol), and 2-cyanoethyl *N,N,N',N'*-tetraisopropylphosphorodiamidite (0.339 mL, 1.07 mmol) were added. The reaction mixture was stirred at ambient temperature for 3 hours. The reaction was quenched with addition of an aqueous NaHCO₃ solution, and subsequently, liquid extraction was performed using an aqueous NaHCO₃ solution and ethyl acetate. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane) (1% TEA), whereby a compound 6 was obtained in the form of a foamy white solid (0.32 g, 0.39 mmol, 60.2%). 1H NMR (DMSO-d6, 400 MHz) δ, ppm: 88.26 (dd, 1H, J = 1.0, 5.1 Hz), 7.94 (dd, 1H, J = 0.6, 1.7 Hz), 7.71 (d, 1H, J = 3.8 Hz), 7.47 (d, 1H, J = 5.1 Hz), 7.39 - 7.35 (m, 3H), 7.27 - 7.21 (m, 7H), 6.98 (dd, 1H, J = 1.4, 3.8 Hz), 6.84 - 6.73 (m, 6H), 4.71 - 4.65 (m, 1H), 4.13 - 4.04 (m, 1H), 3.71 - 3.51 (m, 10H), 3.28 - 3.14 (m, 2H), 2.87 - 2.81 (m, 1H), 2.78 (t, 1H, J = 5.9 Hz), 2.68 (t, 1H, J = 5.9 Hz), 2.51 - 2.40 (m, 1H with DMSO peak), 1.16 - 1.12 (m, 10H), 1.05 - 1.04 (m, 2H). 31P NMR (DMSO-d6, 162 MHz) δ, ppm: 147.57, 146.94 (diastereoisomer).

### (5) Chemical synthesis of dEs amidite

The compounds 2 to 6 corresponding to (2) to (6) in the reaction path scheme shown below were synthesized using the following method.

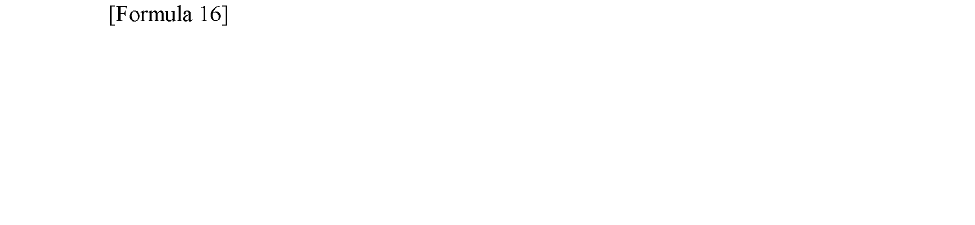

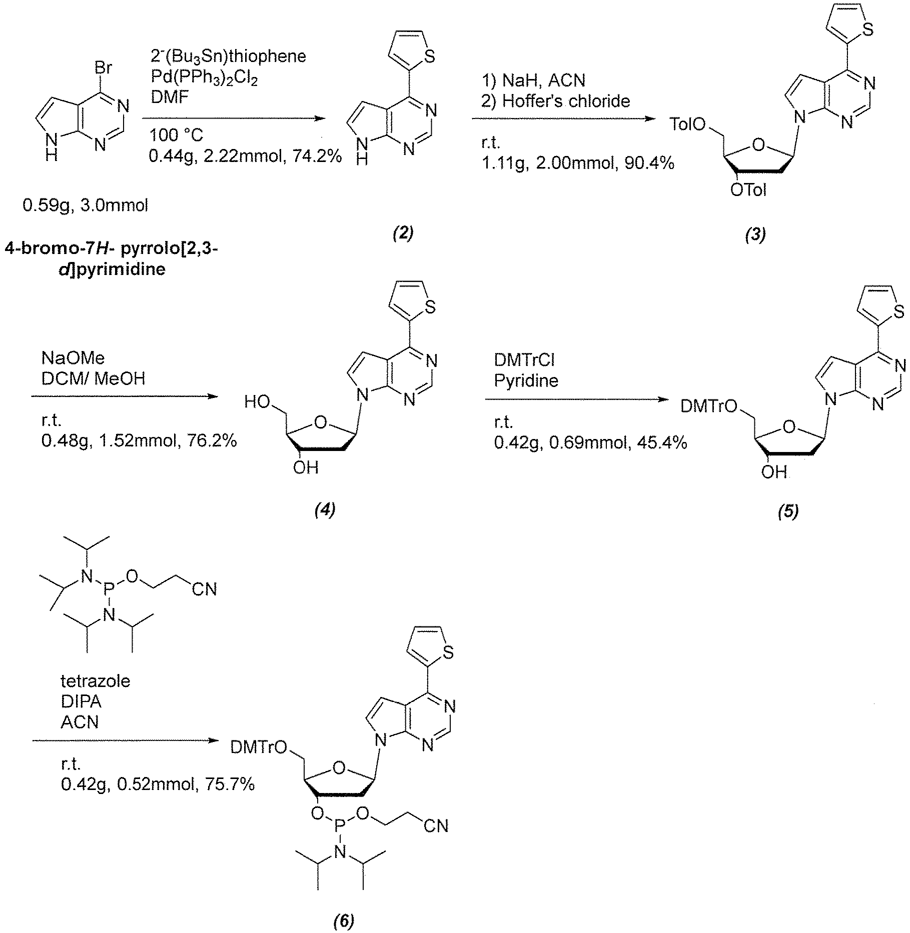

### Compound 2: 4-(thiophene-2-yl)-7H-pyrrolo[2,3-d]pyrimidine

A mixture of 4-bromo-7*H*-pyrrolo[2,3-*d*]pyrimidine (0.594 g, 3.0 mmol) and Pd(PPh₃)₂Cl₂ (0.105 g, 0.15 mmol) in DMF (14.8 mL) was degassed in vacuo, and the gas phase was replaced with argon for 10 minutes. After 2-(tributylstannyl)thiophene (1.455 mL, 4.5 mmol) was added, the resulting mixture was further degassed under argon for 10 minutes, and stirred at 100°C for 3 hours. The reaction mixture was subjected to liquid extraction using brine and ethyl acetate. The organic layer was dried over anhydrous MgSO4, and filtrated. The resulting filtrate was concentrated in vacuo to obtain a crude product. The crude product was washed with dichloromethane twice to obtain a compound 2 in the form of a light yellow solid (0.44 g, 2.22 mmol, 74.2%). ¹H NMR (DMSO-d₆, 400 MHz) δ, ppm: 12.26 (bs, 1H), 8.70 (s, 1H), 8.14 (dd, 1H, J = 1.0, 3.8 Hz), 7.83 (dd, 1H, J = 1.0, 5.0 Hz), 7.66 (dd, 1H, J = 2.4, 3.5 Hz), 7.29 (dd, 1H, J = 3.8, 5.1 Hz), 7.04 (dd, 1H, J = 1.6, 3.6 Hz).

### Compound 3: methyl ((2R,3S,5R)-3-((4-methylbenzoyl)oxy)-5-(4-(thiophene-2-yl)-7H-pyrrolo[2,3-d]pyrimidine-7-yl)tetrahydrofuran-2-yl)4-methylbenzoate

To a mixture of the compound 2 (0.44 g, 2.22 mmol) dissolved in ACN (28.4 mL), sodium hydride (60%) (0.106 g, 2.664 mmol) was added. The resulting mixture was stirred at ambient temperature for 20 minutes. Then, Hoffer chloride (1.035 g, 2.664 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane), whereby a compound 3 as a product of interest was obtained in the form of a light yellow solid (1.11 g, 2.00 mmol, 90.4%). ¹H NMR (DMSO-d₆, 400 MHz) δ, ppm: 8.75 (s, 1H), 8.17 (dd, 1H, J = 1.0, 3.8 Hz), 7.97 (d, 2H, J = 8.2 Hz), 7.93 (d, 1H, J = 3.9 Hz), 7.88 - 7.86 (m, 3H), 7.38 (d, 2H, J = 8.0 Hz), 7.32 - 7.29 (m, 3H), 7.20 (d, 1H, J = 3.9 Hz), 6.83 (dd, 1H, J = 6.0, 8.4 Hz), 5.78 - 5.76 (m, 1H), 4.66 - 4.61 (m, 1H), 4.57 - 4.53 (m, 2H), 3.21 - 3.14 (m, 1H), 2.79 - 2.73 (m, 1H), 2.41, 2.37 (2s, 6H).

### Compound 4: (2R,3S,5R)-2-(hydroxymethyl)-5-(4-(thiophene-2-yl)-7H-pyrrolo[2,3-d]pyrimidine-7-yl)tetrahydrofuran-3-ol

To a solution of the compound 3 (1.11 g, 2.00 mmol) in DCM/MeOH (1:1) (15.2 mL), sodium methoxide (5 M in MeOH) (1.04 mL, 5.20 mmol) was added, and the resulting solution was stirred at ambient temperature for 30 minutes. The solvent was removed in vacuo to obtain a crude product. The crude product was subjected to silica gel column chromatography (0 to 4% MeOH in DCM), whereby a compound 4 was isolated in the form of a white solid (0.48 g, 1.5 mmol, 76.2%). ¹H NMR (DMSO-d₆, 400 MHz) δ, ppm: 8.75 (s, 1H), 8.17 (dd, 1H, J = 1.1, 3.8 Hz), 7.95 (d, 1H, J = 3.8 Hz), 7.86 (dd, 1H, J = 1.0, 5.0 Hz), 7.31 (dd, 1H, J = 3.8, 5.0 Hz), 7.16 (d, 1H, J = 3.8 Hz), 6.70 (dd, 1H, J = 6.1, 7.9 Hz), 5.32 (d, 1H, J = 4.2 Hz), 4.99 (t, 1H, J = 5.5 Hz), 4.41 - 4.38 (m, 1H), 3.88 - 3.85 (m, 1H), 3.63 - 3.51 (m, 2H), 2.60 - 2.53 (m, 1H), 2.29 - 2.24 (m, 1H).

### Compound 5: (2R,3S,5R)-2-((bis(4-methoxyphenyl)(phenyl)methoxy)methyl)-5-(4-(thiophene-2-yl)-7H-pyrrolo[2,3-d]pyrimidine-7-yl)tetrahydrofuran-3-ol

The compound 4 (0.48 g, 1.52 mmol) was evaporated together with pyridine (three times) before pyridine (1.5 mL) and DMTrCl (0.514 g, 1.52 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a DCM/MeOH solvent (0 to 1% MeOH in DCM) (1% TEA), whereby a compound 5 as a desired product was obtained in the form of a foamy white solid (0.42 g, 0.69 mmol, 45.4%). ¹H NMR (DMSO-d₆, 400 MHz) δ, ppm: 8.74 (s, 1H), 8.17 (dd, 1H, J = 1.0, 3.8 Hz), 7.86 (dd, 1H, J = 1.0, 5.0 Hz), 7.77 (d, 1H, J = 3.8 Hz), 7.37 - 7.35 (m, 2H), 7.30 (dd, 1H, J = 3.8, 5.0 Hz), 7.27 - 7.19 (m, 7H), 7.15 (d, 1H, J = 3.8 Hz), 6.84 - 6.79 (m, 4H), 6.70 (t, 1H, J = 6.6 Hz), 5.38 (d, 1H, J = 4.6 Hz), 4.46 - 4.41 (m, 1H), 3.99 - 3.96 (m, 1H), 3.70, 3.69 (2s, 6H), 3.18 - 3.17 (m, 2H), 2.72 - 2.65 (m, 1H), 2.37 - 2.31 (m, 1H).

### Compound 6: (2R,3S,5R)-2-((bis(4-methoxyphenyl)(phenyl)methoxy)methyl)-5-(4-(thiophene-2-yl)-7H-pyrrolo[2,3-d]pyrimidine-7-yl)tetrahydrofuran-3-yl(2-cyanoethyl)diisopropylphosphoramidite

The compound 5 (0.42 g, 0.69 mmol) and 1*H*-tetrazole in ACN (0.4 M) (0.794 mL, 0.317 mmol) were evaporated (three times) together before ACN (18 mL), DIPA (0.044 mL, 0.317 mmol), and 2-cyanoethyl *N,N,N',N'*-tetraisopropylphosphorodiamidite (0.350 mL, 1.10 mmol) were added. The reaction mixture was stirred at ambient temperature for 3.5 hours. The reaction was quenched with addition of aq. NaHCO₃, and subsequently, liquid extraction was performed using aq. NaHCO₃ and ethyl acetate. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane) (1% TEA), whereby a compound 6 was obtained in the form of a foamy white solid (0.43 g, 1.52 mmol, 75.7%). ¹H NMR (DMSO-d₆, 400 MHz) δ, ppm: 8.72 (d, 1H, J = 2.2 Hz), 8.18 (dd, 1H, J = 1.0, 3.8 Hz), 7.86 (dd, 1H, J = 1.0, 5.0 Hz), 7.81 (t, 1H, J = 3.4 Hz), 7.37 - 7.16 (m, 11H), 6.84 - 6.78 (m, 4H), 6.70 - 6.66 (m, 1H), 4.75 - 4.69 (m, 1H), 4.15 - 4.06 (m, 1H), 3.78 - 3.52 (m, 10H), 3.29 - 3.15 (m, 2H), 2.92 - 2.85 (m, 1H), 2.78 (t, 1H, J = 5.9 Hz), 2.67 (t, 1H, J = 5.9 Hz), 2.59 - 2.44 (m, 1H, with DMSO peak), 1.16 - 1.12 (m, 10H), 1.05 - 1.03 (m, 2H). ³¹P NMR (DMSO-d₆, 162 MHz) δ, ppm: 147.68, 147.05 (diastereoisomer).

### (6) Chemical synthesis of dYsm amidite

The compounds 2 to 6 corresponding to (2) to (6) in the reaction path scheme shown below were synthesized using the following method.

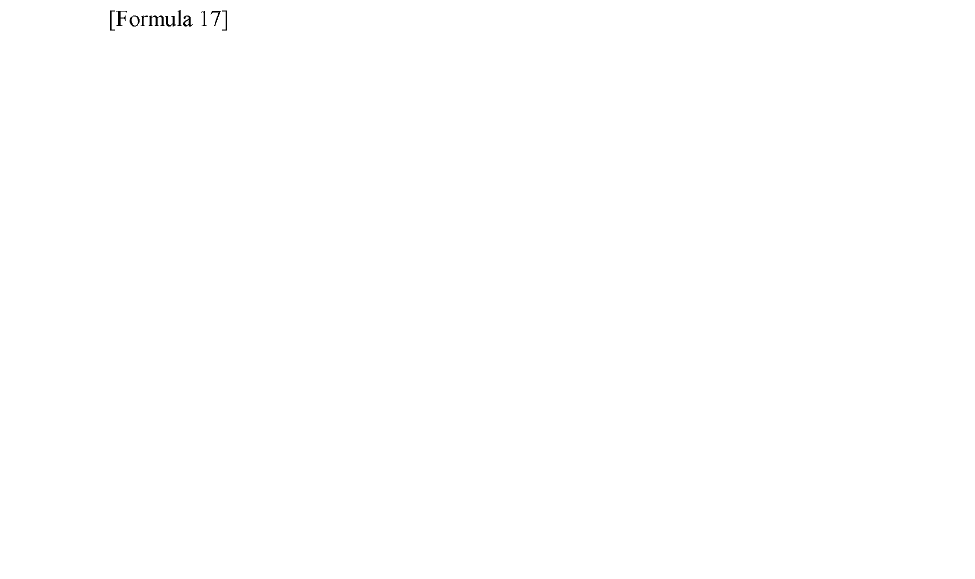

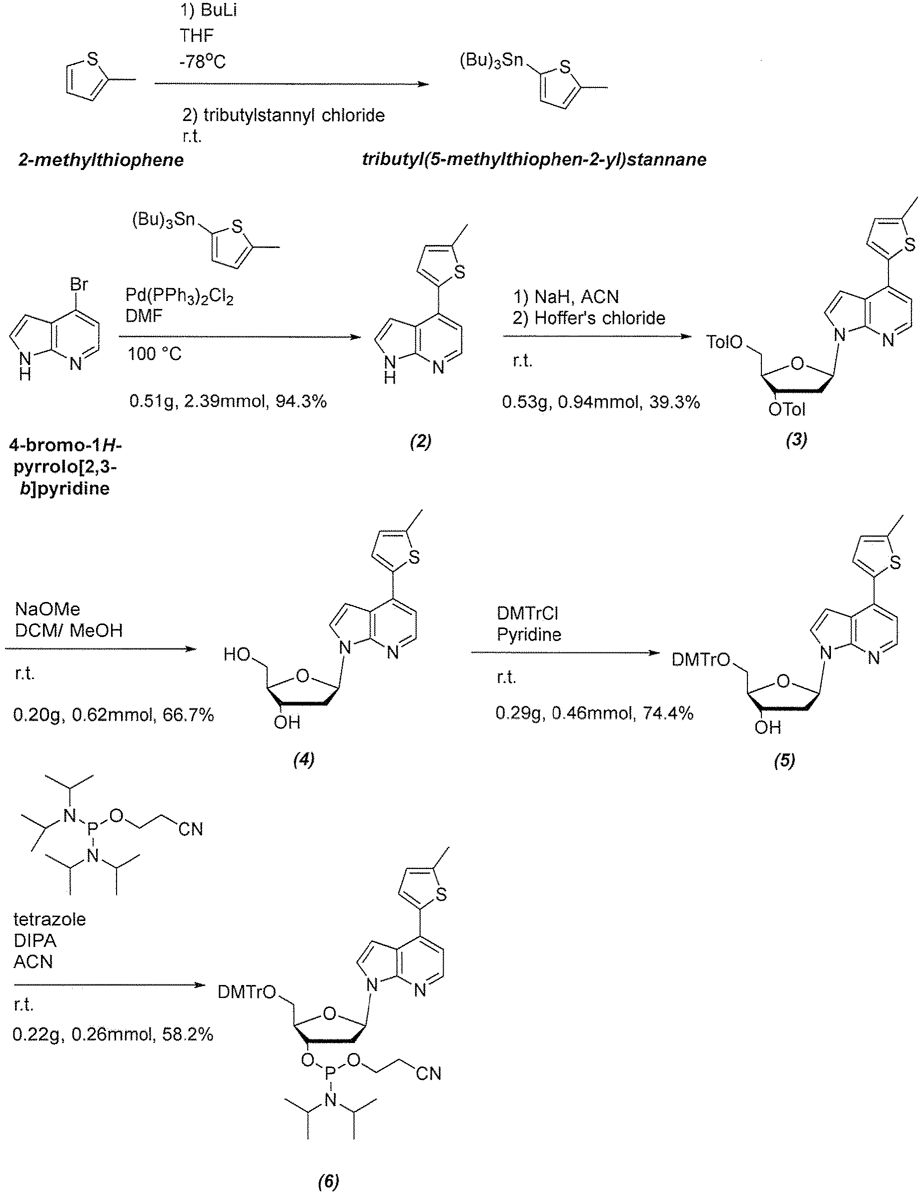

### Tributyl(5-methylthiophene-2-yl)

To a stirred solution of 2-methylthiophene (0.405 mL, 4.21 mmol) in THF (21 mL) at -78°C, n-BuLi (2M in hexane) (2.11 mL, 4.21 mmol) was added, and the resulting mixture was stirred for 30 minutes. Then, tributylstannyl chloride (1.14 mL, 4.21 mmol) was added. The resulting solution was heated to ambient temperature, and further stirred for 30 minutes. The reaction mixture was quenched with addition of ice-cold H₂O, and extracted with ethyl acetate. The organic layer combined was washed with brine, dried over anhydrous MgSO₄, and filtrated. The resulting filtrate was concentrated in vacuo to obtain a Sn reagent.

### Compound 2: 4-(5-methylthiophene-2-yl)-1H-pyrrolo[2,3-b]pyridine.

A mixture of 4-bromo-1*H*-pyrrolo-[2,3-*b*]pyridine (0.50 g, 2.54 mmol) and Pd(PPh₃)₂Cl₂ (0.089 g, 0.127 mmol) in DMF (12.5 mL) was degassed in vacuo, and the gas phase was replaced with argon for 10 minutes. After tributyl(5-methylthiophene-2-yl)stannane (4.21 mmol) was added, the resulting mixture was further degassed under argon for 10 minutes, and stirred at 100°C for 3 hours. The mixture was cooled to room temperature, and concentrated in vacuo. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was washed with dichloromethane twice to obtain a compound 2 in the form of a yellow solid (0.33 g, 1.54 mmol, 60.7%). The product washed was concentrated in vacuo, further purified by silica gel column chromatography using a hexane/EtOAc solvent (0 to 50% EtOAc in hexane). Then, a compound 2 was collected in the form of a yellow solid (0.18 g, 0.85 mmol, 33.4%). ¹H NMR (DMSO-d₆, 400 MHz) δ, ppm: 11.80 (bs, 1H), 8.19 (d, 1H, J = 5.0 Hz), 7.61 (d, 1H, J = 3.6 Hz), 7.55 (dd, 1H, J = 2.7, 3.4 Hz), 7.25 (d, 1H, J = 5.0 Hz), 6.95 (dd, 1H, J = 1.1, 3.6 Hz), 6.80 (dd, 1H, J = 1.9, 3.5 Hz), 2.53 (dd, 3H, J = 0.8 Hz).

### Compound 3: methyl ((2R,3S,5R)-3-((4-methylbenzoyl)oxy)-5-(4-(5-methylthiophene-2-yl)-1H-pyrrolo[2,3-b]pyridine-1-yl)tetrahydrofuran-2-yl)4-methylbenzoate

To a stirred mixture of the compound 2 (0.33 g, 1.54 mmol) in ACN (20 mL), sodium hydride (60%) (0.074 g, 1.848 mmol) was added. The resulting mixture was stirred at ambient temperature for 20 minutes. Then, Hoffer chloride (0.718 g, 1.848 mmol) was added, and the resulting mixture was further stirred at ambient temperature for 3.5 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was first purified with silica gel column chromatography using a hexane/EtOAc solvent (0 to 20% EtOAc in hexane) to obtain a compound 3 (0.37 g, 0.66 mmol, 42.9%). The compound 2 (0.18 g, 0.85 mmol) collected was subjected to this reaction repeatedly to further obtain a compound 3 (0.24 g, 0.42 mmol, 49.8%). The two lots were combined, and subjected to repeated purification by silica gel column chromatography using a hexane/EtOAc solvent (0 to 20% EtOAc in hexane), whereby a compound 3 as a product of interest was obtained in the form of a light yellow oil (0.53 g, 0.94 mmol, 39.3%). ¹H NMR (DMSO-d₆, 400 MHz) δ, ppm: 8.25 (d, 1H, J = 5.1 Hz), 7.98 - 7.96 (m, 2H), 7.90 - 7.88 (m, 3H), 7.83 (d, 1H, J = 3.9 Hz), 7.64 (d, 1H, J = 3.6 Hz), 7.40 - 7.32 (m, 6H), 6.98 - 6.96 (m, 1H), 6.93 (d, 1H, J = 3.8 Hz), 6.88 (dd, 1H, J = 5.9, 8.6 Hz), 5.76 - 7.74 (m, 1H), 4.65 - 4.60 (m, 1H), 4.56 - 4.51 (m, 2H), 3.17 - 3.09 (m, 1H), 2.74 - 2.68 (m, 1H), 2.54 (m, 3H), 2.41 - 2.38 (m, 13H).

### Compound 4: (2R,3S,5R)-2-(hydroxymethyl)-5-(4-(5-methylthiophene-2-yl)-1H-pyrrolo[2,3-b]pyridine-1-yl)tetrahydrofuran-3-ol

To a solution of the compound 3 (0.53 g, 0.94 mmol) in DCM/MeOH (1:1) (7.2 mL), sodium methoxide (5 M in MeOH) (0.491 mL, 2.45 mmol) was added, and the resulting solution was stirred at ambient temperature for 30 minutes. The solvent was removed in vacuo to obtain a crude product. The crude product was subjected to silica gel column chromatography (0 to 5% MeOH in DCM), whereby a compound 4 was isolated in the form of a white solid (0.20 g, 0.62 mmol, 66.7%). ¹H NMR (DMSO-d₆, 400 MHz) δ, ppm: 8.23 (d, 1H, J = 5.1 Hz), 7.83 (d, 1H, J = 3.8 Hz), 7.63 (d, 1H, J = 3.6 Hz), 7.33 (d, 1H, J = 5.1 Hz), 6.96 (dd, 1H, J = 1.1, 3.6 Hz), 6.89 (d, 1H, J = 3.8 Hz), 6.73 (dd, 1H, J = 6.0, 8.2 Hz), 5.29 (d, 1H, J = 4.1 Hz), 5.05 (t, 1H, 5.6 Hz), 4.40 - 4.36 (m, 1H), 3.86 - 3.83 (m, 1H), 3.61 - 3.49 (m, 2H), 2.59 - 2.52 (m, 4H), 2.25 - 2.19 (m, 1H).

### Compound 5: (2R,3S,5R)-2-((bis(4-methoxyphenyl)(phenyl)methoxy)methyl)-5-(4-(5-methylthiophene-2-yl)-1H-pyrrolo[2,3-b]pyridine-1-yl)tetrahydrofuran-3-ol

The compound 4 (0.20 g, 0.62 mmol) was evaporated together with pyridine (three times) before pyridine (1.0 mL) and DMTrCl (0.21 g, 0.62 mmol) were added to the compound. The reaction mixture was stirred at ambient temperature for 1 hour. The reaction was quenched with addition of water, and the solvent was removed in vacuo. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a DCM/MeOH solvent (0 to 0.5% MeOH) (1% TEA), whereby a compound 5 as a product of interest was obtained in the form of a foamy white solid (0.29 g, 0.46 mmol, 74.4%). ¹H NMR (DMSO-d₆, 400 MHz) δ, ppm: 8.23 (d, 1H, J = 5.1 Hz), 7.67 (d, 1H, J = 3.8 Hz), 7.63 (d, 1H, J = 3.6 Hz), 7.38 - 7.34 (m, 3H), 7.28 - 7.18 (m, 7H), 6.96 (dd, 1H, J = 1.1, 3.6 Hz), 6.87 (d, 1H, J = 3.8 Hz), 6.85 - 6.81 (m, 4H), 6.76 (t, 1H, J = 6.7 Hz), 5.35 (d, 1H, J = 4.5 Hz), 4.41 - 4.37 (m, 1H), 3.97 - 3.94 (m, 1H), 3.71 (2s, 6H), 3.17 - 3.15 (m, 2H), 2.64 - 2.58 (m, 1H), 2.54 (m, 3H), 2.32 - 2.27 (m, 1H).

### Compound 6: (2R,3S,5R)-2-((bis(4-methoxyphenyl)(phenyl)methoxy)methyl)-5-(4-(5-methylthiophene-2-yl)-1H-pyrrolo[2,3-b]pyridine-1-yl)tetrahydrofuran-3-yl(2-cyanoethyl)diisopropylphosphoramidite

The compound 5 (0.29 g, 0.46 mmol) and 1*H*-tetrazole in ACN (0.4 M) (0.529 ml, 0.211 mmol) were evaporated (three times) together with ACN before ACN (12 mL), DIPA (0.030 mL, 0.211 mmol), and 2-cyanoethyl *N,N,N',N'*-tetraisopropylphosphorodiamidite (0.234 mL, 0.736 mmol) were added. The reaction mixture was stirred at ambient temperature for 3 hours. The reaction was quenched with addition of an aqueous NaHCO₃ solution, and subsequently, liquid extraction was performed using an aqueous NaHCO₃ solution and ethyl acetate. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane) (1% TEA), whereby a compound 6 was obtained in the form of a foamy white solid (0.22 g, 0.26 mmol, 58.2%). ¹H NMR (DMSO-d₆, 400 MHz) δ, ppm: 8.22 (d, 1H, J = 5.1 Hz), 7.70 (d, 1H, J = 3.8 Hz), 7.64 (d, 1H, J = 3.6 Hz), 7.39 - 7.34 (m, 3H), 7.28 - 7.19 (m, 7H), 6.96 (dd, 1H, J = 1.1, 3.6 Hz), 6.89 (d, 1H, J = 1.7, 3.8 Hz), 6.85 - 6.80 (m, 4H), 6.74 (t, 1H, J = 6.8 Hz), 4.71 - 7.65 (m, 1H), 4.13 - 4.04 (m, 1H), 3.78 - 3.54 (m, 10H), 3.28 - 3.15 (m, 2H), 2.85 - 2.76 (m, 2H), 2.68 (t, 1H, J = 5.9 Hz), 2.54 (m, 3H), 2.45 - 2.39 (m, 1H), 1.16 - 1.12 (m, 9H), 1.05 - 1.04 (m, 3H). ³¹P NMR (DMSO-d₆, 162 MHz) δ, ppm: 147.57, 146.95 (diastereoisomer).

### (7) Chemical synthesis of dBss amidite

The compounds 2 to 6 corresponding to (2) to (6) in the reaction path scheme shown below were synthesized using the following method.

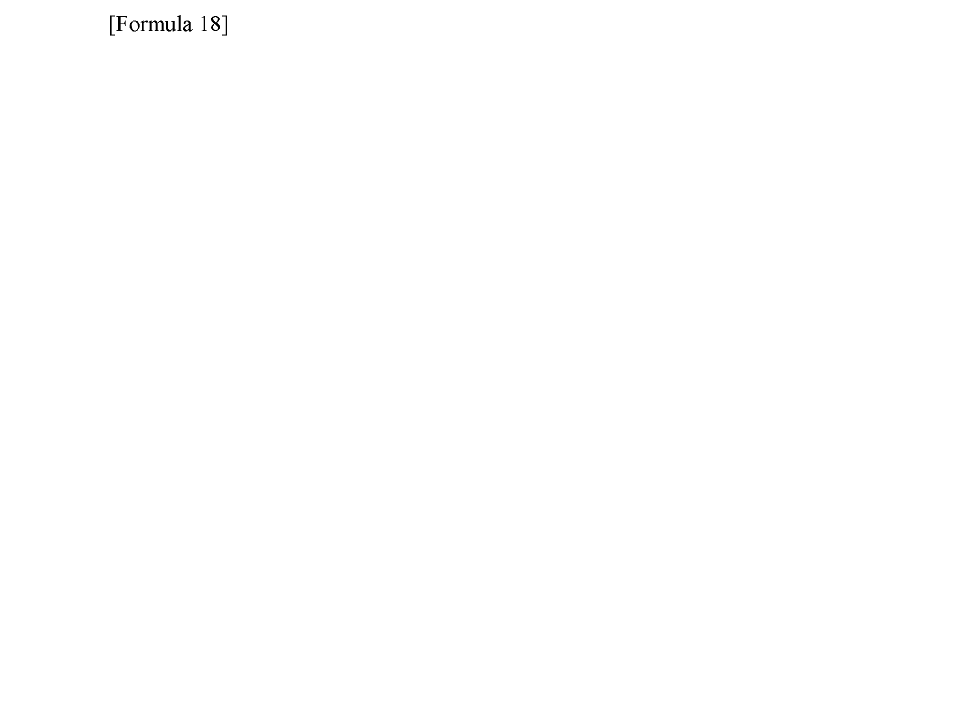

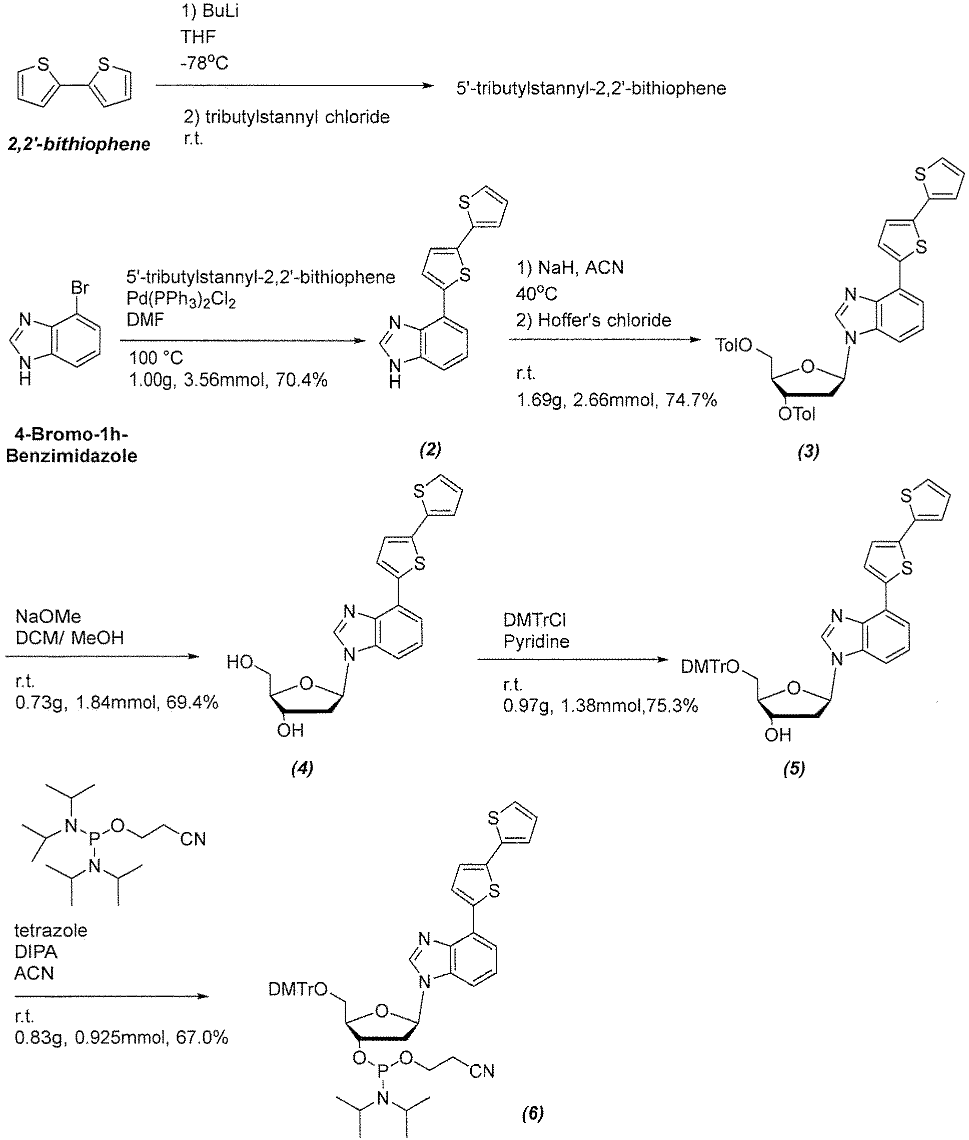

### 5'-tributylstannyl-2,2'-bithiophene

To a stirred solution of 2,2'-bithiophene (1.40 g, 8.42 mmol) in THF (42 mL) at -78°C, n-BuLi (2 M in hexane) (4.21 mL, 8.42 mmol) was added, and the resulting mixture was stirred for 30 minutes. Then, tributylstannyl chloride (2.28 mL, 8.42 mmol) was added. The resulting solution was heated to ambient temperature, and further stirred for 30 minutes. The reaction mixture was added to ice-cold H₂O to be quenched, and extracted with ethyl acetate. The organic layer combined was washed with brine, dried over anhydrous MgSO₄, and filtrated. The resulting filtrate was concentrated in vacuo to obtain a Sn reagent.

### Compound 2: 4-([2,2'-bithiophene]-5-yl)-1H-benzo[d]imidazole

A mixture of 4-bromo-1h-benzimidazole (1.00 g, 5.07 mmol) and Pd(PPh₃)₂Cl₂ (0.178 g, 0.253 mmol) in DMF (25 mL) was degassed in vacuo, and the gas phase was replaced with argon for 10 minutes. Next, 5'-tributylstannyl-2,2'-bithiophene (8.42 mmol) was added. The resulting mixture was further degassed under argon for 10 minutes, and stirred at 100°C for 17 hours. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was precipitated using DCM, whereby a compound 2 as a desired product was obtained in the form of a brown solid (1.00 g, 3.56 mmol, 70.4%) without further purification. ¹H NMR (DMSO-d₆, 400 MHz) δ, ppm: 12.68 (bs, 1H), 8.34 (s, 1H), 8.04 (d, 1H, J = 3.8 Hz), 7.60 - 7.47 (m, 4H). 7.38 - 7.24 (m, 4H), 7.13 - 7.07 (m, 2H).

### Compound 3: (2R,3S,5R)-5-(4-([2,2'-bithiophene]-5-yl)-1H-benzo[d]imidazole-1-yl)-2-(((4-methylbenzoyl)oxy)methyl)tetrahydrofuran-3-yl 4-methylbenzoate

To a mixture of the compound 2 (1.00 g, 3.56 mmol) dissolved in ACN (45 mL), sodium hydride (60%) (0.171 g, 4.27 mmol) was added. The resulting mixture was stirred at ambient temperature for 30 minutes. Then, Hoffer chloride (1.66 g, 4.27 mmol) was added, and the resulting mixture was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 40% EtOAc in hexane), whereby a compound 3 as a product of interest was obtained in the form of a yellow solid (1.69 g, 2.66 mmol, 74.7%). ¹H NMR (DMSO-d₆, 400 MHz) δ, ppm: 8.61 (s, 1H), 8.01 - 7.99 (m, 2H), 7.85 - 7.83 (m, 2H), 7.69 - 7.63 (m, 2H), 7.53 (dd, 1H, J = 1.1, 5.1 Hz), 7.40 - 7.30 (m, 7H), 7.18 (t, 1H, J = 7.9 Hz), 7.12 (dd, 1H, J = 3.6, 5.1 Hz), 6.63 (dd, 1H, J = 6.1, 8.6 Hz), 5.77 - 5.74 (m, 1H), 4.66 - 4.55 (m, 3H), 3.14 - 3.08 (m, 1H), 2.85 - 2.79 (m, 1H), 2.42, 2.36 (2s, 6H).

### Compound 4: (2R,3S,5R)-5-(4-([2,2'-bithiophene]-5-yl)-1H-benzo[d]imidazole-1-yl)-2-(hydroxymethyl)tetrahydrofuran-3-ol

To a solution of the compound 3 (1.69 g, 2.66 mmol) in DCM/MeOH (1:1) (20 mL), sodium methoxide (5 M in MeOH) (1.38 mL, 6.92 mmol) was added, and the resulting solution was stirred at ambient temperature for 30 minutes. The solvent was removed in vacuo to obtain a crude product. The crude product was subjected to silica gel column chromatography (0 to 5% MeOH in DCM), whereby a compound 4 was isolated in the form of a yellow solid (0.73 g, 1.84 mmol, 69.4%). ¹H NMR (DMSO-d₆, 400 MHz) δ, ppm: 8.60 (s, 1H), 8.02 (d, 1H, J = 3.9 Hz), 7.67 - 7.64 (m, 2H), 7.53 (dd, 1H, J = 1.1, 5.1 Hz), 7.38 (dd, 1H, J = 1.1, 3.6 Hz), 7.35 (d, 1H, J = 3.8 Hz), 7.31 (t, 1H, J = 7.9 Hz), 7.12 (dd, 1H, J = 3.6, 5.1 Hz), 6.40 (t, 1H, J = 6.7 Hz), 5.36 (d, 1H, J = 4.2 Hz), 4.98 (t, 1H, J = 5.3 Hz), 4.44 - 4.40 (m, 1H), 3.90 - 3.87 (m, 1H), 3.62 - 3.52 (m, 2H), 2.67 - 2.60 (m, 1H), 2.38 - 2.32 (m, 1H).

### Compound 5: (2R,3S,5R)-5-(4-([2,2'-bithiophene]-5-yl)-1H-benzo[d]imidazole-1-yl)-2-((bis(4-methoxyphenyl)(phenyl)methoxy)methyl)tetrahydrofuran-3-ol

The compound 4 (0.73 g, 1.84 mmol) was evaporated together with pyridine (three times) before pyridine (2.0 mL) and DMTrCl (0.623 g, 1.84 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 100% EtOAc) (1% TEA), whereby a compound 5 as a product of interest was obtained in the form of a light yellow solid (0.97 g, 1.38 mmol, 75.3%). ¹H NMR (DMSO-d₆, 400 MHz) δ, ppm: 8.53 (s, 1H), 8.02 (d, 1H, J = 3.9 Hz), 7.66 (dd, 2H, J = 2.7, 8.0 Hz), 7.53 (dd, 1H, J = 1.1, 5.1 Hz), 7.38 (dd, 1H, J = 1.1, 3.5 Hz), 7.36 (d, 1H, J = 3.8 Hz), 7.31 - 7.11 (m, 11H), 6.78 - 6.72 (m, 4H), 6.45 (t, 1H, J = 6.1 Hz), 5.43 (d, 1H, J = 4.8 Hz), 4.49 - 4.44 (m, 1H), 4.02 - 3.99 (m, 1H), 3.68, 3.66 (2s, 6H), 3.17 - 3.08 (m, 2H), 2.85 - 2.78 (m, 1H), 2.46 - 2.39 (m, 1H).

### Compound 6: (2R,3S,5R)-5-(4-([2,2'-bithiophene]-5-yl)-1H-benzo[d]imidazole-1-yl)-2-((bis(4-methoxyphenyl)(phenyl)methoxy)methyl)tetrahydrofuran-3-yl(2-cyanoethyl)diisopropylphosphoramidite

The compound 5 (0.97 g, 1.38 mmol) and 1*H*-tetrazole in ACN (0.4 M) (1.587 mL, 0.634 mmol) were evaporated (three times) together with ACN before ACN (35 mL), DIPA (0.089 mL, 0.634 mmol), and 2-cyanoethyl *N,N,N',N'*-tetraisopropylphosphoroamidite (0.701 mL, 2.20 mmol) were added. The reaction mixture was stirred at ambient temperature for 3 hours. The reaction was quenched with addition of an aqueous NaHCO₃ solution, and extraction was performed using an aqueous NaHCO₃ solution and ethyl acetate. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 50% EtOAc in hexane) (1% TEA), whereby a compound 6 was obtained in the form of a foamy yellow solid (0.83 g, 0.925 mmol, 67.0%). ¹H NMR (DMSO-d₆, 400 MHz) δ, ppm: 8.52 (d, 1H, J = 2.7 Hz), 8.02 (dd, 1H, J = 1.3, 3.8 Hz), 7.69 - 7.65 (m, 2H), 7.53 (d, 1H, J = 1.0, 5.1 Hz), 7.38 (dd, 1H, J = 1.0, 3.6 Hz), 7.36 (d, 1H, J = 3.8 Hz), 7.33 - 7.11 (m, 11H), 6.80 - 6.74 (m, 4H), 6.48 (dd, 1H, J = 6.2, 12.4 Hz), 4.75 - 4.68 (m, 1H), 4.19 - 4.10 (m, 1H), 3.81 - 3.50 (m, 10H), 3.30 - 3.13 (m, 2H), 2.97 - 2.90 (m, 1H), 2.79 (t, 1H, J = 5.9 Hz), 2.66 (t, 1H, J = 6.0 Hz), 2.62 - 2.57 (m, 1H), 1.15 - 1.11 (m, 10H), 1.01 - 0.99 (m, 2H). ³¹P NMR (DMSO-d₆, 162 MHz) δ, ppm: 147.99, 147.28 (diastereoisomer).

### (8) Chemical synthesis of dYss amidite

The compounds 2 to 6 corresponding to (2) to (6) in the reaction path scheme shown below were synthesized using the following method.

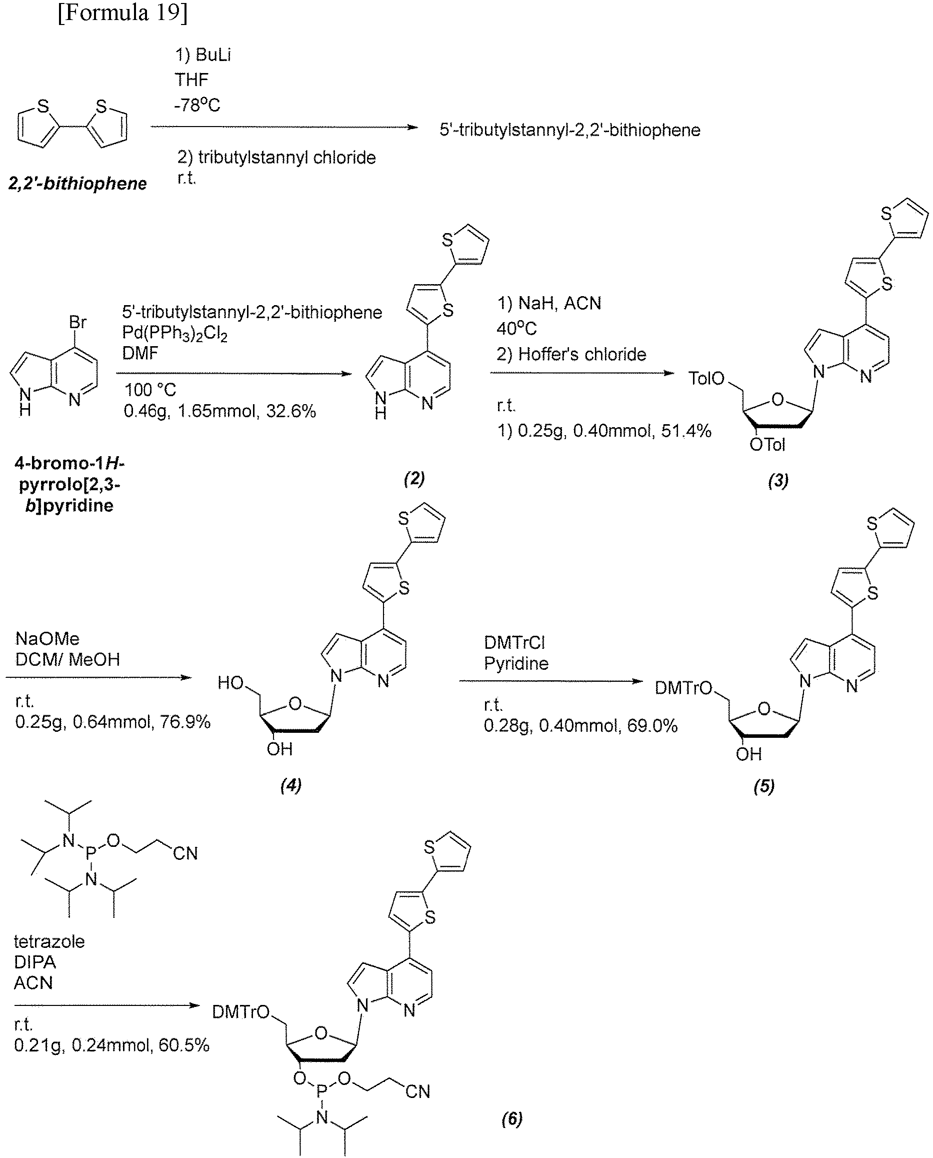

### 5'-tributylstannyl-2,2'-bithiophene

To a stirred solution of 2,2'-bithiophene (1.40 g, 8.42 mmol) in THF (42 mL) at -78°C, n-BuLi (2 M in hexane) (4.21 mL, 8.42 mmol) was added, and the resulting mixture was stirred for 30 minutes. Then, tributylstannyl chloride (2.28 mL, 8.42 mmol) was added. The resulting solution was heated to ambient temperature, and further stirred for 30 minutes. The reaction mixture was added to ice-cold H₂O to be quenched, and was extracted using ethyl acetate. The organic layer combined was washed with brine, dried over anhydrous MgSO₄, and filtrated. The resulting filtrate was concentrated in vacuo to obtain a Sn reagent.

### Compound 2: 4-([2,2'-bithiophene]-5-yl)-1H-pyrrolo[2,3-b]pyridine

A mixture of 4-bromo-1*H*-pyrrolo-[2,3-*b*]pyridine (1.0 g, 5.07 mmol) and Pd(PPh₃)₂Cl₂ (0.178 g, 0.253 mmol) in DMF (25 mL) was degassed in vacuo, and the gas phase was replaced with argon for 10 minutes. Next, 5'-tributylstannyl-2,2'-bithiophene (8.42 mmol) was added. The resulting mixture was further degassed under argon for 10 minutes, and stirred at 100°C for 3 hours. The mixture was cooled to room temperature, and then, the mixture was concentrated in vacuo. The reaction mixture was subjected to liquid extraction using brine and ethyl acetate. The organic layer was dried over anhydrous MgSO₄, and filtrated. The resulting filtrate was concentrated in vacuo to obtain a crude product. The crude product was subjected to silica gel column chromatography using a hexane/EtOAc solvent (20 to 50 to 80% EtOAc in hexane), whereby a compound 2 as a product of interest was obtained in the form of a brown solid (0.46 g, 1.65 mmol, 32.6%). ¹H NMR (DMSO-d₆, 400 MHz) δ, ppm: 11.88 (s, 1H), 8.24 (d, 1H, J = 5.1 Hz), 7.79 (d, 1H, J = 3.9 Hz), 7.64 - 7.54 (m, 4H), 7.45 (dd, 1H, J = 1.2, 3.6 Hz), 7.44 (d, 1H, J = 5.0 Hz), 7.36 (d, 1H, J = 5.0 Hz), 7.15 (dd, 1H, J = 3.6, 5.1 Hz), 6.87 (dd, 1H, J = 1.9, 3.5 Hz).

### Compound 3: (2R,3S,5R)-5-(4-([2,2'-bithiophene]-5-yl)-1H-pyrrolo[2,3-b]pyridine-1-yl)-2-(((4-methylbenzoyl)oxy)methyl)tetrahydrofuran-3-yl 4-methylbenzoate

The compound 2 (0.44 g, 0.78 mmol) was dissolved in ACN (10 mL), and sodium hydride (60%) (0.037 g, 0.936 mmol) was added. The resulting mixture was stirred at ambient temperature for 30 minutes, and further heated at 40°C for 30 minutes. After the resulting mixture was cooled to ambient temperature, Hoffer chloride (0.363 g, 0.936 mmol) was added, and the resulting mixture was further stirred at ambient temperature for 13 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using hexane/EtOAc (0 to 20% EtOAc in hexane) as a solvent, whereby a compound 3 as a product of interest was obtained in the form of a yellow solid (0.25 g, 0.40 mmol, 51.4%). ¹H NMR (DMSO-d₆, 400 MHz) δ, ppm: 8.29 (d, 1H, J = 5.1 Hz), 7.98 (d, 2H, J = 8.2 Hz), 7.90 - 7.88 (m, 3H), 7.82 (d, 1H, J = 3.9 Hz), 7.60 (dd, 1H, J = 1.1, 5.1 Hz), 7.47 - 7.45 (m, 3H), 7.39 (d, 2H, J = 8.0 Hz), 7.33 (d, 2H, J = 8.0 Hz), 7.15 (dd, 1H, J = 3.6, 5.1 Hz), 7.00 (d, 1H, J = 3.8 Hz), 6.90 (dd, 1H, J = 5.9, 8.6 Hz), 5.77 - 5.75 (m, 1H), 4.66 - 4.61 (m, 1H) 4.57 - 4.52 (m, 2H), 3.19 - 3.11 (m, 1H), 2.76 - 2.70 (m, 1H), 2.42, 2.38 (2s, 6H).

### Compound 4: (2R,3S,5R)-5-(4-([2,2'-bithiophene]-5-yl)-1H-pyrrolo[2,3-b]pyridine-1-yl)-2-(hydroxymethyl)tetrahydrofuran-3-ol

To a solution of the compound 3 (0.53 g, 0.84 mmol) in DCM/MeOH (1:1) (6.4 mL), sodium methoxide (5 M in MeOH) (0.436 mL, 2.18 mmol) was added, and the resulting solution was stirred at ambient temperature for 30 minutes. The solvent was removed in vacuo to obtain a crude product. The crude product was subjected to silica gel column chromatography (0 to 5% MeOH in DCM), whereby a compound 4 was isolated in the form of a yellow solid (0.25 g, 0.64 mmol, 76.9%). ¹H NMR (DMSO-d₆, 400 MHz) δ, ppm: 8.27 (d, 1H, J = 5.1 Hz), 7.89 (d, 1H, J = 3.8 Hz), 7.81 (d, 1H, J = 3.9 Hz), 7.59 (dd, 1H, J = 1.2, 5.1 Hz), 7.47 - 7.43 (m, 3H), 7.15 (dd, 1H, J = 3.6, 5.1 Hz), 6.97 (d, 1H, J = 3.8 Hz), 6.75 (dd, 1H, J = 6.0, 8.2 Hz), 5.29 (d, 1H, J = 4.1 Hz), 5.03 (t, 1H, J = 5.6 Hz), 4.41 - 4.37 (m, 1H), 3.87 - 3.84 (m, 1H), 3.62 - 3.50 (m, 2H), 2.61 - 2.54 (m, 1H), 2.26 - 2.21 (m, 1H).

### Compound 5: (2R,3S,5R)-5-(4-([2,2'-bithiophene]-5-yl)-1H-pyrrolo[2,3-b]pyridine-1-yl)-2-((bis(4-methoxyphenyl)(phenyl)methoxy)methyl)tetrahydrofuran-3-ol

The compound 4 (0.24 g, 0.59 mmol) was evaporated together with pyridine (three times) before pyridine (1.0 mL) and DMTrCl (0.201 g, 0.59 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0 to 80% EtOAc) (1% TEA), whereby a compound 5 as a product of interest was obtained in the form of a foamy yellow solid (0.28 g, 0.40 mmol, 69.0%). ¹H NMR (DMSO-d₆, 400 MHz) δ, ppm: 8.28 (d, 1H, J = 5.1 Hz), 7.81 (d, 1H, J = 3.9 Hz), 7.72 (d, 1H, J = 3.8 Hz), 7.60 (dd, 1H, J = 1.2, 5.1 Hz), 7.47 - 7.44 (m, 3H), 7.38 -7.36 (m, 2H), 7.28 - 7.20 (m, 7H), 7.15 (dd, 1H, J = 3.6, 5.1 Hz), 6.94 (d, 1H, J = 3.8 Hz), 6.85 - 6.81 (m, 4H), 6.77 (t, 1H, J = 6.7 Hz), 5.36 (d, 1H, J = 4.5 Hz), 4.43 - 4.38 (m, 1H), 4.05 - 3.95 (m, 1H), 3.71 (2s, 6H), 3.18 - 3.16 (m, 2H), 2.67 - 2.60 (m, 1H), 2.34 - 2.29 (m, 1H).

### Compound 6: (2R,3S,5R)-5-(4-([2,2'-bithiophene]-5-yl)-1H-pyrrolo[2,3-b]pyridine-1-yl)-2-((bis(4-methoxyphenyl)(phenyl)methoxy)methyl)tetrahydrofuran-3-yl(2-cyanoethyl)diisopropylphosphoramidite.

The compound 5 (0.28 g, 0.40 mmol) and 1*H*-tetrazole in ACN (0.4 M) (0.46 mL, 0.184 mmol) were evaporated (three times) together with pyridine before ACN (10 mL), DIPA (0.026 mL, 0.184 mmol), and 2-cyanoethyl *N,N,N',N*'-tetraisopropylphosphorodiamidite (0.203 mL, 0.64 mmol) were added. The reaction mixture was stirred at ambient temperature for 3.5 hours. The reaction was quenched with addition of aq. NaHCO₃, and subsequently, liquid extraction was performed using aq. NaHCO₃ and ethyl acetate. The organic layer combined was washed with water, dried over anhydrous MgSO₄, and filtrated. The resulting filtrate was concentrated in vacuo to obtain a crude product. The crude product was subjected to silica gel column chromatography using a hexane/EtOAc solvent (0 to 30% EtOAc in hexane) (1% TEA), whereby a compound 6 was obtained in the form of a foamy yellow solid (0.21 g, 0.24 mmol, 60.5%). ¹H NMR (DMSO-d₆, 400 MHz) δ, ppm: 8.26 (dd, 1H, J = 0.8, 5.1 Hz), 7.82 (d, 1H, J = 3.9 Hz), 7.75 (d, 1H, J = 3.8 Hz), 7.59 (dd, 1H, J = 1.1, 5.1 Hz), 7.47 - 7.44 (m, 3H), 7.39-7.35 (m, 2H), 7.29 -7.19 (m, 7H), 7.15 (dd, 1H, J = 3.6, 5.1 Hz), 6.97 (dd, 1H, J = 2.1, 3.8 Hz), 6.85 - 6.80 (m, 4H), 6.75 (t, 1H, J = 6.9 Hz), 4.72 - 4.66 (m, 1H), 4.14 - 4.05 (m, 1H), 3.81 - 3.52 (m, 10H), 3.24 - 3.17 (m, 2H), 2.87 - 2.82 (m, 1H), 2.78 (t, 1H, J = 5.9 Hz), 2.68 (t, 1H, J = 5.9 Hz), 2.47 - 2.41 (m, 1H), 1.16 - 1.12 (m, 10H), 1.06 -1.04 (m, 2H). ³¹P NMR (DMSO-d₆, 162 MHz) δ, ppm: 147.57, 146.96 (diastereoisomer).

### (9) Chemical synthesis of dBo amidite

The compounds 2 to 6 corresponding to (2) to (6) in the reaction path scheme shown below were synthesized using the following method.

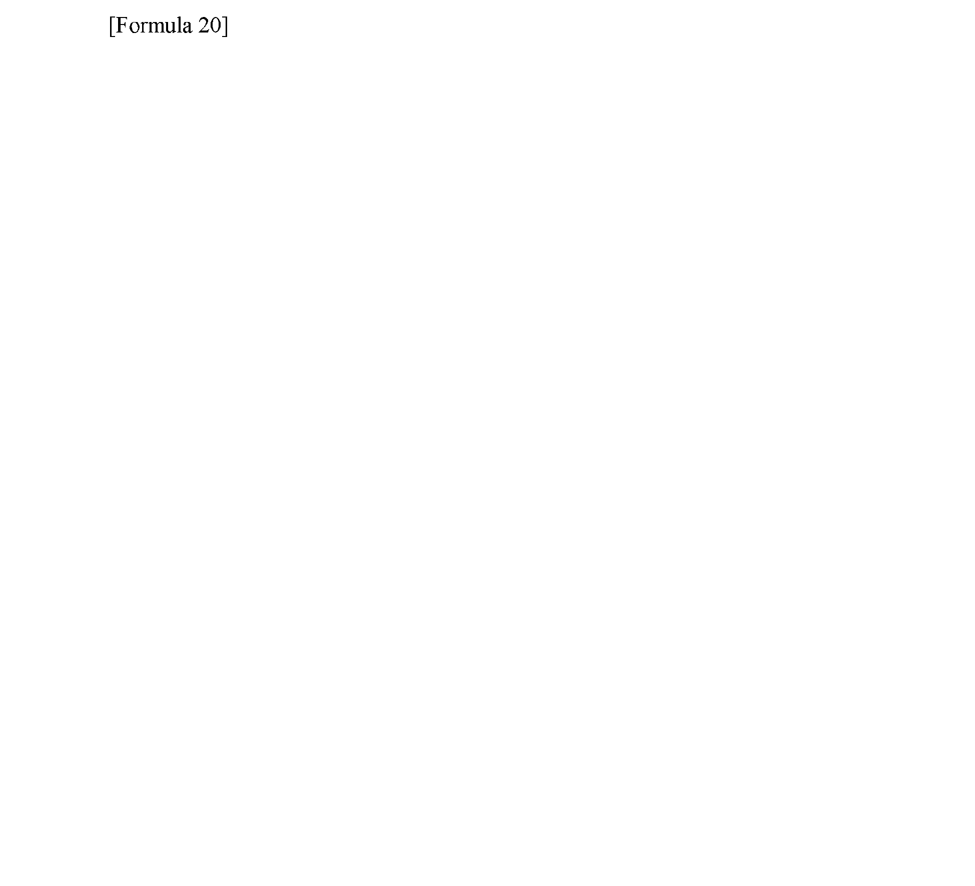

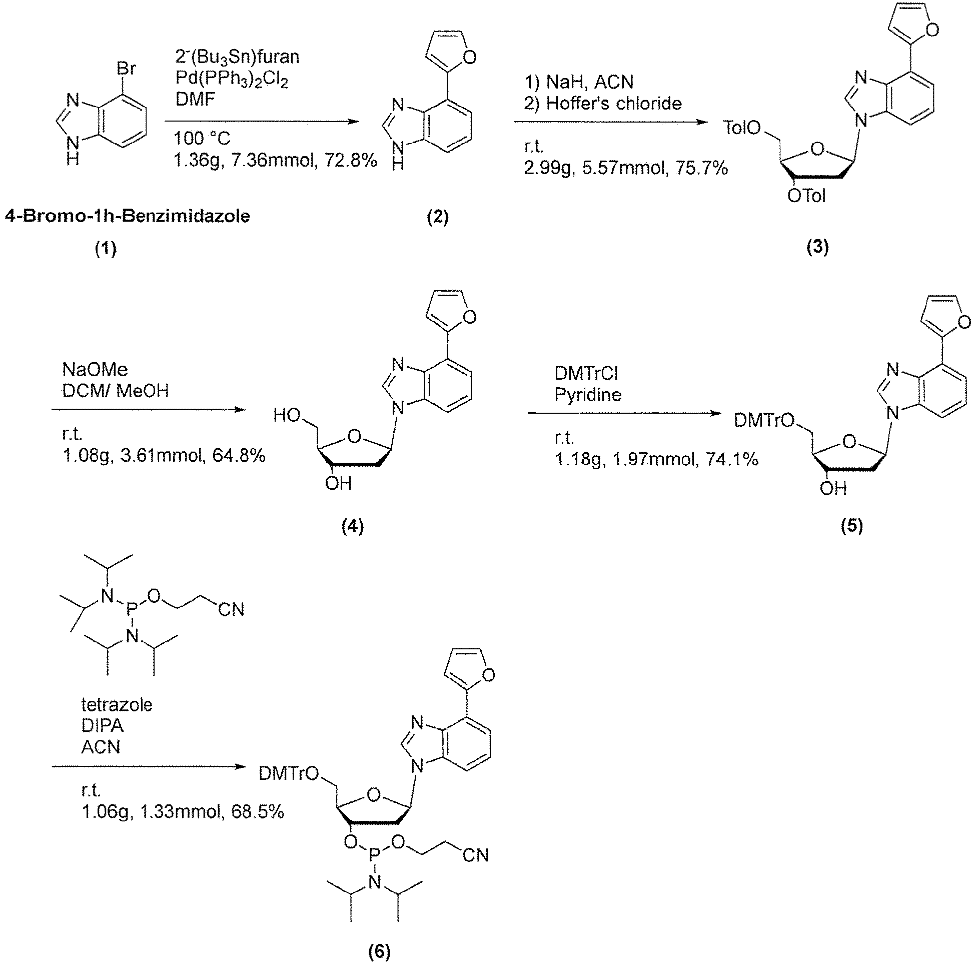

### Compound 2: 4-(furan-2-yl)-1H-benzo[d]imidazole

A mixture of 4-bromo-1h-benzimidazole (2.00 g, 10.1 mmol) and Pd(PPh₃)₂Cl₂ (0.354 g, 0.505 mmol) in DMF (50 mL) was degassed in vacuo, and the gas phase was replaced with argon. After 10 minutes, 2-(tributylstannyl)furan (4.77 mL, 15.15 mmol) was added. Then, the resulting mixture was further degassed under argon for 10 minutes, and stirred at 100°C for 24 hours. The reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (20 to 90% EtOAc in hexane). Thus, a compound 2 as a mixture with an unreacted starting material was obtained in the form of a pink solid (1.36 g, 7.36 mmol, 72.8%).

### Compound 3: (2R,3S,5R)-5-(4-(furan-2-yl)-1H-benzo[d]imidazole-1-yl)-2-(((4-methylbenzoyl)oxy)methyl)tetrahydrofuran-3-yl 4-methylbenzoate.

To a stirred mixture of the compound 2 (1.35 g, 7.36 mmol) in ACN (94 mL), sodium hydride (60%) (0.353 g, 8.83 mmol) was added. The resulting mixture was stirred at ambient temperature for 20 minutes. Then, Hoffer chloride (3.43 g, 8.83 mmol) was added, and the resulting solution was further stirred at ambient temperature for 3 hours. After the solvent was removed, the reaction mixture was subjected to liquid extraction using brine and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (0% to 40% EtOAc in hexane), whereby a compound 3 as a product of interest was obtained in the form of a foamy white solid (2.99 g, 5.57 mmol, 75.7%). ¹H NMR (DMSO-d₆, 400 MHz) δ, ppm: 8.59 (s, 1H), 8.00 - 7.98 (m, 2H), 7.85 - 7.83 (m, 2H), 7.79 (dd, 1H, J = 0.8, 1.8 Hz), 7.67 (dd, 1H, J = 0.8, 8.2 Hz), 7.60 (dd, 1H, J = 0.7, 8.2 Hz), 7.55 (dd, 1H, J = 0.6, 4.0 Hz), 7.40 - 7.38 (m, 2H), 7.32 - 7.30 (m, 2H), 7.21 (t, 1H, J = 7.9 Hz), 6.66 (dd, 1H, J = 1.8, 3.3 Hz), 6.62 (dd, 1H, J = 5.8, 8.4 Hz), 5.76 - 5.74 (m, 1H), 4.66 - 4.54 (m, 3H), 3.15 - 3.07 (m, 1H), 2.84 - 2.78 (m, 1H), 2.41, 2.36 (2s, 6H).

### Compound 4: (2R,3S,5R)-5-(4-(furan-2-yl)-1H-benzo[d]imidazole-1-yl)-2-(hydroxymethyl)tetrahydrofuran-3-ol

To a solution of the compound 3 (2.99 g, 5.57 mmol) in DCM/MeOH (1:1) (42 mL), sodium methoxide (5 M in MeOH) (2.90 mL, 14.5 mmol) was added, and the resulting solution was stirred at ambient temperature for 30 minutes. The solvent was removed in vacuo to obtain a crude product. The crude product was subjected to silica gel column chromatography (0 to 10% MeOH in DCM) and then C18 RP-HPLC (isocratic elution with 25% CH₃CN in H₂O). Then, a compound 4 was isolated in the form of a white solid (1.08 g, 3.61 mmol, 64.8%). ¹H NMR (DMSO-d₆, 400 MHz) δ, ppm: 8.57 (s, 1H), 7.79 (dd, 1H, J = 0.8, 1.8 Hz), 7.66 - 7.60 (m, 2H), 7.55 (dd, 1H, J = 0.7, 3.3 Hz), 7.32 (t, 1H, J = 7.9 Hz), 6.66 (dd, 1H, J = 1.8, 3.3 Hz), 6.39 (dd, 1H, J = 6.3, 7.2 Hz), 5.35 (d, 1H, J = 4.2 Hz), 4.97 (t, 1H, J = 5.3 Hz), 4.43 - 4.39 (m, 1H), 3.90 - 3.87 (m, 1H), 3.62 - 3.52 (m, 2H), 2.66 - 2.59 (m, 1H), 2.36 - 2.30 (m, 1H).

### Compound 5: (2R,3S,5R)-2-((bis(4-methoxyphenyl)(phenyl)methoxy)methyl)-5-(4-(furan-2-yl)-1H-benzo[d]imidazole-1-yl)tetrahydrofuran-3-ol

The compound 4 (0.80 g, 2.66 mmol) was evaporated together with pyridine (three times) before pyridine (2.7 mL) and DMTrCl (0.90 g, 2.66 mmol) were added to the compound. The reaction mixture was stirred at ambient temperature for 1 hour. A workup was performed using liquid extraction with aq. NaHCO₃ and ethyl acetate. The organic layer combined was dried over anhydrous MgSO₄, and filtrated. The resulting filtrate was concentrated in vacuo to obtain a crude product. The crude product was subjected to silica gel column chromatography using a hexane/EtOAc solvent (20 to 100% EtOAc in hexane) (1% TEA), whereby a compound 5 as a desired product was obtained in the form of a foamy white solid (1.18 g, 1.97 mmol, 74.1%). ¹H NMR (DMSO-d₆, 400 MHz) δ, ppm: 8.49 (s, 1H), 7.79 (d, 1H, J = 0.8, 1.8 Hz), 7.67 - 7.60 (m, 2H), 7.57 (dd, 1H, J = 0.7, 3.3 Hz), 7.30 - 7.13 (m, 10H), 6.78 - 6.71 (m, 4H), 6.66 (dd, 1H, J = 1.8, 3.3 Hz), 6.44 (t, 1H, J = 6.2 Hz), 5.42 (d, 1H, J = 4.8 Hz), 4.48 - 4.43 (m, 1H), 4.02 - 3.98 (m, 1H), 3.68, 3.66 (2s, 6H), 3.16 - 3.08 (m, 2H), 2.83 - 2.77 (m, 1H), 2.45 - 2.38 (m, 1H).

### Compound 6: (2R,3S,5R)-2-((bis(4-methoxyphenyl)(phenyl)methoxy)methyl)-5-(4-(furan-2-yl)-1H-benzo[d]imidazole-1-yl)tetrahydrofuran-3-yl(2-cyanoethyl)diisopropylphosphoramidite

The compound 5 (1.18 g, 1.97 mmol) and 1*H*-tetrazole in ACN (0.4 M) (2.27 mL, 0.906 mmol) were evaporated (three times) together with pyridine before ACN (50 mL), DIPA (0.127 mL, 0.906 mmol), and 2-cyanoethyl *N*,*N*,*N'*,*N'*-tetraisopropylphosphorodiamidite (1.00 mL, 3.152 mmol) were added. The reaction mixture was stirred at ambient temperature for 3 hours. The organic layer combined was washed with water, dried over anhydrous MgSO4, and filtrated. The resulting filtrate was concentrated in vacuo to obtain a crude product. The crude product was subjected to silica gel column chromatography using a hexane/EtOAc solvent (0 to 50% EtOAc in hexane) (1% TEA), whereby a compound 6 was obtained in the form of a foamy white solid (1.06 g, 1.33 mmol, 68.5%). ¹H NMR (DMSO-d₆, 400 MHz) δ, ppm: 8.49 (d, 1H, J = 2.6 Hz), 7.79 (m, 1H), 7.68 - 7.56 (m, 3H), 7.33 - 7.13 (m, 10H), 6.79 - 6.73 (m, 4H), 6.67 - 6.65 (m, 1H), 6.47 (dd, 1H, J = 6.3, 12.4 Hz), 4.74 - 4.67 (m, 1H), 4.18 - 4.09 (m, 1H), 3.79 - 3.50 (m, 10H), 3.27 - 3.12 (m, 2H), 2.96 - 2.89 (m, 1H), 2.78 (t, 1H, J = 5.9 Hz), 2.67 - 2.54 (m, 2H), 1.16 - 1.11 (m, 10H), 1.00 - 0.99 (m, 2H). ³¹P NMR (DMSO-d₆, 162 MHz) δ, ppm: 147.98, 147.27 (diastereoisomer).

### <Example 2: Production of nucleic acid aptamer>

### (Purpose)

The purpose was to synthesize a nucleic acid aptamer comprising Ds and/or a Ds alternative that was/were synthesized in Example 1.

### (Method and results)

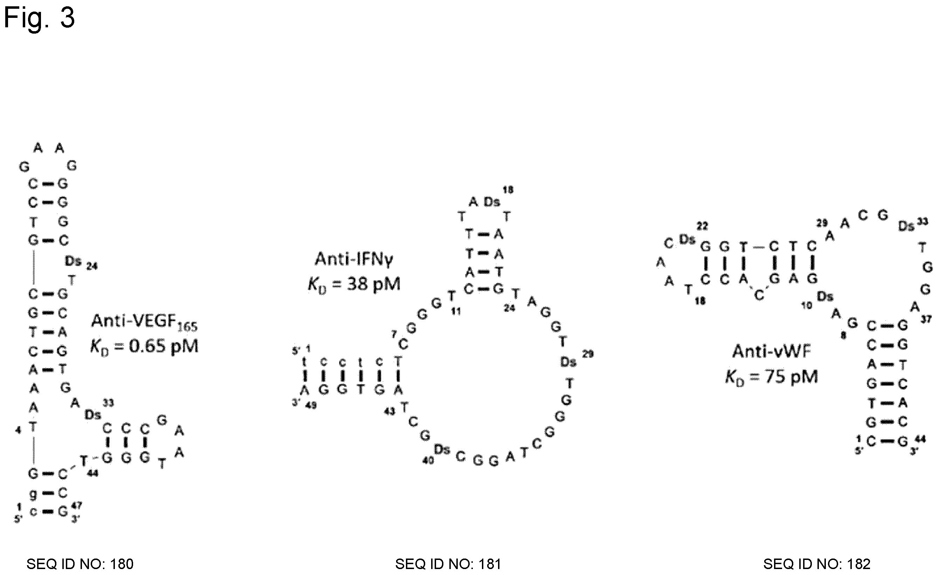

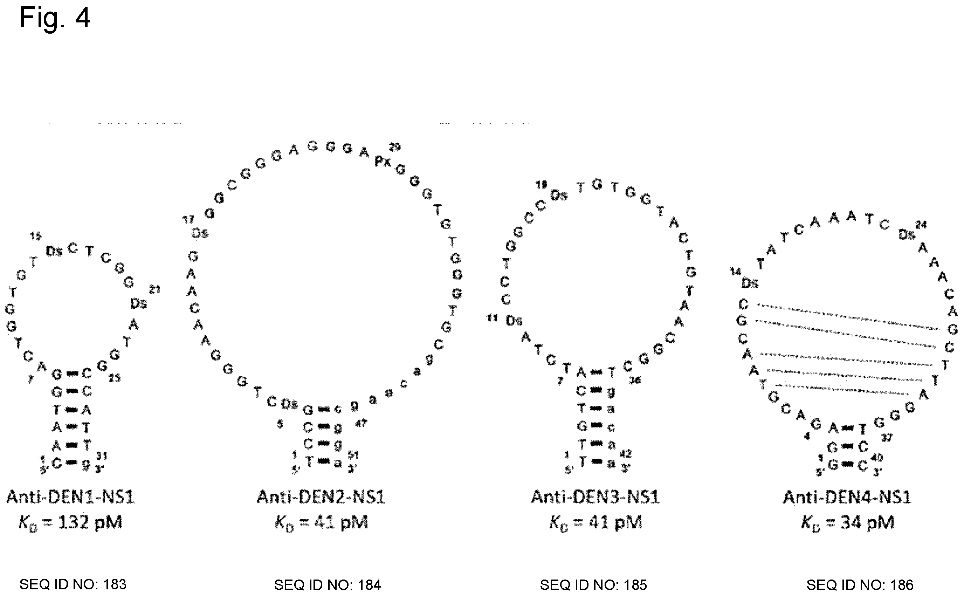

The nucleic acid aptamers shown in Table 1 below are: nucleic acid aptamers that each comprise two Ds's, and that bind to interferon gamma (IFNy), von Willebrand factor A1-domain (vWF), and dengue NS1 protein serum type 1 and serum type 3 (DEN1-NS1 and DEN3-NS1) respectively as a target (which are referred to as "IFNy-DsDs", "vWF-DsDs", "AptD1-DsDs", and "AptD3-DsDs" respectively in Table below; hereinafter often referred to as a "Ds aptamer"); and nucleic acid aptamers resulting from substituting at least one Ds in each nucleic acid aptamer with any of the different Ds alternatives or combinations thereof (hereinafter, the resulting aptamer is often referred to as a "UB aptamer"). The Ds aptamers are represented by Anti-IFNy and Anti-vWF in Figure 3 and Anti-DEN1-NS1 and Anti-DEN3-NS1 in Figure 4, and based on the sequences isolated by ExSELEX (genetic alphabet Expansion for SELEX) using the DNA library comprising a Ds base as a fifth base in the past literature (Kimoto, M., *et al., Nat. Biotechnol.,* 2013, *31*: 453-457.; Matsunaga, K., *et al., J. Am. Chem. Soc.,* 2017, *139*: 324-334.; Matsunaga, K., *et al., Nucleic Acids Res.,* 2021, *49*: 11407-11424.). The Ds aptamers each comprise two Ds bases, and have the biotin-bound mini-hairpin sequence GGCG(biotin-T)AGCC linked therein. IFNy-DsDs and vWF-DsDs have the mini-hairpin sequence GCGAAGC with which an internal stem-loop structure has been substituted. The nucleic acid aptamers shown in Table 1 below were chemically synthesized with a DNA/RNA synthesizer using phosphoramidite chemistry, and using a commercially available natural base amidites, biotin-dT amidites, and unnatural base amidites synthesized described above. The nucleic acid aptamers were deprotected with a concentrated ammonia solution. Then, the resulting full-length DNA fragments were purified using denaturing polyacrylamide gel electrophoresis.

**[Table 1-1]**

| Table 1: List of nucleic acid aptamers | | | | |
|---|---|---|---|---|
| Name of nucleic acid aptamer | Base sequence of nucleic acid aptamer * "L" in the sequence represents Biotin-dT, and Ds or a Ds alternative represented by "I" is shown in the right column. | I on the 5' side | I on the 3' side | SEQ ID NO: |
| AptIFNy-DsDs | CCCGCCCGGGTCCGCGAAGCGGTAGGTITGGGCT AGGCIGCTGGCGGGCGCGLAGCG | Ds | Ds | 21 |
| AptIFNγ-BsBs | CCCGCCCGGGTCCGCGAAGCGGTAGGTITGGGCT AGGCIGCTGGCGGGCGCGLAGCG | Bs | Bs | 21 |
| AptIFNγ-IsIs | CCCGCCCGGGTCCGCGAAGCGGTAGGTITGGGCT AGGCIGCTGGCGGGCGCGLAGCG | Is | Is | 21 |
| AptIFNγ-YsYs | CCCGCCCGGGTCCGCGAAGCGGTAGGTITGGGCT AGGCIGCTGGCGGGCGCGLAGCG | Ys | Ys | 21 |
| AptIFNγ-YiYi | CCCGCCCGGGTCCGCGAAGCGGTAGGTITGGGCT AGGCIGCTGGCGGCGCGLAGCG | Yi | Yi | 21 |
| AptIFNγ-DoDo | CCCGCCCGGGTCCGCGAAGCGGTAGGTITGGGCT AGGCIGCTGGCGGGCGCGLAGCG | Do | Do | 21 |
| AptIFNγ-DpDp | CCCGCCCGGGTCCGCGAAGCGGTAGGTITGGGCT AGGCIGCTGGCGGGCGCGLAGCG | Dp | Dp | 21 |
| AptIFNγ-DssDss | CCCGCCCGGGTCCGCGAAGCGGTAGGTITGGGCT AGGCIGCTGGCGGGCGCGLAGCG | Dss | Dss | 21 |
| AptIFNγ-DtDt | CCCGCCCGGGTCCGCGAAGCGGTAGGTITGGGCT AGGCIGCTGGCGGGCGCGLAGCG | Dt | Dt | 21 |
| AptIFNγ-YoYo | CCCGCCCGGGTCCGCGAAGCGGTAGGTITGGGCT AGGCIGCTGGCGGGCGCGLAGCG | Yo | Yo | 21 |
| AptIFNγ-PsPs | CCCGCCCGGGTCCGCGAAGCGGTAGGTITGGGCT AGGCIGCTGGCGGGCGCGLAGCG | Ps | Ps | 21 |
| AptIFNγ-EsEs | CCCGCCCGGGTCCGCGAAGCGGTAGGTITGGGCT AGGCIGCTGGCGGGCGCGLAGCG | Es | Es | 21 |
| AptIFNγ-DsYs | CCCGCCCGGGTCCGCGAAGCGGTAGGTITGGGCT AGGCIGCTGGCGGGCGCGLAGCG | Ds | Ys | 21 |
| AptIFNγ-YsDs | CCCGCCCGGGTCCGCGAAGCGGTAGGTITGGGCT AGGCIGCTGGCGGGCGCGLAGCG | Ys | Ds | 21 |
| AptIFNγ-YsYo | CCCGCCCGGGTCCGCGAAGCGGTAGGTITGGGCT AGGCIGCTGGCGGGCGCGLAGCG | Ys | Yo | 21 |
| AptIFNγ-YoYs | CCCGCCCGGGTCCGCGAAGCGGTAGGTITGGGCT AGGCIGCTGGCGGGCGCGLAGCG | Yo | Ys | 21 |

**[Table 1-2]**

| | | | | |
|---|---|---|---|---|
| AptvWF-DsDs | CGCGGCCGAIGAGCACCGAAGGTCTCAACGITGG AGGCCGCGCGCGTAGCG | Ds | Ds | 7 |
| AptvWF-BsBs | CGCGGCCGAIGAGCACCGAAGGTCTCAACGITGG AGGCCGCGCGCGTAGCG | Bs | Bs | 7 |
| AptvWF-IsIs | CGCGGCCGAIGAGCACCGAAGGTCTCAACGITGG AGGCCGCGCGCGTAGCG | Is | Is | 7 |
| AptvWF-YsYs | CGCGGCCGAIGAGCACCGAAGGTCTCAACGITGG AGGCCGCGCGCGTAGCG | Ys | Ys | 7 |
| AptvWF-YiYi | CGCGGCCGAIGAGCACCGAAGGTCTCAACGITGG AGGCCGCGCGCGTAGCG | Yi | Yi | 7 |
| AptvWF-DoDo | CGCGGCCGAIGAGCACCGAAGGTCTCAACGITGG AGGCCGCGCGCGTAGCG | Do | Do | 7 |
| AptvWF-DpDp | CGCGGCCGAIGAGCACCGAAGGTCTCAACGITGG AGGCCGCGCGCGTAGCG | Dp | Dp | 7 |
| AptvWF-DssDss | CGCGGCCGAIGAGCACCGAAGGTCTCAACGITGG AGGCCGCGCGCGTAGCG | Dss | Dss | 7 |
| AptvWF-DtDt | CGCGGCCGAIGAGCACCGAAGGTCTCAACGITGG AGGCCGCGCGCGTAGCG | Dt | Dt | 7 |
| AptvWF-YoYo | CGCGGCCGAIGAGCACCGAAGGTCTCAACGITGG AGGCCGCGCGCGTAGCG | Yo | Yo | 7 |
| AptvWF-PsPs | CGCGGCCGAIGAGCACCGAAGGTCTCAACGITGG AGGCCGCGCGCGTAGCG | Ps | Ps | 7 |
| AptvWF-EsEs | CGCGGCCGAIGAGCACCGAAGGTCTCAACGITGG- AGGCCGCGCGCGTAGCG | Es | Es | 7 |
| AptvWF-BsYs | CGCGGCCGAIGAGCACCGAAGGTCTCAACGITGG AGGCCGCGCGCGTAGCG | Bs | Ys | 7 |
| AptvWF-BsYo | CGCGGCCGAIGAGCACCGAAGGTCTCAACGITGG AGGCCGCGCGCGTAGCG | Bs | Yo | 7 |
| AptvWF-YsBs | CGCGGCCGAIGAGCACCGAAGGTCTCAACGITGG AGGCCGCGCGCGTAGCG | Ys | Bs | 7 |
| AptD1-DsDs | CCCCAGACGGACTGGTGTICTCGGIATGGCCGTC TGGGGCGCGTAGCG | Ds | Ds | 33 |
| AptD1-BsBs | CCCCAGACGGACTGGTGTICTCGGIATGGCCGTC TGGGGCGCGTAGCG | Bs | Bs | 33 |
| AptD1-IsIs | CCCCAGACGGACTGGTGTICTCGGIATGGCCGTC TGGGGCGCGTAGCG | Is | Is | 33 |

**[Table 1-3]**

| | | | | |
|---|---|---|---|---|
| AptD1-YsYs | CCCCAGACGGACTGGTGTICTCGGIATGGCCGTC TGGGGCGCGTAGCG | Ys | Ys | 33 |
| AptD1-YiYi | CCCCAGACGGACTGGTGTICTCGGIATGGCCGTC TGGGGCGCGTAGCG | Yi | Yi | 33 |
| AptD1-DoDo | CCCCAGACGGACTGGTGTICTCGGIATGGCCGTC TGGGGCGCGTAGCG | Do | Do | 33 |
| AptD1-DpDp | CCCCAGACGGACTGGTGTICTCGGIATGGCCGTC TGGGGCGCGTAGCG | Dp | Dp | 33 |
| AptD1-DssDss | CCCCAGACGGACTGGTGTICTCGGIATGGCCGTC TGGGGCGCGTAGCG | Dss | Dss | 33 |
| AptD1-DtDt | CCCCAGACGGACTGGTGTICTCGGIATGGCCGTC TGGGGCGCGTAGCG | Dt | Dt | 33 |
| AptD1-YoYo | CCCCAGACGGACTGGTGTICTCGGIATGGCCGTC TGGGGCGCGTAGCG | Yo | Yo | 33 |
| AptD1-PsPs | CCCCAGACGGACTGGTGTICTCGGIATGGCCGTC TGGGGCGCGTAGCG | Ps | Ps | 33 |
| AptD1-EsEs | CCCCAGACGGACTGGTGTICTCGGIATGGCCGTC TGGGGCGCGTAGCG | Es | Es | 33 |
| AptD1-DsYs | CCCCAGACGGACTGGTGTICTCGGIATGGCCGTC TGGGGCGCGTAGCG | Ds | Ys | 33 |
| AptD1-YsDs | CCCCAGACGGACTGGTGTICTCGGIATGGCCGTC TGGGGCGCGTAGCG | Ys | Ds | 33 |
| AptD1-DsBs | CCCCAGACGGACTGGTGTICTCGGIATGGCCGTC TGGGGCGCGTAGCG | Ds | Bs | 33 |
| AptD1-BsDs | CCCCAGACGGACTGGTGTICTCGGIATGGCCGTC TGGGGCGCGTAGCG | Bs | Ds | 33 |
| AptD1-DsDt | CCCCAGACGGACTGGTGTICTCGGIATGGCCGTC TGGGGCGCGTAGCG | Ds | Dt | 33 |
| AptD1-DtDs | CCCCAGACGGACTGGTGTICTCGGIATGGCCGTC TGGGGCGCGTAGCG | Dt | Ds | 33 |
| AptD1-YsBs | CCCCAGACGGACTGGTGTICTCGGIATGGCCGTC TGGGGCGCGTAGCG | Ys | Bs | 33 |
| AptD1-YoBs | CCCCAGACGGACTGGTGTICTCGGIATGGCCGTC TGGGGCGCGTAGCG | Yo | Bs | 33 |
| AptD1-YsmBs | CCCCAGACGGACTGGTGTICTCGGIATGGCCGTC TGGGGCGCGTAGCG | Ysm | Bs | 33 |

**[Table 1-4]**

| | | | | |
|---|---|---|---|---|
| AptD1-BsYs | CCCCAGACGGACTGGTGTICTCGGIATGGCCGTC TGGGGCGCGTAGCG | Bs | Ys | 33 |
| AptD3-DsDs | CCGCTTGTCATCTAICCTGGCCITGTGGTACTGT AACGGCTGACAAGCGG | Ds | Ds | 44 |
| AptD3-BsBs | CCGCTTGTCATCTAICCTGGCCITGTGGTACTGT AACGGCTGACAAGCGG | Bs | Bs | 44 |
| AptD3-IsIs | CCGCTTGTCATCTAICCTGGCCITGTGGTACTGT AACGGCTGACAAGCGG | Is | Is | 44 |
| AptD3-YsYs | CCGCTTGTCATCTAICCTGGCCITGTGGTACTGT AACGGCTGACAAGCGG | Ys | Ys | 44 |
| AptD3-YiYi | CCGCTTGTCATCTAICCTGGCCITGTGGTACTGT AACGGCTGACAAGCGG | Yi | Yi | 44 |
| AptD3-DoDo | CCGCTTGTCATCTAICCTGGCCITGTGGTACTGT AACGGCTGACAAGCGG | Do | Do | 44 |
| AptD3-DpDp | CCGCTTGTCATCTAICCTGGCCITGTGGTACTGT AACGGCTGACAAGCGG | Dp | Dp | 44 |
| AptD3-DssDss | CCGCTTGTCATCTAICCTGGCCITGTGGTACTGT AACGGCTGACAAGCGG | Dss | Dss | 44 |
| AptD3-DtDt | CCGCTTGTCATCTAICCTGGCCITGTGGTACTGT AACGGCTGACAAGCGG | Dt | Dt | 44 |
| AptD3-YoYo | CCGCTTGTCATCTAICCTGGCCITGTGGTACTGT AACGGCTGACAAGCGG | Yo | Yo | 44 |
| AptD3-PsPs | CCGCTTGTCATCTAICCTGGCCITGTGGTACTGT AACGGCTGACAAGCGG | Ps | Ps | 44 |
| AptD3-EsEs | CCGCTTGTCATCTAICCTGGCCITGTGGTACTGT AACGGCTGACAAGCGG | Es | Es | 44 |
| AptD3-YsDss | CCGCTTGTCATCTAICCTGGCCITGTGGTACTGT AACGGCTGACAAGCGG | Ys | Dss | 44 |
| AptD3-DssYs | CCGCTTGTCATCTAICCTGGCCITGTGGTACTGT AACGGCTGACAAGCGG | Dss | Ys | 44 |
| AptD3-YssYss | CCGCTTGTCATCTAICCTGGCCITGTGGTACTGT AACGGCTGACAAGCGG | Yss | Yss | 44 |
| AptD3-YsmYsm | CCGCTTGTCATCTAICCTGGCCITGTGGTACTGT AACGGCTGACAAGCGG | Ysm | Ysm | 44 |
| AptD3-YssYs | CCGCTTGTCATCTAICCTGGCCITGTGGTACTGT AACGGCTGACAAGCGG | Yss | Ys | 44 |

**[Table 1-5]**

| | | | | |
|---|---|---|---|---|
| AptD3-YssYo | CCGCTTGTCATCTAICCTGGCCITGTGGTACTGT AACGGCTGACAAGCGG | Yss | Yo | 44 |
| AptD3-YssYsm | CCGCTTGTCATCTAICCTGGCCITGTGGTACTGT AACGGCTGACAAGCGG | Yss | Ysm | 44 |
| AptD3-YsmYss | CCGCTTGTCATCTAICCTGGCCITGTGGTACTGT AACGGCTGACAAGCGG | Ysm | Yss | 44 |

| | | | | |
|---|---|---|---|---|
| * The underlines denote sequences complementary to each other, and capable of forming a stem structure; the black frame denotes a hairpin sequence; and "L" in the sequence represents Biotin-dT. | | | | |

### <Example 3: Binding assay of nucleic acid aptamer>

### (Purpose)

The purpose was to evaluate the affinity of the nucleic acid aptamers produced in Example 2, to a target protein.

### (Method)

The affinity of each kind of nucleic acid aptamer to a target protein was evaluated using the below-described EMSA, SPR method, and ELISA.

### (1) EMSA (gel-electrophoresis mobility shift assay)

EMSA was performed by staining, with SYBR Gold, the gel after electrophoresis, and detecting, with a bio-imager, a band pattern shifted on the gel. The band densities of the nucleic acid aptamers bound to a target and of free nucleic acid aptamers were quantitated using the software attached to the bio-imager. The relative binding (%) was calculated by normalizing the ratio of the band of the Ds aptamer shifted in the same gel. The experimental conditions for EMSA are shown in Table 2 below. In this regard, the Ds aptamers-IFNy-DsDs, vWF-DsDs, AptD1-DsDs, and AptD3-DsDs-and the UB aptamers derived from the respective Ds aptamers are hereinafter referred to as the "IFNy-targeting nucleic acid aptamer", "vWF-targeting nucleic acid aptamer", "DEN1-NS1-targeting nucleic acid aptamer", and "DEN3-NS1-targeting nucleic acid aptamer" respectively.

**[Table 2]**

| Table 2: Experimental conditions for EMSA | | | | |
|---|---|---|---|---|
| Kind of nucleic acid aptamer | Target protein (Concentration) | Binding buffer | Gel (comprising 5% glycerol) | Electrophoresis buffer |
| IFNy-targeting nucleic acid aptamer | IFNγ (50 nM) | 1× PBS, 0.05% Nonidet P-40 | 8% polyacrylamide (29:1), 0.5×TBE, 3 M Urea | 0.5×TBE |
| vWF-targeting nucleic acid aptamer | vWF (50 nM) | | | |
| DEN1-NS1-targeting nucleic acid aptamer | DEN1-NS1 (25 nM, hexamer) | 20 mM Tris-HCl (pH 7.5), 150 mM NaCl, 1 mM MgCl₂, 2.7 mM KCl | 4% polyacrylamide (29:1), 0.5×TB, 1 mM MgCl₂, 2.7 mM KCl | 0.5× TB, 1 mM MgCl₂, 2.7 mM KCl |
| DEN3-NS1-targeting nucleic acid aptamer | DEN3-NS1 (25 nM, hexamer) | | | |

### (2) SPR method (surface plasmon resonance method)

To compare the binding affinity profile between the Ds aptamers and the UB aptamers, an SPR analysis was performed using a Biacore T200 (GE Healthcare Technologies, Inc.) platform. Each nucleic acid aptamer (ligand) was immobilized on a flow cell of a streptavidin-coated sensor chip by injecting a 0.5 nM ligand solution into a running buffer. As the running buffer, 1 × PBS comprising 0.05% Nonidet P-40 was used for the vWF-targeting nucleic acid aptamer, 1 × PBS comprising 50 mM NaCl 0.05% Nonidet P-40 was used for the IFNy-targeting nucleic acid aptamer, and 20 mM Tris-HCl at a pH of 7.5, 150 mM NaCl, 1 mM MgCl₂, 2.7 mM KCl, and 0.05% Tween 20 were used for the DEN1-NS1-targeting nucleic acid aptamer and the DEN3-NS1-targeting nucleic acid aptamer. The conditions were set as follows: a flow rate of 5 µL/minute at 25°C for 150 to 960 seconds. A 0.15625 nM to 40 nM target protein solution was prepared in a running buffer, and injected at a flow rate of 100 µL/minute. The association time was set to 150 seconds, and the dissociation time was set to 450 seconds (for the IFNy-targeting nucleic acid aptamer and the vWF-targeting nucleic acid aptamer) or 600 seconds (for the DEN1-NS1-targeting nucleic acid aptamer and the DEN3-NS1-targeting nucleic acid aptamer). The sensor chip was regenerated with a 5-second injection of 50 mM NaOH, followed by a 10-minute equilibration with the running buffer. Using BIAevaluation T200 software version 1.0 (GE Healthcare Technologies, Inc.), the K_{D} values were determined by fitting the blank-subtracted kinetics data with a 1:1 binding model. The K_{D} values of the Ds aptamers (IFNy-DsDs, vWF-DsDs, AptD1-DsDs, and AptD3-DsDs) are shown in Figures 3 and 4.

### (3) ELISA

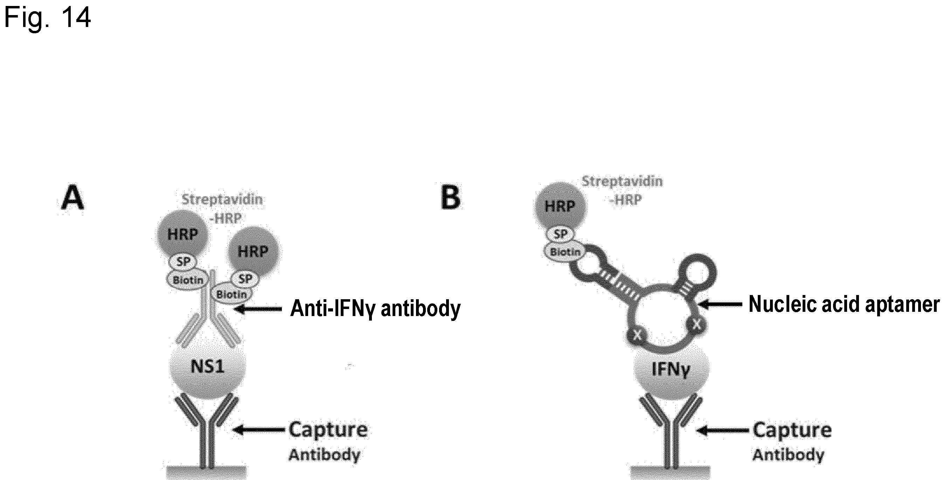

A commercially available ELISA kit was used in accordance with the protocol of the manufacturer (#88-7316-88, Thermo Fisher Scientific Inc.). A biotinylated antibody for detection in this ELISA kit was substituted with each kind of nucleic acid aptamer to measure the sensitivity of an IFNγ antibody (Figure 14). The antibody detection concentration used in the protocol was estimated to be 120 nM from the absorbance of a stock solution at 280 nm, and thus, a biotinylated aptamer at 120 nM as the standard working concentration was used. As the target proteins, two kinds of recombinant human IFNγ proteins-a product (#300-02; the amino acid residue at position 160 is Q) of Peprotech and a product (#ab51240; the amino acid residue at position 160 is R) of Abcam plc-were used.

### (Results)

### (1) IFNy-targeting nucleic acid aptamer

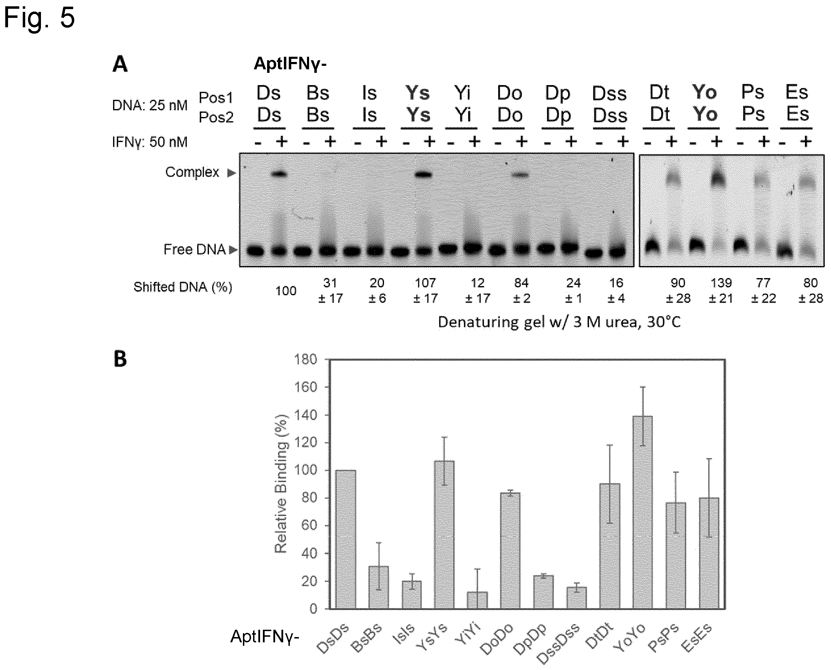

Figure 5 shows the results obtained by analyzing the affinity of the Ds aptamer (AptIFNγ-DsDs) and 11 kinds of UB aptamers (AptIFNy-BsBs, AptIFNγ-IsIs, AptIFNγ-YsYs, AptIFNγ-YiYi, AptIFNγ-DoDo, AptIFNγ-DpDp, AptIFNγ-DssDss, AptIFNγ-DtDt, AptIFNγ-YoYo, AptIFNγ-PsPs, and AptIFNy-EsEs) to IFNγ, using EMSA. The AptIFNγ-YsYs and AptIFNγ-YoYo aptamers exhibited a stronger band shift than the AptIFNγ-DsDs aptamer. The K_{D} values determined using the SPR method were 42.4 pM for AptIFNγ-DsDs, 19.5 pM for AptIFNγ-YsYs, and 24.4 pM for AptIFNγ-YoYo. These two kinds of UB aptamers had an increased binding capacity than the Ds aptamers.

### (2) vWF-targeting nucleic acid aptamer

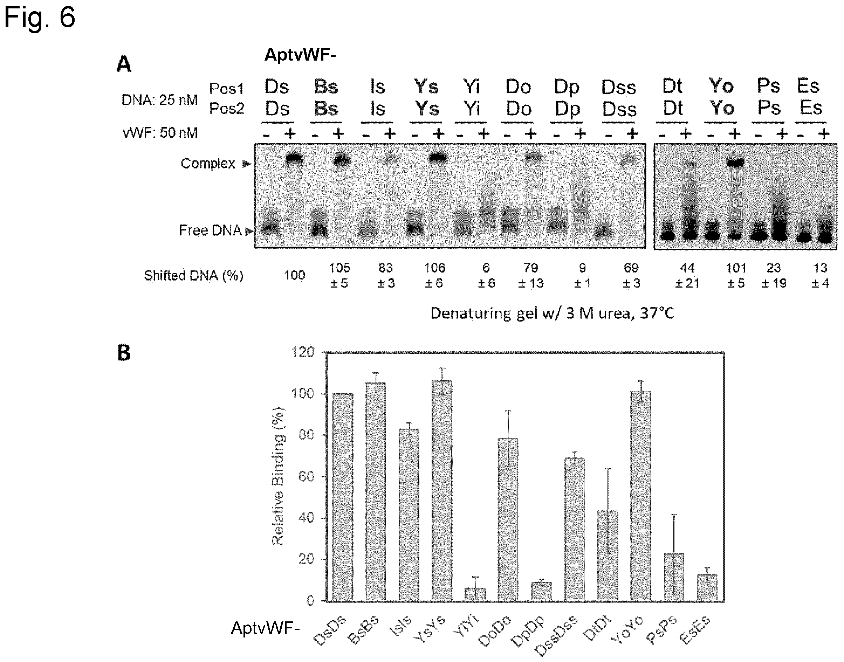

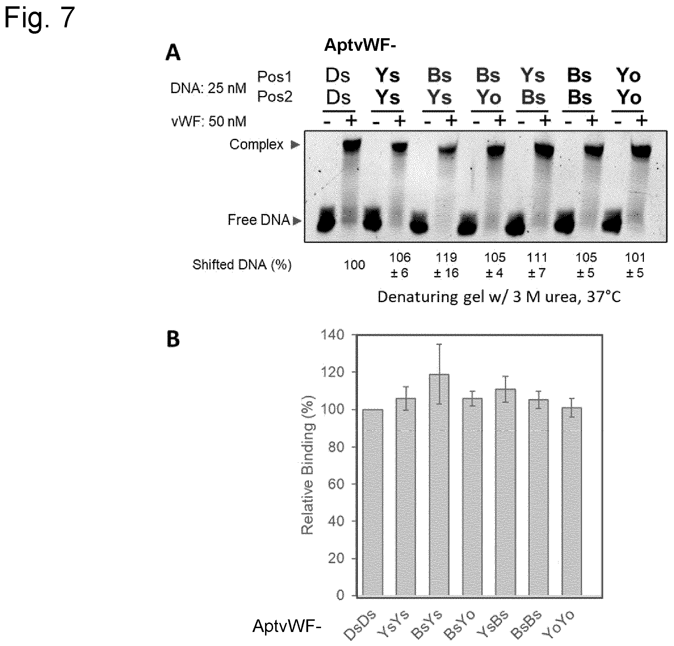

Figure 6 shows the results obtained by analyzing the affinity of the Ds aptamer (AptvWF-DsDs) and 11 kinds of UB aptamers (AptvWF-BsBs, AptvWF-IsIs, AptvWF-YsYs, AptvWF-YiYi, AptvWF-DoDo, AptvWF-DpDp, AptvWF-DssDss, AptvWF-DtDt, AptvWF-YoYo, AptvWF-PsPs, and AptvWF-EsEs) to vWF, using EMSA. The AptvWF-YsYs and AptvWF-YoYo aptamers and the AptvWF-BsBs aptamer exhibited a band shift the same as or stronger than the AptvWF-DsDs aptamer. Next, Figure 7 shows the results obtained by analyzing the affinity of three kinds of UB aptamers (AptvWF-BsYs, AptvWF-BsYo, and AptvWF-YsBs) to vWF, using EMSA, in which the UB aptamers resulted from substituting two Ds's in AptvWF-DsDs with a combination of different Ds alternatives. AptvWF-BsYs, AptvWF-BsYo, and AptvWF-YsBs exhibited a stronger band shift than the AptvWF-DsDs aptamer. The K_{D} values determined using the SPR method were 54.7 pM for AptvWF-DsDs, 24.0 pM for AptvWF-BsBs, 24.2 pM for AptvWF-BsYs, 22.3 pM for AptvWF-BsYo, and 27.4 pM for AptvWF-YsBs. It has been revealed that these UB aptamers had an increased binding capacity than the Ds aptamers, and that AptvWF-BsYo had the highest affinity.

### (3) DEN1-NS1-targeting nucleic acid aptamer

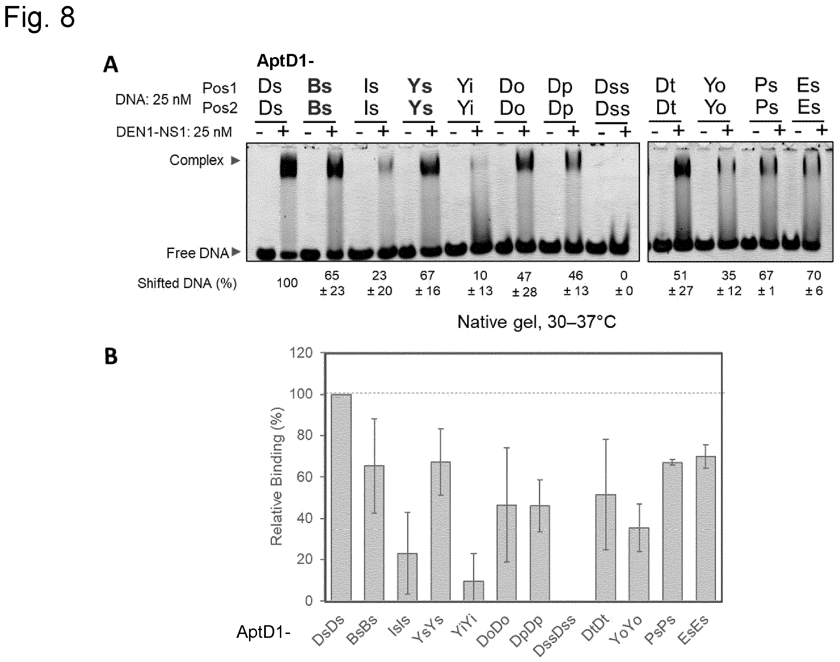

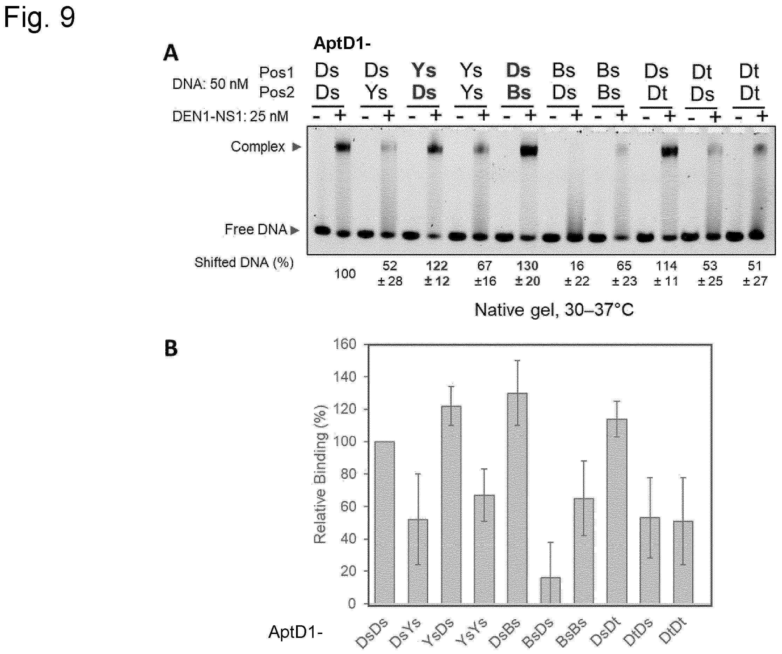

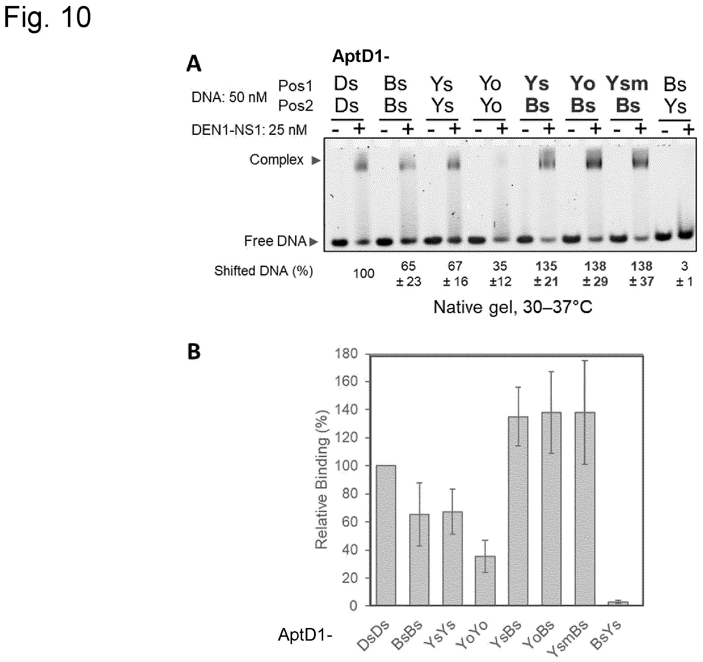

Figure 8 shows the results obtained by analyzing the affinity of the Ds aptamer (AptD1-DsDs) and 11 kinds of UB aptamers (AptD1-BsBs, AptD1-IsIs, AptD1-YsYs, AptD1-YiYi, AptD1-DoDo, AptD1-DpDp, AptD1-DssDss, AptD1-DtDt, AptD1-YoYo, AptD1-PsPs, and AptD1-EsEs) to DEN1-NS1, using EMSA. All of the UB aptamers exhibited a weaker binding than the AptIFNy-DsDs aptamer. However, AptD1-BsBs, AptD1-YsYs, and AptD1-DtDt exhibited a clear band shift, and thus, the aptamers (AptD1-DsYs, AptD1-YsDs, AptD1-DsBs, AptD1-BsDs, AptD1-DsDt, and AptD1-DtDs) comprising, in combination, Bs, Ys, and/or Dt in addition to Ds were prepared, and analyzed using EMSA (Figures 9 and 10). As a result, AptD1-YsDs, AptD1-BsDs, and AptD1-DsDt (Figure 9) and AptD1-YsBs, AptD1-YoBs, and AptD1-YsmBs (Figure 10) exhibited a stronger band shift than the AptIFNy-DsDs aptamer. The K_{D} values determined using the SPR method were 254 to 439 pM for AptD1-DsDs, 48.6 pM for AptD1-DsBs, 35.9 to 44.2 pM for AptD1-YsBs, 56.2 pM for AptD1-YoBs, 63.1 pM for AptD1-YsmBs, and 8.55 pM for AptD1-BsYs. AptD1-YsBs and AptD1-BsYs exhibited the strongest affinity.

### (4) DEN3-NS1-targeting nucleic acid aptamer

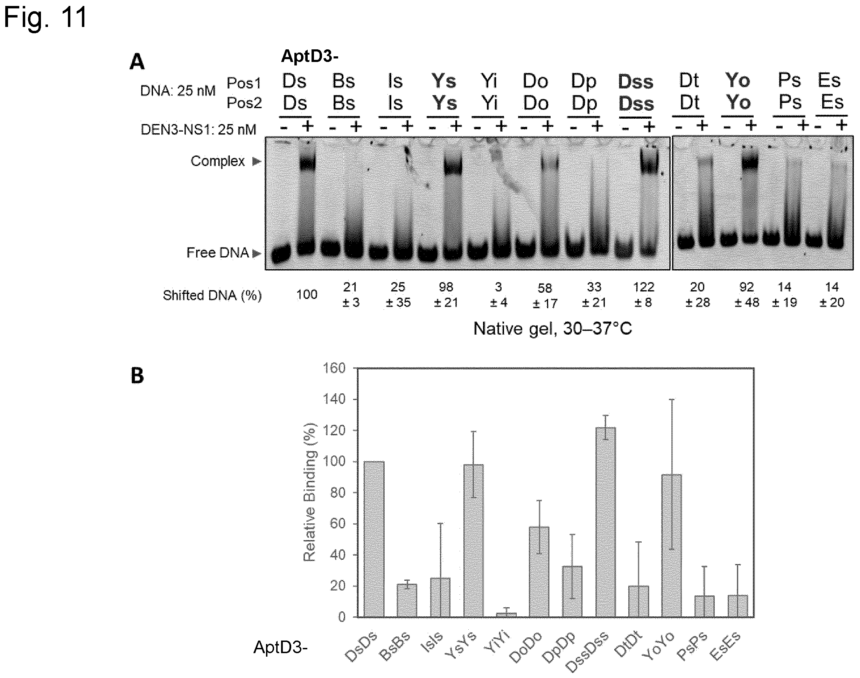

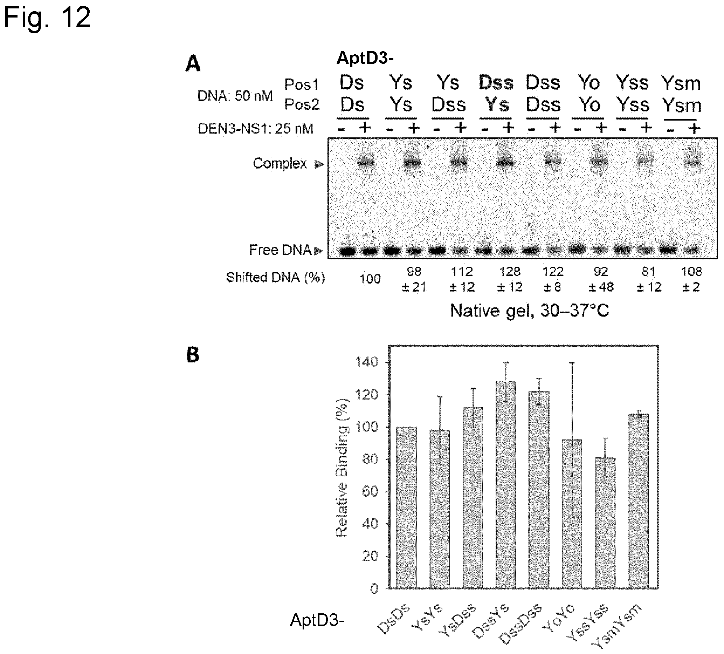

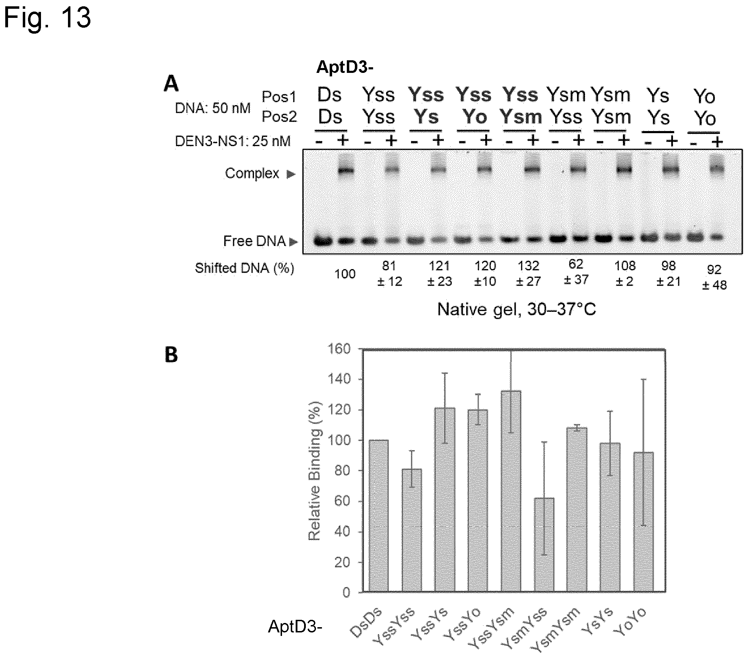

Figure 11 shows the results obtained by analyzing the affinity of the Ds aptamer (AptD3-DsDs) and 11 kinds of UB aptamers (AptD3-BsBs, AptD3-IsIs, AptD3-YsYs, AptD3-YiYi, AptD3-DoDo, AptD3-DpDp, AptD3-DssDss, AptD3-DtDt, AptD3-YoYo, AptD3-PsPs, and AptD3-EsEs) to DEN3-NS1, using EMSA. The AptD3-YsYs, AptD3-DssDss, and AptD3-YoYo aptamers exhibited a relatively strong band shift. On the basis of this result, the aptamers (AptD3-YsDss, AptD3-DssYs, AptD3-YssYs, AptD3-YssYo, AptD3-YssYsm, and AptD3-YsmYss) comprising Ds, Ys, Dss, Yss, Yo, and/or Ysm in combination were produced and analyzed using EMSA (Figures 12 and 13). As a result, AptD3-YsDss, AptD3-DssYs, AptD3-DssDss, and AptD3-YsmYsm (Figure 12) and AptD3-YssYs, AptD3-YssYo, AptD3-YssYsm, and AptD3-YsmYsm (Figure 13) exhibited a stronger band shift than the AptIFNγ-DsDs aptamer. The K_{D} values determined using the SPR method were 71.4 pM for AptD3-DsDs, 41.9 pM for AptD3-DssYs, 41.9 pM for AptD3-YssYs, 55.7 pM for AptD3-YssYo, and 91.1 pM for AptD3-YssYsm. AptD3-DssYs and AptD3-YssYs exhibited the strongest affinity.

### (5) Comparison between IFNy-targeting nucleic acid aptamer and anti-IFNγ antibody (ELISA)

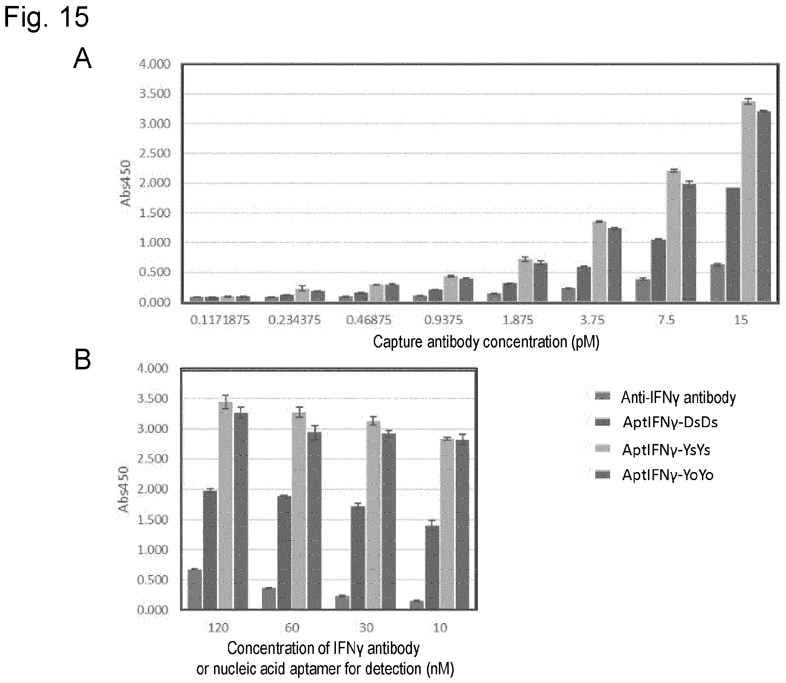

The detection sensitivity of the AptIFNγ-DsDs, AptIFNγ-YsYs, and AptIFNγ-YoYo aptamers to IFNγ was studied using ELISA. ELISA was performed using, for detection, a 120 nM nucleic acid aptamer and an anti-IFNγ antibody. As a result, all of the AptIFNγ-DsDs, AptIFNγ-YsYs, and AptIFNγ-YoYo aptamers exhibited a higher signal intensity than an anti-IFNγ antibody that was an accessory of an ELISA kit (#88-7316-88, Thermo Fisher Scientific Inc.) (Figure 15A). In addition, the AptIFNγ-YsYs and AptIFNγ-YoYo aptamers exhibited a markedly higher signal intensity than AptIFNy-DsDs.

Next, the dependence on the detection reagent concentration was studied. According to the protocol of the ELISA kit, 120 nM is recommended as the concentration of a detection antibody to be used. As the concentration of the antibody was decreased, the signal intensity was decreased markedly. On the other hand, it has been revealed that the signal intensity was not significantly affected even if the concentration of each of AptIFNγ-DsDs, AptIFNγ-YsYs, and AptIFNγ-YoYo used was decreased, and that each of AptIFNγ-YsYs and AptIFNγ-YoYo, even at 10 nM, was detected with sufficient sensitivity (Figure 15B).

### <Example 4: Isolation of new IFNy-targeting nucleic acid aptamer, Thrombin-targeting nucleic acid aptamer, and HMGB1-targeting nucleic acid aptamer on the basis of 6-base ExSELEX>

### (Purpose)

The purpose was to isolate, using ExSELEX, a new nucleic acid aptamer targeted at IFNγ, nucleic acid aptamer targeted at Thrombin, and nucleic acid aptamer targeted at HMGB 1.

### (Method and results)

### (1) Isolation and analysis of new IFNy-targeting nucleic acid aptamer

### (a) 6-base ExSELEX

To discover a new nucleic acid aptamer that binds to human IFNγ, ExSELEX-in which two of the three kinds of unnatural bases represented by the following general formulas (XVIII) to (XX) were used in addition to four natural bases-(hereinafter, the ExSELEX is referred to as "6-base ExSELEX" to be distinguished from conventional 5-base ExSELEX) was performed. In this regard, the unnatural base (7-(2-thienyl)-3H-imidazo[4,5-b]pyridine-3-yl) represented by the following general formula (XVIII) is herein referred to as "Ds" or a "Ds base". In addition, the unnatural base (4-propynylpyrrole-2-carbaldehyde) represented by the following general formula (XIX) is herein referred to as "Pa'" or a "Pa' base". The unnatural base (2-nitro-4-propynylpyrrole) represented by the following general formula (XX) is herein referred to as "Px" or a "Px base". 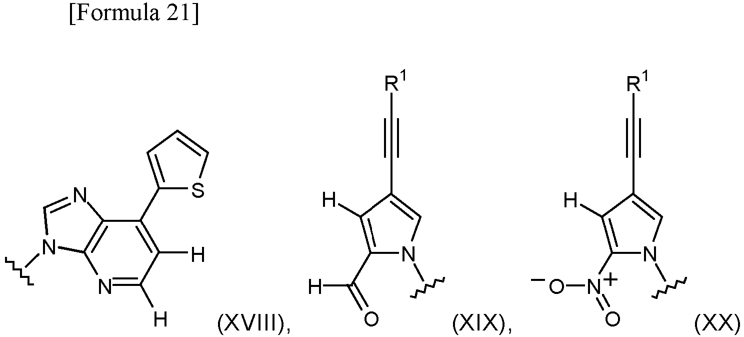 (wherein, R¹ represents a methyl group).

An XL1 library and an XL2 library (comprising 46 sublibraries) were produced as two different libraries having two Ds bases located at different positions in the respective 29-mer random regions consisting of five kinds of bases, i.e., the unnatural base 4-propynylpyrrole-2-carbaldehyde (hereinafter referred to as "Pa'" or a "Pa' base") in addition to the base A, base G, base C, and base T. The XL1 library comprises a region consisting of seven bases located on the 5' side of the random region and a region consisting of six bases located on the 3' side of the random region, comprises a primer sequence for PCR on each of the 5' side and the 3' side further from the regions, and comprises 40 kinds of sublibraries. The XL2 library comprises a region consisting of four bases located on the 5' side of the random region and a region consisting of three bases located on the 3' side of the random region, comprises a 5-base complementary sequence and a primer sequence for PCR on each of the 5' side and the 3' side further from the regions, and comprises 46 kinds of sublibraries.

Each sublibrary was chemically synthesized using a standard phosphoramidite method with amidites of four natural bases, Ds and Pa', and purified with denaturing polyacrylamide gel electrophoresis (PAGE). In ExSELEX, the sublibraries were mixed in equivalent amounts.

As a first selection, a 3.6 nmol library (comprising 2.2 × 10¹⁵ different sequences consisting of six bases) was used. A 50 nM or 100 nM library was rapidly cooled from 90°C to 4°C, and mixed with a target protein (human IFNy).

### (b) XL1 library

The 6-base ExSELEX was performed, using, as a target protein, a commercially available glycosylated human IFNγ expressed in a HEK293 cell, and using the above-described XL1 library. A library in each stage of the 6-base ExSELEX was incubated with a target protein in a binding buffer, and the target protein was biotinylated with an NHS-activated biotin reagent. A biotinylated protein having a DNA fragment bound thereto was captured with streptavidin-bound magnetic beads, and a DNA fragment in a complex on the beads was eluted with 150 mM NaOH. The DNA fragment isolated was amplified by PCR using dDsTP (deoxynucleoside triphosphate comprising a Ds base) and dPxTP (deoxynucleoside triphosphate comprising the base 2-nitro-4-propynylpyrrole (hereinafter referred to as "Px" or a "Px base")) in addition to dNTP. The Pa' base in the first library was substituted with Px during the PCR amplification, and used in the next selection. After the fifth to seventh selection, theDNA-protein complex on the beads was washed with 2 to 3 M urea. After the seventh selection, it was verified using EMSA that the library concentrated bound to a target protein. As the final eighth selection, the DNA fragment bound was isolated from a complex in a band that shifted in gel.

The concentrated library after the seventh selection (R7) and the concentrated library after the eighth selection (R8), obtained from the XL1 library, were amplified using a replacement PCR method in which an unnatural base in the library was replaced with a natural base. In deep sequencing performed using an Ion-PGM system, 87,013 (R7) and 78,581 (R8) extracted sequence reads were obtained, and classified into a family having similar sequences. A higher-level family larger in the number of reads was analyzed. Part of the family was further modified to obtain the IFNy-targeting nucleic acid aptamers shown in Table 3 below.

**[Table 3]**

| Table 3: IFNγ-targeting nucleic acid aptamer obtained from XL1 library | | |
|---|---|---|
| Name of nucleic acid aptamer | Base sequence of nucleic acid aptamer (5'-3') | SEQ ID NO: |
| AptIFNγ-1 | CTCCGC AGTC TGTGGTGGGACPa'GGGCTDsATGTADsGGGATCT GACAA GCGGAG | 55 |
| AptIfNγ-2 | CTCCGC AGTC TGTGGTGGGACT GGGCTA ATGTAA GGGATCT GACAA GCGGAG | 161 |
| AptIFNγ-3 | CTCCGC GTC TGTGGTGGGACPa'GGGCTDsATGTADsGGGATCT GAC GCGGAG | 57 |
| AptIFNγ-4 | CTCCGC GTC TGTGGTGGGACT GGGCTDsATGTADsGGGATCT GAC GCGGAG | 58 |
| AptIFNγ-5 | CTCCGC GTC TGTGGTGGGACPa'GGGCTA ATGTADsGGGATCT GAC GCGGAG | 60 |
| AptIFNγ-6 | CTCCGC GTC TGTGGTGGGACPa'GGGCTDsATGTAA GGGATCT GAC GCGGAG | 162 |
| Bio-AptIFNγ-3 | CTCCGC GTC TGTGGTGGGACPa'GGGCTDsATGTADsGGGATCT GAC GCGGAG CGCGLAGCG | 163 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Pa'" represents a Pa' base. | | |

The affinity of the IFNy-targeting nucleic acid aptamer shown in Table 3 above to human glycosylated IFNγ was analyzed using EMSA. The nucleic acid aptamer was incubated with the human glycosylated IFNγ at 25°C for 30 minutes in a binding buffer supplemented with 0.05% Nonidet P-40 or Tween 20. The sample solution was mixed with glycerol (at a final concentration of 5%), and the resulting complex was analyzed with native 8% PAGE at 26 to 28°C. The other conditions were the same as in Example 3.

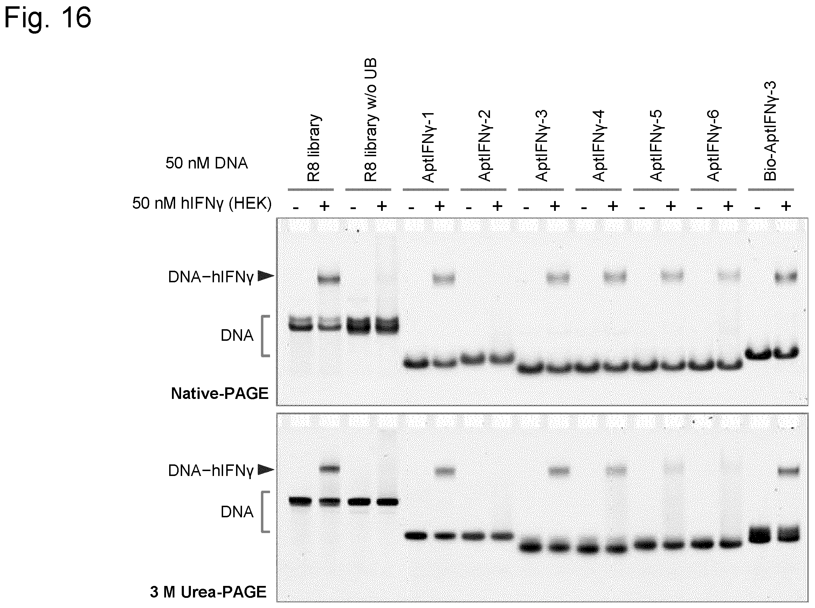

The results are shown in Figure 16. The nucleic acid aptamers (AptIFNy-1, AptIFNγ-3, and Bio-AptIFNy-3) comprising two Ds's and one Pa' bound strongly to the glycosylated IFNγ, but the nucleobase not comprising these artificial bases did not exhibit a band shift.

Next, the above-described novel nucleic acid aptamers were compared with AptDs-IFNγ that is an IFNy-targeting nucleic acid aptamer reported in the past, and with a biotinylated mini-hairpin DNA conjugate (Bio-AptDs-IFNγ) of the AptDs-IFNγ. AptDs-IFNγ and Bio-AptDs-IFNγ are aptamers isolated using the 5-base ExSELEX, and using, as a target protein, a non-glycosylated human IFNγ expressed in *Escherichia coli.* The sequences of the aptamers are shown in Table 4 below.

**[Table 4]**

| Table 4: IFNγ-targeting nucleic acid aptamer used as comparative control | | |
|---|---|---|
| Name of nucleic acid aptamer | Base sequence of nucleic acid aptamer (5'-3') | SEQ ID NO: |
| AptDs-IFNγ | CCCGCC CGGGTCCGCGAAGCGGTAGGTDsTGGCTAGGCDsGCT GGCGGG | 164 |
| Bio-AptDs-IFNγ | CCCGCC CGGGTCCGCGAAGCGGTAGGTDsTGGGCTAGGCDsGCT GGCGGG CGCGLAGCG | 165 |

| | | |
|---|---|---|
| * The underlines denote sequences complementary to each other, and capable of forming a stem structure; the black frame denotes a hairpin sequence; "L" in the sequence represents Biotin-dT; and "Ds" represents a Ds base. | | |

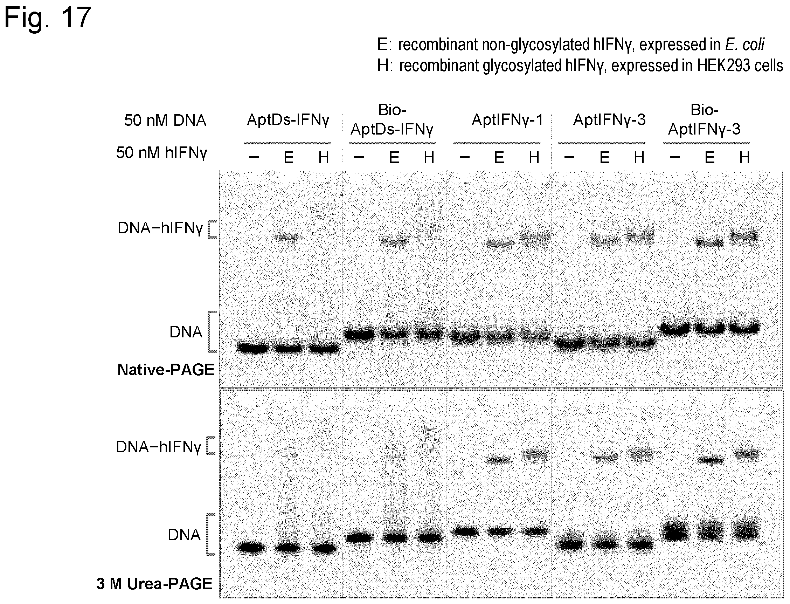

Regarding AptIFNγ-1, AptIFNy-3, and Bio-AptIFNy-3 that were derived from the XL1 library, and AptDs-IFNγ and Bio-AptDs-IFNγ as comparative controls, EMSA was performed in the same manner as the above-described method. The results are shown in Figure 17. It has been revealed that AptDs-IFNγ and Bio-AptDs-IFNγ exhibited a significant decrease in binding to glycosylated IFNγ, but that on the other hand, AptIFNγ-1, AptIFNy-3, and Bio-AptIFNγ-3 bound efficiently to both glycosylated IFNγ and non-glycosylated IFNγ.

### (c) XL2 library

The 6-base ExSELEX was performed using a glycosylated IFNγ as a target protein, and using the XL2 library. The concentrated library after the seventh selection was sequenced using the Ion-PGM sequencer system. A higher-level family larger in the number of reads was analyzed. Part of the family was further modified to obtain the IFNy-targeting nucleic acid aptamers shown in Table 5 below.

**[Table 5]**

| Table 5: IFNy-targeting nucleic acid aptamer obtained from XL2 library | | |
|---|---|---|
| Name of nucleic acid aptamer | Base sequence of nucleic acid aptamer (5'-3') | SEQ ID NO: |
| XL2-01a | GTCCTCACG CCGT CATA A TTGTTDs TTTTGAT CCTTGDs AGTGGGGA CGTGAGGAC | 62 |
| XL2-01b | GTCCTCACG CCGPa'CATA A TTGTTDs TTTTGAT CCTTGDs AGTGGGGA CGTGAGGAC | 166 |
| XL2-01c | GTCCTCACG CCGT CATPa'A TTGTTDs TTTTGAT CCTTGDs AGTGGGGA CGTGAGGAC | 68 |
| XL2-01d | GTCCTCACG CCGT CATA Pa'TTGTTDs TTTTGAT CCTTGDs AGTGGGGA CGTGAGGAC | 70 |
| XL2-01e | GTCCTCACG CCGT CATA A TTGTTDs TTTTGAPa'CCTTGDs AGTGGGGA CGTGAGGAC | 167 |
| XL2-01h | GTCCTCACG CCGT CATDs A TTGTTDs TTTTGAT CCTTGDs AGTGGGGA CGTGAGGAC | 64 |
| XL2-01i | GTCCTCACG CCGT CATA Ds TTGTTDs TTTTGAT CCTTGDs AGTGGGGA CGTGAGGAC | 66 |
| XL2-01j | GTCCTCACG CCGT CATA A TTGTTDs TTTTGAT CCTTGPa'AGTGGGGA CGTGAGGAC | 168 |
| XL2-01k | GTCCTCACG CCGT CATDs A TTGTTDs TTTTGAT CCTTGPa'AGTGGGGA CGTGAGGAC | 169 |
| XL2-01l | GTCCTCACG CCGT CATA DsTTGTTDs TTTTGAT CCTTGPa'AGTGGGGA CGTGAGGAC | 170 |

| | | |
|---|---|---|
| * The underlines denote sequences complementary to each other, and capable of forming a stem structure; "Ds" in the base sequence represents a Ds base; and "Pa'" represents a Pa' base. | | |

The affinity of the IFNy-targeting nucleic acid aptamer shown in Table 5 above to glycosylated IFNγ and non-glycosylated IFNγ was analyzed using EMSA. The nucleic acid aptamer was incubated with IFNγ at 25°C for 30 minutes in a binding buffer supplemented with 0.05% Nonidet P-40 or Tween 20. The sample solution was mixed with glycerol (at a final concentration of 5%), and the resulting complex was analyzed with native 8% PAGE at 26 to 28°C. The other conditions were the same as in Example 3.

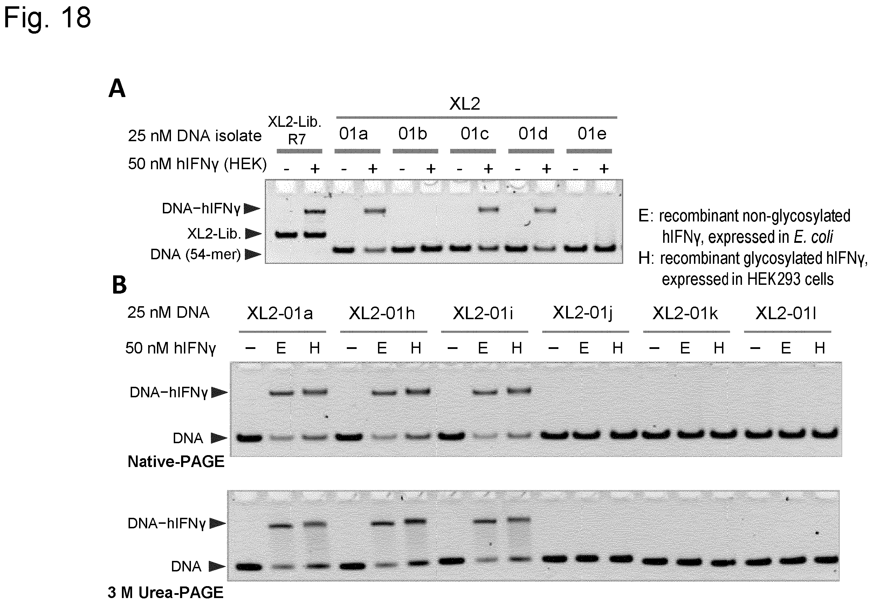

The results are shown in Figure 18. XL2-01a, XL2-01h, and XL2-01i exhibited high affinity to glycosylated IFNγ and non-glycosylated IFNγ also in the 3M urea gel.

Next, a biotin-bound mini-hairpin was linked to XL2-01a and XL2-01i to produce IFNγ-targeting nucleic acid aptamers shown in Table 6 below.

**[Table 6]**

| Table 6: IFNy-targeting nucleic acid aptamer having biotin-bound mini-hairpin linked therein | | |
|---|---|---|
| Name of nucleic acid aptamer | Base sequence of nucleic acid aptamer (5'-3') | SEQ ID NO: |
| Bio-XL2-01a | GTCCTCACG CCGTCATAA TTGTTDs TTTTGATCCTTGDsAGTGGGGA CGTGAGGAC CGCGLAGCG | 171 |
| Bio-XL2-01i | GTCCTCACG CCGTCATADsTTGTTDs TTTTGATCCTTGDsAGTGGGGA CGTGAGGAC CGCGLAGCG | 172 |
| Bio-AptDs-IFNγ | CCCGCC CGGGTCCGCGAAGCGGTAGGTDsTGGGCTAGGCDsGCT GGCGGG CGCGLAGCG | 165 |

| | | |
|---|---|---|
| * The underlines denote sequences complementary to each other, and capable of forming a stem structure; the black frame denotes a hairpin sequence; and "L" in the sequence represents Biotin-dT. | | |

The affinity of the IFNy-targeting nucleic acid aptamer shown in Table 6 above to glycosylated IFNγ was analyzed using ELONA (enzyme-linked oligonucleotides assay). Specifically, human IFNγ (0 to 25 nM) was fixed in a 96-well maxisorp plate, and blocked with 1% BSA. A biotinylated aptamer (100 nM/100 µL well) as a primary detector was added, and bound for 1 hour. Then, SA-HRP as a secondary detector was bound for 30 minutes to allow coloring detection. Reaction between HRP and TMB was allowed for 30 minutes. The reaction was terminated with 1 N HCl. Absorbance at 450 nm was measured using NanoDrop.

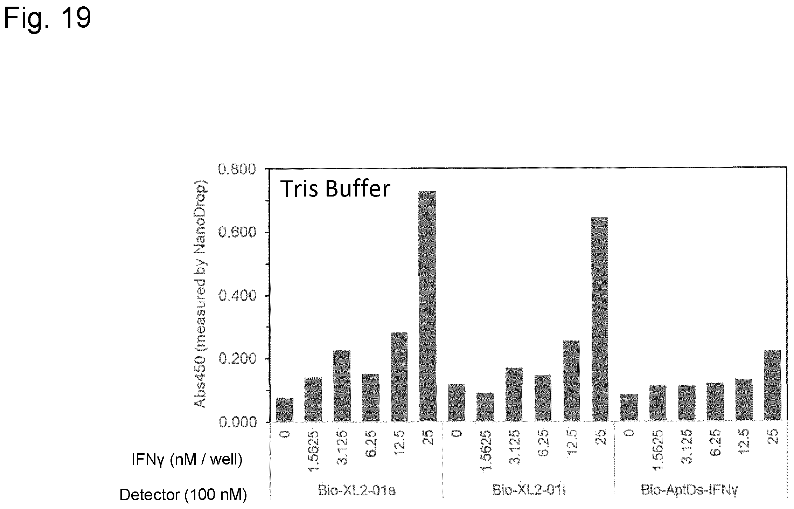

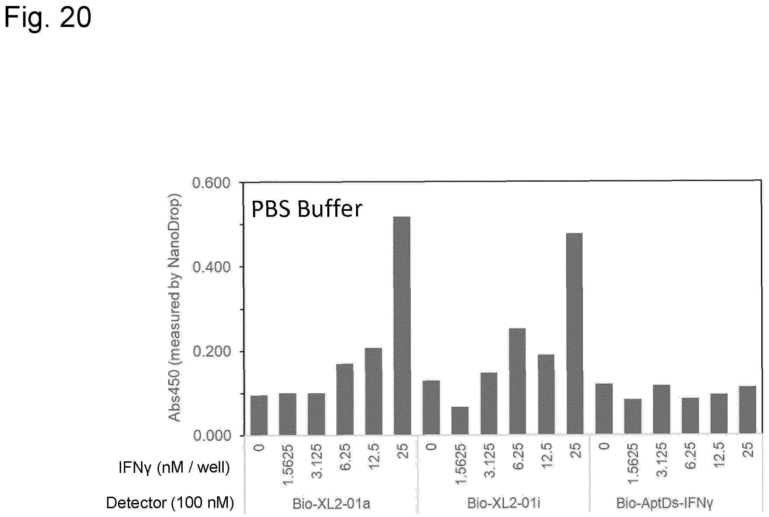

The results are shown in Figure 19 and Figure 20. With Bio-XL2-01a and Bio-XL2-01i, a markedly higher signal intensity was observed, compared with Bio-AptDs-IFNγ obtained using non-glycosylated IFNγ as a target through the 5-base ExSELEX.

### (2) Isolation of Thrombin-targeting nucleic acid aptamer

In accordance with the same method as in (1) above, the 6-base ExSELEX was performed using, as a target protein, Thrombin (Enzyme Research Lab) isolated from human blood plasma, and using the XL1 library and the XL2 library. The concentrated library after the fifth selection was sequenced using the Ion PGM system. A higher-level family larger in the number of reads was analyzed to obtain the aptamers shown in Table 7 below. Any of the sequences comprised four or more G tracts, suggesting the formation of a guanine-quadruplex. In addition, a TTADsTGG sequence and an AAGGADsGGTGGG sequence were found out in the form of a consensus sequence in the sequence obtained from the XL1 and XL2 libraries.

**[Table 7]**

| Table 7: Thrombin-targeting nucleic acid aptamer | | |
|---|---|---|
| Name of nucleic acid aptamer | Base sequence of nucleic acid aptamer (5'-3') | SEQ ID NO: |
| Thr101 | GCTGTCAC GCAGCATGGAACCTTTADsTGGCTAGGGGTGGGDsGGAG GTGACAGC | 75 |
| Thr102 | GCTGTCC CGCCGCGCAGATTADsTGGTTTGADsATAGGGTGGTTA GGACAGC | 173 |
| Thr104 | GCGTTG CGTGAAGTTDsGGGTGGDsATGTGATGGGPa'GGTAGATAGA CAACGC | 87 |
| Thr106 | GCTGATCT CGGAAGGADsGGTGGGDsTCTGGTTGGGPa'GGPa'GCGT AGATCAGC | 89 |
| Thr201 | GCACGAGG CA GGGTTGGGAGGGTAAGGADsGGTGGGTGDs CCTCGTGC | 91 |
| Thr202 | GTCCGTTCACG GCAAACGCACTTTADsTGGTGDsGGGTGGGCTPa'GTCTT CGTGAACGGAC | 174 |
| Thr203 | GTCCTTTCACG TAGCDsGGTCCGGGTTADsTGGGPa'GGGTGGCTTGGGGC CGTGAAAGGAC | 175 |
| Thr204 | GTCACGCGC TGTGGTDsGTTTTDsTTGGGGGGTGGTTAAG GCGCGTGAC | 81 |
| Thr205 | GCACGCTTT DsACTTADsTGGCTGGGTGGPa'CTGGGT AAAGCGTGC | 83 |

| | | |
|---|---|---|
| * The underlines denote sequences complementary to each other. | | |

The affinity of the Thrombin-targeting nucleic acid aptamer shown in Table 7 above to Thrombin was analyzed using EMSA. The nucleic acid aptamer (25 nM) was incubated with human (α) Thrombin (50 nM) in a binding buffer (1 × D-PBS supplemented with 0.005% Nonidet P-40 and 1 mM MgCl₂) at 25°C for 30 minutes. The sample solution was mixed with glycerol (at a final concentration of 5%), and the resulting complex was analyzed with native or denaturing 8% acrylamide gel. The other conditions were the same as in Example 3.

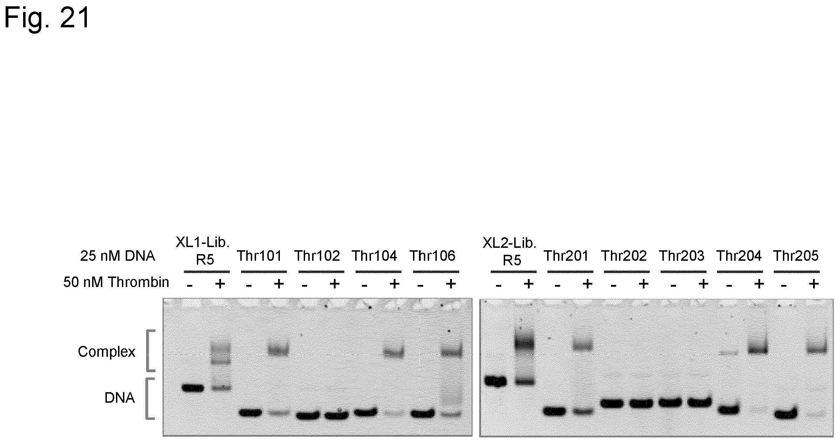

Thr104, Thr204, and Thr205 exhibited high affinity to Thrombin (Figure 21). To the 3' end side of each of these, a biotinylated mini-hairpin sequence was linked to produce aptamers shown in Table 8 below. In the Table below, BioMH-RE31 is derived from the DNA aptamer (RE31) described in the literature (Russo, K.I. et al., Nucleic Acids Res., 2016, 44: 983-991).

**[Table 8]**

| Table 8: Thrombin-targeting nucleic acid aptamer having biotin-bound mini-hairpin linked therein | | |
|---|---|---|
| Name of nucleic acid aptamer | Base sequence of nucleic acid aptamer (5'-3') | SEQ ID NO: |
| Bio-Thr104b | GCGT GCGTGAAGTTDsGGGTGGDsATGTGATGGGPa'GGTAGA TACGCAACGC CGCGLAGCG | 176 |
| Bio-Thr204 | GTCACGCGC TGTGGTDsGTTTTDsTTGGGGGGTGGTTAAG GCGCGTGAC CGCGLAGCG | 177 |
| Bio-Thr205 | GCACGCTTT DsACTTADsTGGCTGGGTGGPa'CTGGGT AAAGCGTGC CGCGLAGCG | 178 |
| BioMH-RE31 | GTGACG TAGGTTGGTGTGGTTGGGG CGTCAC CGCGLAGCG | 179 |

| | | |
|---|---|---|
| * The underlines denote sequences complementary to each other, and capable of forming a stem structure; the black frame denotes a hairpin sequence; and "L" in the sequence represents Biotin-dT. | | |

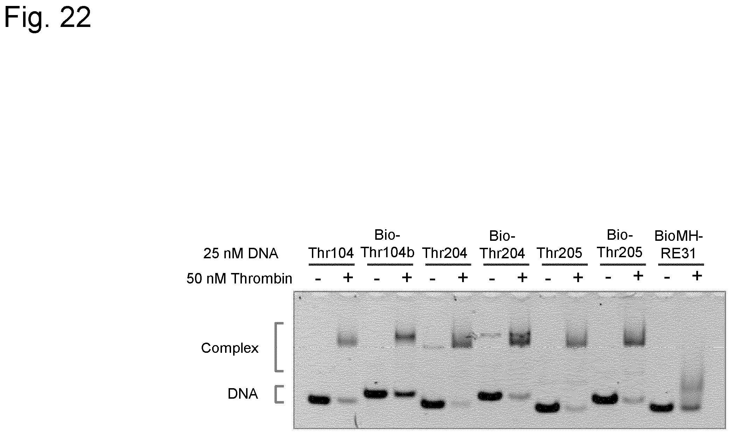

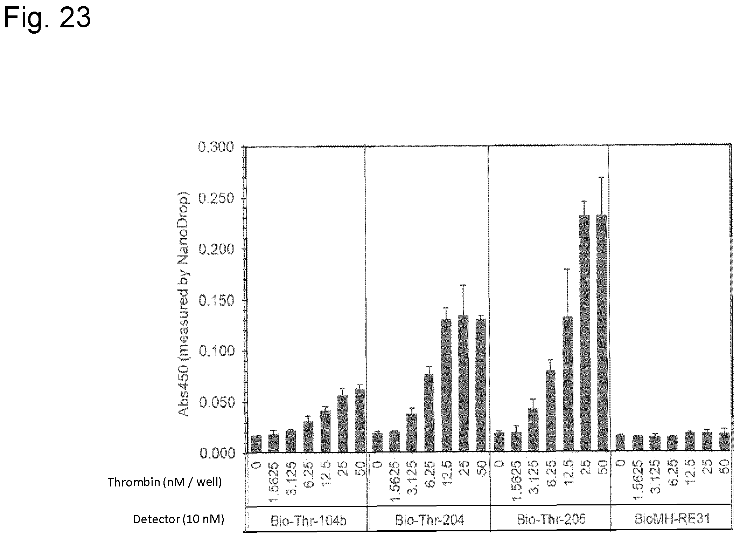

The affinity of the Thrombin-targeting nucleic acid aptamer shown in Table 8 above to Thrombin was analyzed using EMSA, ELONA, and the SPR method. The results are shown in Figure 22 and Figure 23. With EMSA and ELONA, Bio-Thr104, Bio-Thr204, and Bio-Thr205 exhibited markedly higher affinity, compared with BioMH-RE31. The K_{D} value measured using the SPR method was 9.0 pM for Bio-Thr204 and 7.3 pM for Bio-Thr205.

### (3) Isolation of HMGB1-targeting nucleic acid aptamer

Using the same method as in (1) above, the 6-base ExSELEX was performed using HMGB1 protein as a target protein, and as a result, the below-described Bio-HMGB1-301-DD aptamer (SEQ ID NO: 143) was isolated.

### <Example 5: Thrombin-targeting nucleic acid aptamer>

### (Purpose)

The purpose was to produce a nucleic acid aptamer targeted at Thrombin, and evaluate the affinity to human Thrombin.

### (Method and results)

### (1) Synthesis of nucleic acid aptamer

Using the same method as in Example 2, the nucleic acid aptamers shown in Table 9 below were synthesized. The nucleic acid aptamers shown in Table 9 are: nucleic acid aptamers that bind to Thrombin as a target, and comprise two Ds's (in Table below, the aptamers are represented by "Thr204-DD"); and the same nucleic acid aptamers as the Thr204-DD aptamers except that at least one of the Ds's is substituted with a Ds alternative or an adenine base. The Thr204-DD aptamer is the same as Thr204 produced in Example 4.

**[Table 9]**

| Table 9: Thrombin-targeting nucleic acid aptamer | | |
|---|---|---|
| Name of nucleic acid aptamer | Base sequence of nucleic acid aptamer (5'-3') | SEQ ID NO: |
| Thr204-DD | GTCACGCGC TGTGGTDsGTTTTDsTTGGGGGGTGGTTAAG GCGCGTGAC | 81 |
| Thr204-BB | GTCACGCGC TGTGGTBsGTTTTBsTTGGGGGGTGGTTAAG GCGCGTGAC | 81 |
| Thr204-BY | GTCACGCGC TGTGGTBsGTTTTYsTTGGGGGGTGGTTAAG GCGCGTGAC | 81 |
| Thr204-YB | GTCACGCGC TGTGGTYsGTTTTBsTTGGGGGGTGGTTAAG GCGCGTGAC | 81 |
| Thr204-YY | GTCACGCGC TGTGGTYsGTTTTYsTTGGGGGGTGGTTAAG GCGCGTGAC | 81 |
| Thr204-AA | GTCACGCGC TGTGGTA GTTTTA TTGGGGGGTGGTTAAG GCGCGTGAC | 81 |
| Bio-Thr204-DD | GTCACGCGC TGTGGTDsGTTTTDsTTGGGGGGTGGTTAAG GCGCGTGAC CGCGLAGCG | 142 |
| Bio-Thr204-BB | GTCACGCGC TGTGGTBsGTTTTBsTTGGGGGGTGGTTAAG GCGCGTGAC CGCGLAGCG | 142 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers shown in Table 9 above to human Thrombin was analyzed using EMSA. The nucleic acid aptamer was incubated with human Thrombin in a binding buffer (1 × D-PBS supplemented with 0.005% Nonidet P-40 and 1 mM MgCl₂) at 25°C for 30 minutes. The sample solution was mixed with glycerol (at a final concentration of 5%). The formation of a complex was analyzed using native 8% polyacrylamide gel electrophoresis (PAGE) at 25°C or 37°C, or 8% PAGE in the presence of 1 M urea at 37°C. The other conditions were the same as in Example 3.

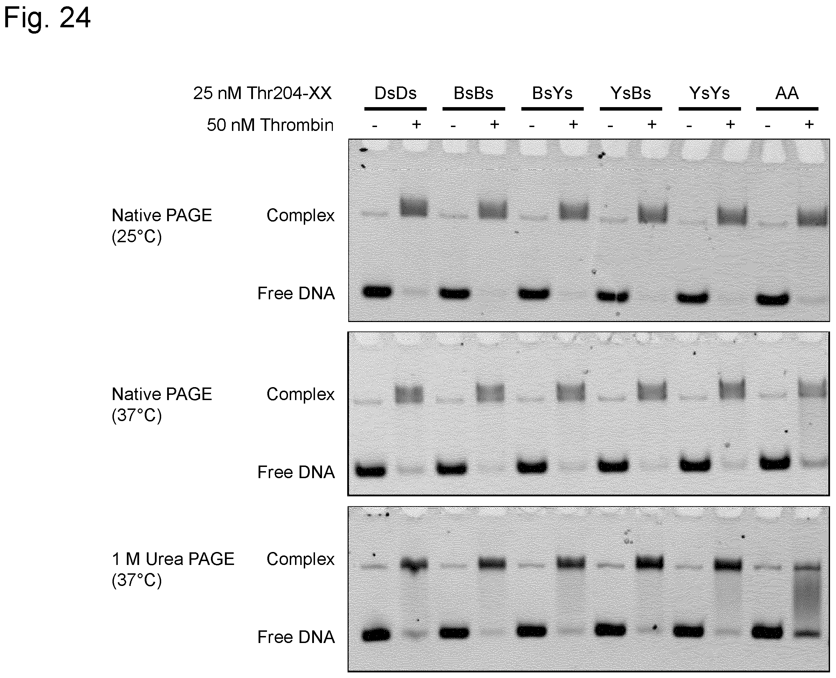

The results are shown in Figure 24. Any of the same nucleic acid aptamers as the Thr204-DD aptamer except that Ds was substituted with a Ds alternative exhibited a binding activity the same as or higher than the Thr204-DD aptamers produced in Example 4. On the other hand, Thr204-AA having natural adenine bases substituted for the Ds bases was markedly decreased in the binding activity.

Next, the K_{D} value was determined using the SPR method. The SPR method was performed at a flow rate of 100 µL/minute at 25°C, using a running buffer (20 mM Tris HCl, a pH of 7.5, 150 mM NaCl, 1 mM MgCl₂, 2.7 mM KCl, and 0.05% Tween 20), in which method, 50 mM NaOH was injected for 5 seconds, and the running buffer was used for equilibration to thereby perform regeneration. The K_{D} values were 9.1 pM for Bio-Thr204-DD and 7.95 pM for Bio-Thr204-BB. With Bio-Thr204-BB aptamer, the affinity was increased, compared with the Ds aptamer.

### <Example 6: HMGB1-targeting nucleic acid aptamer>

### (Purpose)

The purpose was to produce a nucleic acid aptamer targeted at HMGB1 (High Mobility Group Box 1) protein, and evaluate the affinity to human HMGB1 protein.

### (Method and results)

### (1) Synthesis of nucleic acid aptamer

Using the same method as in Example 2, the nucleic acid aptamers shown in Table 10 below were synthesized. The nucleic acid aptamers shown in Table 10 are: nucleic acid aptamers that bind to HMGB1 protein as a target, and comprise two Ds's (in Table below, the aptamers are represented by "Bio-HMGB1-301-DD"); and the same nucleic acid aptamers as the Bio-HMGB1-301-DD aptamers except that at least one of the Ds's is substituted with a Ds alternative or an adenine base. The Bio-HMGB1-301-DD aptamer is a nucleic acid aptamer isolated in accordance with (3) in Example 4 above.

**[Table 10]**

| Table 10: HMGB1-targeting nucleic acid aptamer | | |
|---|---|---|
| Name of nucleic acid aptamer | Base sequence of nucleic acid aptamer (5'-3') | SEQ ID NO: |
| Bio-HMGB1-301-DD | CTCCTTCAGC GCCACCGTATGDsACGCTCACAGDsAGTTGCCGA GCTGAAGGAG CGCGLAGCG | 143 |
| Bio-HMGB1-301-BB | CTCCTTCAGC GCCACCGTATGBsACGCTCACAGBsAGTTGCCGA GCTGAAGGAG CGCGLAGCG | 143 |
| Bio-HMGB1-301-BY | CTCCTTCAGC GCCACCGTATGBsACGCTCACAGYsAGTTGCCGA GCTGAAGGAG CGCGLAGCG | 143 |
| Bio-HMGB1-301-YB | CTCCTTCAGC GCCACCGTATGYsACGCTCACAGBsAGTTGCCGA GCTGAAGGAG CGCGLAGCG | 143 |
| Bio-HMGB1-301-YY | CTCCTTCAGC GCCACCGTATGYsACGCTCACAGYsAGTTGCCGA GCTGAAGGAG CGCGLAGCG | 143 |
| Bio-HMGB1-301-AA | CTCCTTCAGC GCCACCGTATGA ACGCTCACAGA AGTTGCCGA GCTGAAGGAG CGCGLAGCG | 144 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers shown in Table 10 above to human HMGB1 protein was analyzed using EMSA. The nucleic acid aptamer was incubated with human HMGB1 in a binding buffer (1 × D-PBS supplemented with 0.005% Nonidet P-40 and 1 mM MgCl₂) at 25°C for 30 minutes. The sample solution was mixed with glycerol (at a final concentration of 5%). The formation of a complex was analyzed using native 6% polyacrylamide gel electrophoresis (PAGE) at 25°C. The other conditions were the same as in Example 3.

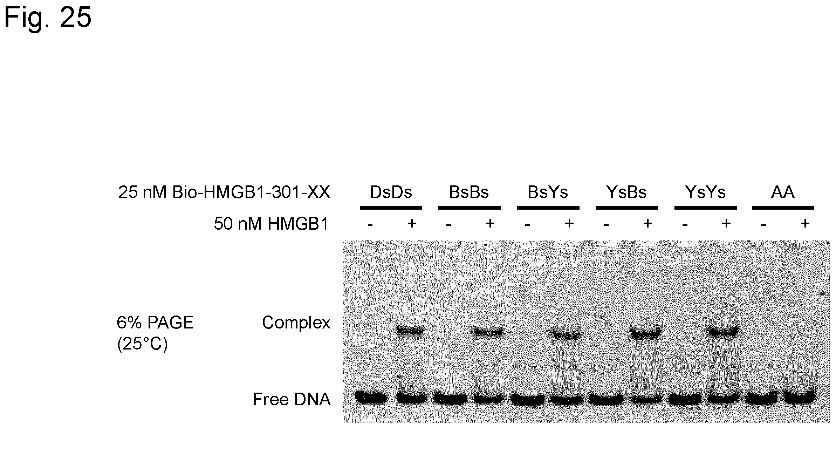

The results are shown in Figure 25. Any of the same nucleic acid aptamers as the Bio-HMGB1-301-DD aptamer except that Ds was substituted with a Ds alternative exhibited a binding activity the same as or higher than the Bio-HMGB1-301-DD aptamer. On the other hand, Bio-HMGB1-301-AA having natural adenine bases substituted for the Ds bases was markedly decreased in the binding activity.

Next, the K_{D} value was determined using the SPR method in the same manner as in Example 5. The K_{D} values were 23.4 pM for Bio-HMGB1-301-DD and 1.4 pM for Bio-HMGB1-301-YB. With the HMGB1-301-YB aptamer, the affinity was increased, compared with the Ds aptamer.

### <Example 7: IFNy-targeting nucleic acid aptamer>

### (Purpose)

The purpose was to further produce a nucleic acid aptamer targeted at IFNγ, and evaluate the affinity to IFNγ.

### (Method and results)

### (1) Synthesis of nucleic acid aptamer

Using the same method as in Example 2, the nucleic acid aptamers shown in Table 11 below were synthesized. The nucleic acid aptamers shown in Table 11 are: nucleic acid aptamers comprising Ds's; and the same nucleic acid aptamers as these aptamers except that at least one of the Ds's is substituted with a Ds alternative or an adenine base. IFNg-201ADD is the same as XL2-01a produced in Example 4.

**[Table 11]**

| Table 11: IFNy-targeting nucleic acid aptamer | | |
|---|---|---|
| Name of nucleic acid aptamer | Base sequence of nucleic acid aptamer (5'-3') | SEQ ID NO: |
| IFNg-201AAD | GTCCTCACG CCGTCATAATTGTTA TTTTGATCCTTGDsAGTGGGGA CGTGAGGAC | 145 |
| IFNg-201AAY | GTCCTCACG CCGTCATAATTGTTA TTTTGATCCTTGYsAGTGGGA CGTGAGGAC | 145 |
| IFNg-201AAB | GTCCTCACG CCGTCATAATTGTTA TTTTGATCCTTGBsAGTGGGGA CGTGAGGAC | 145 |
| IFNg-201ADD | GTCCTCACG CCGTCATAATTGTTDsTTTTGATCCTTGDsAGTGGGGA CGTGAGGAC | 62 |
| IFNg-201AYD | GTCCTCACG CCGTCATAATTGTTYsTTTTGATCCTTGDsAGTGGGGA CGTGAGGAC | 62 |
| IFNg-201ABD | GTCCTCACG CCGTCATAATTGTTBsTTTTGATCCTTGDsAGTGGGGA CGTGAGGAC | 62 |
| B-I-Apt1-DD | CCCGCC CGGGTCCGCGAAGCGGTAGGTDsTGGGCTAGGCDsGCT GGCGGG CGCGLAGCG | 146 |
| B-I-Apt1-YY | CCCGCC CGGGTCCGCGAAGCGGTAGGTYsTGGGCTAGGCYsGCT GGCGGG CGCGLAGCG | 146 |
| B-IFNg-201AAD | GTCCTCACG CCGTCATAATTGTTA TTTTGATCCTTGDsAGTGGGGA CGTGAGGAC CGCGLAGCG | 147 |
| B-IFNg-201AAB | GTCCTCACG CCGTCATAATTGTTA TTTTGATCCTTGBsAGTGGGGA CGTGAGGAC CGCGLAGCG | 147 |
| B-IFNg-201AYD | GTCCTCACG CCGTCATAATTGTTYsTTTTGATCCTTGDsAGTGGGGA CGTGAGGAC CGCGLAGCG | 148 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers from IFNg-201AAD to B-I-Apt1-YY in Table 11 above to human IFNγ was analyzed using EMSA. With non-glycosylated human IFNγ expressed in *Escherichia coli* or glycosylated human IFNγ expressed in a HEK293 cell, the nucleic acid aptamer was incubated in a binding buffer (1 × D-PBS supplemented with 0.05% Nonidet P-40 and 1 mM MgCl₂) at 25°C for 30 minutes. The complexes incubated were each analyzed at 25°C with native 8% PAGE or denaturing 8% PAGE comprising 3 M urea. The other conditions were the same as in Example 3.

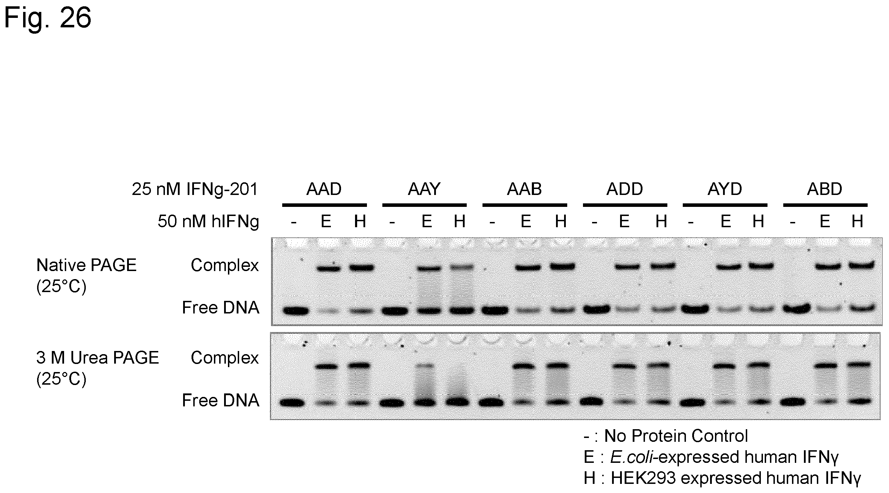

The results are shown in Figure 26. IFNg-201AAD, IFNg-201AAB, IFNg-201ADD, IFNg-201AYD, and IFNg-201ABD, except IFNg-201AAY, exhibited the same binding activity.

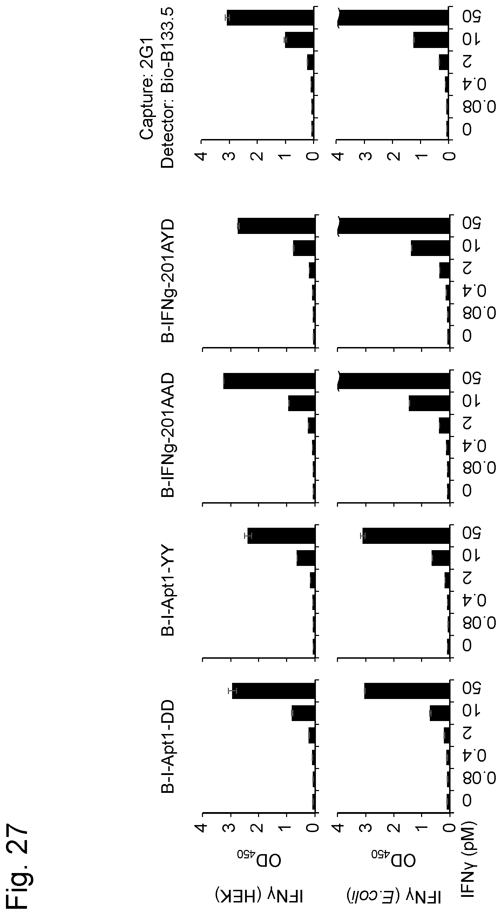

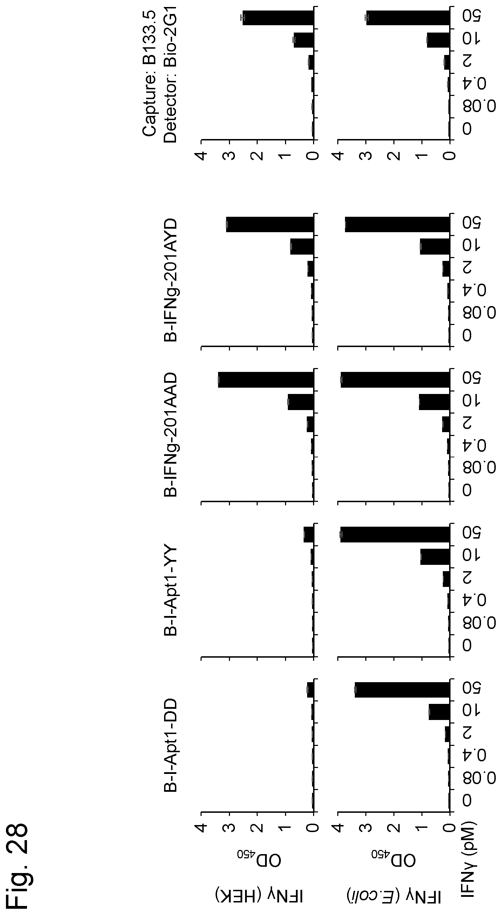

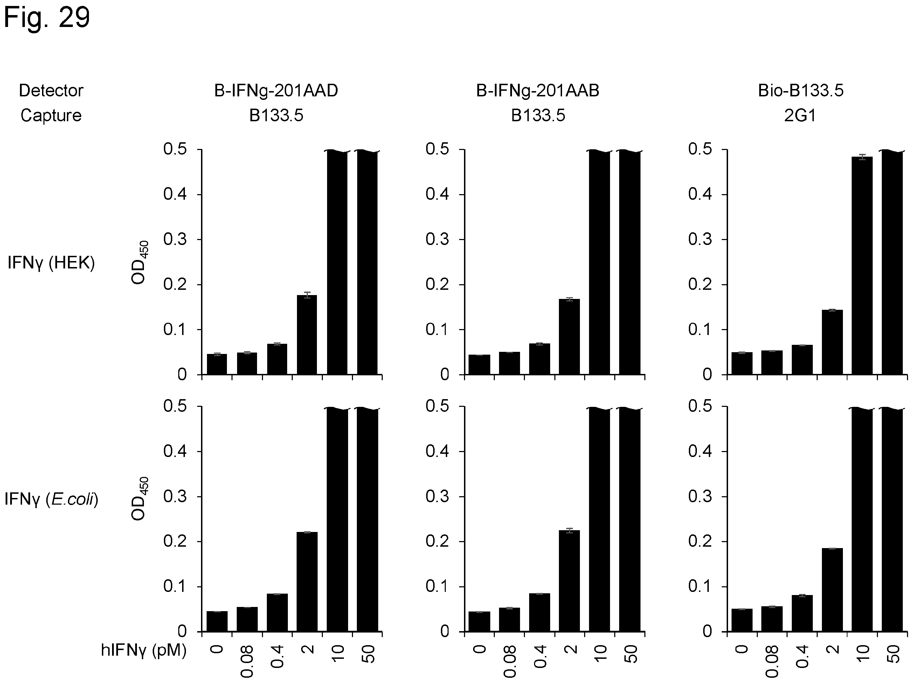

Next, the detection sensitivity of each of the B-I-Apt1-DD, B-I-Apt1-YY, B-IFNg-201AAD, B-IFNg-201AAB, and B-IFNg-201AYD nucleic acid aptamers to IFNγ was studied using ELISA. For ELISA, commercially available monoclonal antibodies-a 2G1 antibody and a B133.5 antibody (Thermo Fisher Scientific Inc.)-were used. The results obtained by using a nucleic acid aptamer for capturing IFNγ, using a B133.5 antibody as a detecting antibody, and using an anti-mouse IgG Ab-HRP as a secondary antibody having HRP bound therein are shown in Figure 27 (the results obtained by using controls are shown in the right column in Figure 27, in which the controls are a 2G1 antibody as a capture antibody and a Bio-B133.5 antibody as a detecting antibody). The results obtained by detecting streptavidin-bound HRP, using a B133.5 antibody as an antibody for capturing IFNγ and using a nucleic acid aptamer for detection are shown in Figure 28 (the results obtained by using control are shown in the right column in Figure 28, in which the controls are a B133.5 antibody as a capture antibody and a Bio-2G1 antibody as a detecting antibody). Furthermore, the results obtained by using a B133.5 antibody as an antibody for capturing IFNγ and using B-IFNg-201AAD or B-IFNg-201AAB for detection are shown in Figure 29 (the results obtained by using control are shown in the right column in Figure 29, in which the controls are a 2G1 antibody as a capture antibody and a Bio-B133.5 antibody as a detecting antibody). B-I-Apt1-DD and B-I-Apt1-YY exhibited low detection sensitivity to glycosylated human IFNγ expressed in a HEK293 cell, but B-IFNg-201AAD, B-IFNg-201AAB, and B-IFNg-201AYD exhibited high detection sensitivity to both of non-glycosylated human IFNγ expressed in *Escherichia coli* and glycosylated human IFNγ expressed in a HEK293 cell.

### <Example 8: DEN1-NS1-targeting nucleic acid aptamer>

### (Purpose)

The purpose was to further produce a nucleic acid aptamer targeted at DEN1-NS1 protein, and evaluate the affinity to DEN1-NS1 protein.

### (Method and results)

### (1) Synthesis of nucleic acid aptamer

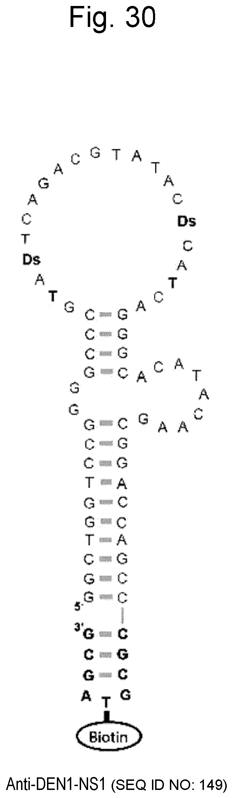

Using the same method as in Example 2, the nucleic acid aptamers shown in Table 12 below were synthesized. The nucleic acid aptamers shown in Table 12 are: a nucleic acid aptamer (shown in Figure 30, and represented by "AptD1c (DsDs)" in Table below) that comprises two Ds's, and binds to DEN1-NS1 protein as a target; AptD2c (DsDs) that is the same nucleic acid aptamer as AptD1c (DsDs) except that the bulge loop portion is deleted to make the stem region shorter; and the same nucleic acid aptamers as the AptD2c (DsDs) aptamers except that at least one of the Ds's is substituted with a Ds alternative, a combination of multiple Ds alternatives, or a natural adenine base. The AptD1c aptamer is based on a sequence isolated using ExSELEX in the past literature (Matsunaga, K., et al., Nucleic Acids Res., 2021, 49: 11407-11424.).

**[Table 12]**

| Table 12: DEN1-NS1-targeting nucleic acid aptamer | | |
|---|---|---|
| Name of nucleic acid aptamer | Base sequence of nucleic acid aptamer (5'-3') | SEQ ID NO: |
| AptD1c(DsDs) | GGCTGG TCCGGGCCCGTADsTCAGACGTATACDsCATCAGGGCACATA CAAGCGGACCAGCCCGCGLAGCG | 149 |
| AptD1c2(DsDs) | GGTCCGGCCC GTADsTCAGACGTATACDsCATCA GGGCCGGACC CGCGLAGCG | 150 |
| AptD1 c2(AA) | GGTCCGGCCC GTAA TCAGACGTATACA CATCA GGGCCGGACC CGCGLAGCG | 151 |
| AptD1c2a(YsYs) | GGTCCGGCCC GTAYsTCAGACGTATACYsCATCA GGGCCGGACC CGCGLAGCG | 150 |
| AptD1c2b(YsBs) | GGTCCGGCCC GTAYsTCAGACGTATACBsCATCA GGGCCGGACC CGCGLAGCG | 150 |
| AptD1c2c(BsYs) | GGTCCGGCCC GTABsTCAGACGTATACYsCATCA GGGCCGGACC CGCGLAGCG | 150 |
| AptD1c2d(BsBs) | GGTCCGGCCC GTABsTCAGACGTATACBsCATCA GGGCCGGACC CGCGLAGCG | 150 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers shown in Table 12 above to DEN1-NS1 protein was analyzed using EMSA. The nucleic acid aptamer was incubated, in the binding buffer described in the above-described literature, with DENV1 NS1 variant 1 purchased from Native Antigen Company or DENV1 NS1 variant 2 expressed in a CHO cell. A sample solution was separated using native 4% acrylamide gel. The other conditions were the same as in Example 3.

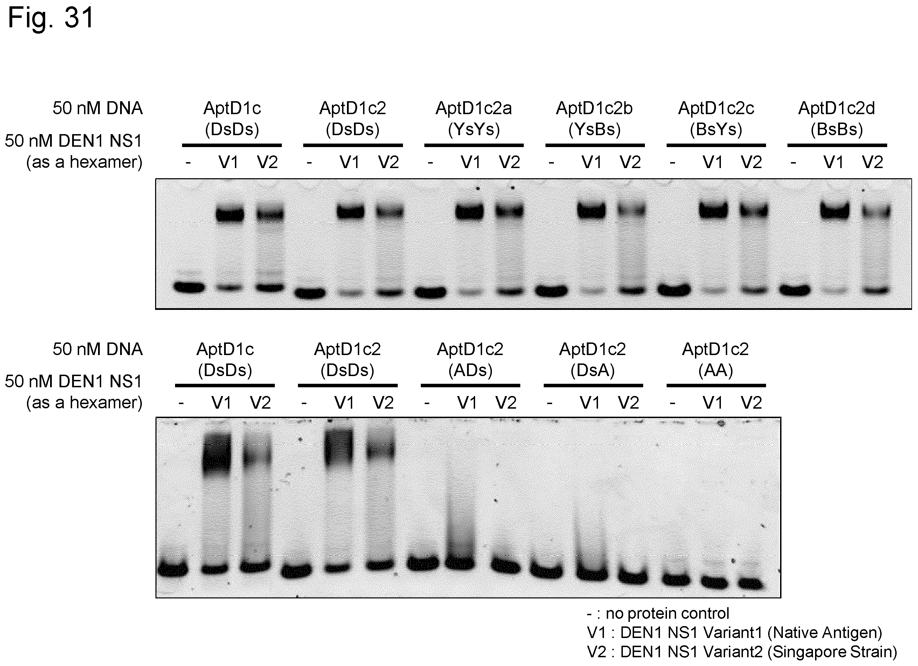

The results are shown in Figure 31. Any of the nucleic acid aptamers having a Ds alternative substituted for Ds exhibited a binding activity the same as or higher than AptD1c1 or AptD1c2. On the other hand, AptD1c2(AA) having a natural adenine base substituted for the Ds base was markedly decreased in the binding activity.

Next, the K_{D} value was determined using the SPR method. The K_{D} values were 143 pM for Bio-AptD1C2(DD), 102 pM for Bio-AptD1C2a(YsYs), and 136 pM for Bio-AptD1C2c(BsYs).

### <Example 9: DEN1/2-NS1-targeting nucleic acid aptamer>

### (Purpose)

The purpose was to produce a DEN1/2-NS1-targeting nucleic acid aptamer targeted at DENV1 NS1 (DEN1-NS1) protein and/or DENV2 NS1 (DEN2-NS1) protein, and evaluate the affinity to the target proteins.

### (Method and results)

### (1) Synthesis of nucleic acid aptamer

Using the same method as in Example 2, the nucleic acid aptamers shown in Table 13 below were synthesized. The nucleic acid aptamers shown in Table 13 are: nucleic acid aptamers that bind to DENV1 NS1 (DEN1-NS1) protein and/or DENV2 NS1 (DEN2-NS1) protein as targets; and the same nucleic acid aptamers as these aptamers except that at least one of the Ds's is substituted with a Ds alternative. Each of the AptD2-b1, AptD2-b2, AptD2-b3, and AptD2-b4 aptamers is based on a sequence isolated using ExSELEX in the past literature (Matsunaga, K., et al., Nucleic Acids Res., 2021, 49: 11407-11424.). In this regard, in the nucleic acid aptamers shown in Table 13 below, the consensus sequence motifs CCAACC and CCAATC are sequences frequently found in a library concentrated using the ExSELEX, and targeted at DENV1 NS1 and DENV2 NS1 in the literature above. It is suggested that such a sequence interacts with DENV NS1 protein in a broad specificity.

**[Table 13]**

| Table 13: DEN1/2-NS1-targeting nucleic acid aptamer | | |
|---|---|---|
| Name of nucleic acid aptamer | Base sequence of nucleic acid aptamer (5'-3') | SEQ ID NO: |
| AptD2-b1 | GGCTGGTCCG TACDsCCCAACCTTTCCAATCCAGCDsTTAGC CGGACCAGCC CGCGLAGCG | 152 |
| AptD2-b2 | GGCTGGTCCG CCAATCDsAGCDsTAGTCTAAAAGACCDsC CCAACCC CGGACCAGCC CGCGLAGCG | 153 |
| AptD2-b3 | GGCTGGTCCG TCCAGCADsCCAATCTCCACCAACCDsCCTGC CADsG CGGACCAGCC CGCGLAGCG | 154 |
| AptD2-b4 | GGCTGGTCCG CGDsCCAACCTCCACCAATCAAAGC CGGACCAGCC CGCGLAGCG | 155 |
| AptD2bb(Ys) | GGCTGGTCCG CGYsCCAACCTCCACCAATCAAAGC CGGACCAGCC CGCGLAGCG | 155 |
| AptD2bb(Bs) | GGCTGGTCCG CGBsCCAACCTCCACCAATCAAAGC CGGACCAGCC CGCGLAGCG | 155 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers shown in Table 13 above to DENV NS1 protein and ZIKA NS1 protein was analyzed using EMSA. In a binding buffer described in the literature above, the nucleic acid aptamer was incubated with DENV1 NS1 variant 1 purchased from The Native Antigen Company, DENV1 NS1 variant 2 expressed in a CHO cell, DENV2 NS1 variant 1 purchased from The Native Antigen Company, or DENV2 NS1 variant 2 (with 178F, not 178S) expressed in a CHO cell. The resulting sample solution incubated was separated with native 4% acrylamide gel. The other conditions were the same as in Example 3.

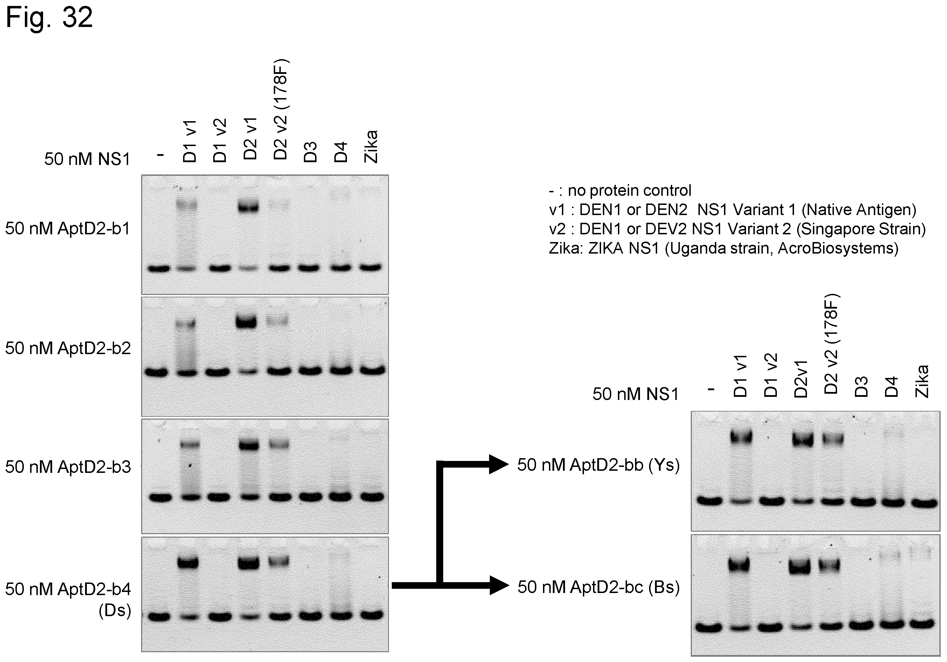

The results are shown in Figure 32. AptD2-b4 exhibited the strongest binding to DEN1-NS1 v1 and DEN2-NS1 v1 and v2. All of the nucleic acid aptamers having a Ds alternative substituted for this exhibited the same or higher binding activity.

### <Example 10: DEN4-NS1-targeting nucleic acid aptamer>

### (Purpose)

The purpose was to further produce a nucleic acid aptamer targeted at DENV4 NS1 (DEN4-NS1) protein, and evaluate the affinity to DEN4-NS1 protein.

### (Method and results)

### (1) Synthesis of nucleic acid aptamer

Using the same method as in Example 2, the nucleic acid aptamers shown in Table 14 below were synthesized. The nucleic acid aptamers shown in Table 14 are: nucleic acid aptamers that bind to DEN4-NS1 protein as a target; and the same nucleic acid aptamers as these aptamers except that at least one of the Ds's is substituted with a Ds alternative or a combination of multiple Ds alternatives. The AptD4-1 aptamer is based on a sequence isolated using ExSELEX in the past literature (Matsunaga, K., et al., Nucleic Acids Res., 2021, 49: 11407-11424.).

**[Table 14]**

| Table 14: DEN4-NS1-targeting nucleic acid aptamer | | |
|---|---|---|
| Name of nucleic acid aptamer | Base sequence of nucleic acid aptamer (5'-3') | SEQ ID NO: |
| AptD4-1(DsDs) | CGGCGGA GACGTAACGCDsTATCAAATCDsAAACAGCTTA GGG TCCGCCG CGCGLAGCG | 156 |
| AptD4-2(YsYs) | CGGCGGA GACGTAACGCYsTATCAAATCYsAAACAGCTTA GGG TCCGCCG CGCGLAGCG | 156 |
| AptD4-3(YsBs) | CGGCGGA GACGTAACGCYsTATCAAATCBsAAACAGCTTA GGG TCCGCCG CGCGLAGCG | 156 |
| AptD4-4(BsYs) | CGGCGGA GACGTAACGCBsTATCAATCYsAAACAGCTTA GGG TCCGCCG CGCGLAGCG | 156 |
| AptD4-5(BsBs) | CGGCGGA GACGTAACGCBsTATCAAATCBsAAACAGCTTA GGG TCCGCCG CGCGLAGCG | 156 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs" and "Ys" represent a Ds alternative. | | |

### (2) Binding assay

The affinity of the nucleic acid aptamers shown in Table 14 above to DEN4-NS1 protein was analyzed using EMSA. In the binding buffer described in the above-described literature, the nucleic acid aptamer was incubated with DENV4 NS1 protein purchased from Native Antigen Company. The resulting sample solution incubated was separated with native 4% acrylamide gel. The other conditions were the same as in Example 3.

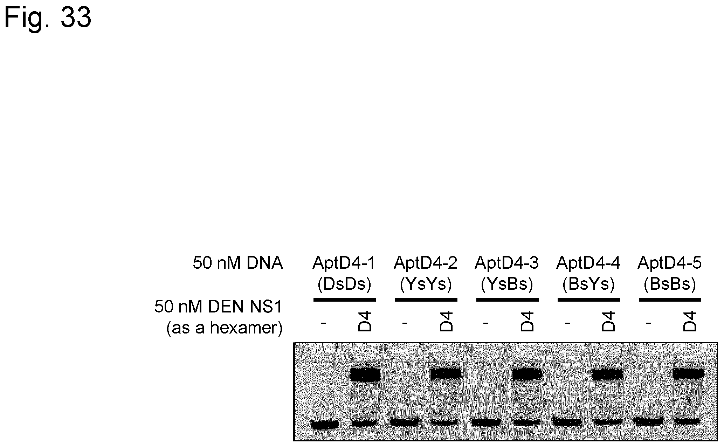

The results are shown in Figure 33. All of the same nucleic acid aptamers as AptD4-1(DsDs) except that Ds was substituted with a Ds alternative exhibited a binding activity the same as or higher than AptD4-1(DsDs).

### <Example 11: VEGF₁₆₅-targeting nucleic acid aptamer>

### (Purpose)

The purpose was to further produce a nucleic acid aptamer targeted at VEGF165 protein, and evaluate the affinity to human VEGF₁₆₅ protein.

### (Method and results)

### (1) Synthesis of nucleic acid aptamer

Using the same method as in Example 2, the nucleic acid aptamers shown in Table 15 below were synthesized. The nucleic acid aptamers shown in Table 15 are: nucleic acid aptamers ("V-Apt1(DD)" in Table below) that bind to VEGF₁₆₅ protein as a target; and the same nucleic acid aptamers as these aptamers except that at least one of the Ds's is substituted with a Ds alternative, a combination of multiple Ds alternatives, or a natural adenine base. The V-Apt1(DD) aptamer is based on a sequence isolated using ExSELEX in the past literature (Kimoto et al., Nature Biotechnology. 2013, 31(5): 453-7.).

**[Table 15]**

| Table 15: VEGF₁₆₅-targeting nucleic acid aptamer | | |
|---|---|---|
| Name of nucleic acid aptamer | Base sequence of nucleic acid aptamer (5'-3') | SEQ ID NO: |
| V-Apt1 (DsDs) | GCGG TAAGCCGCGTCCGAAGGGGCDsTGCGGCGADsCCCGAA TGGGT CCGC CGCGLAGCG | 157 |
| V-Apt4(AA) | GCGG TAAGCCGCGTCCGAAGGGGCA TGCGGCGAA CCCGAA TGGGT CCGC CGCGLAGCG | 158 |
| b58(YsYs) | GCGG TAAGCCGCGTCCGAAGGGGCYsTGCGGCGAYsCCCGAA TGGGT CCGC CGCGLAGCG | 157 |
| c58(YsBs) | GCGG TAAGCCGCGTCCGAAGGGGCYsTGCGGCGABsCCCGAA TGGGT CCGC CGCGLAGCG | 157 |
| d58(BsYs) | GCGG TAAGCCGCGTCCGAAGGGCBsTGCGGCGAYsCCCGAA TGGGT CCGC CGCGLAGCG | 157 |
| e58(BsBs) | GCGG TAAGCCGCGTCCGAAGGGCBsTGCGGCGABsCCCGAA TGGGT CCGC CGCGLAGCG | 157 |
| f58(YoYo) | GCGG TAAGCCGCGTCCGAAGGGGCYoTGCGGCGAYoCCCGAA TGGGT CCGC CGCGLAGCG | 157 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs", "Ys", and "Yo" represent a Ds alternative. | | |

### (2) Binding assay

The affinity of the nucleic acid aptamers shown in Table 15 above to human VEGF₁₆₅ was analyzed using EMSA. In a binding buffer (1 × D-PBS supplemented with 0.005% Nonidet P-40), the nucleic acid aptamer was incubated, at 25°C for 30 minutes, with human VEGF₁₆₅ manufactured by Peprotech. The resulting sample solution incubated was mixed with glycerol (at a final concentration of 5%), and the resulting solution was analyzed with native 8% PAGE at 25°C. The other conditions were the same as in Example 3.

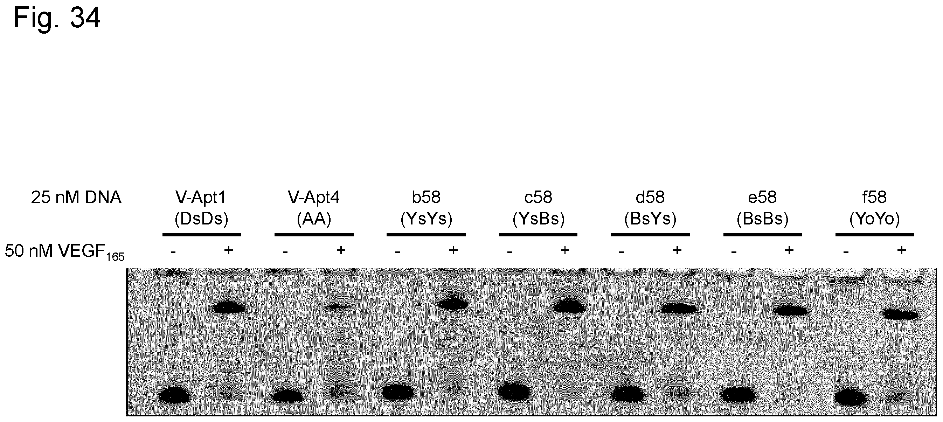

The results are shown in Figure 34. All of the nucleic acid aptamers having a Ds alternative substituted for Ds exhibited a binding activity the same as or higher than V-Apt1(DsDs). On the other hand, V-Apt4(AA) having a natural adenine base substituted for the Ds base was markedly decreased in the binding activity.

### <Example 12: TrfR1-targeting nucleic acid aptamer>

### (Purpose)

The purpose was to produce a nucleic acid aptamer targeted at Transferrin receptor 1 (TrfR1), and evaluate the affinity to human TrfR1.

### (Method and results)

### (1) Synthesis of nucleic acid aptamer

Using the same method as in Example 2, the nucleic acid aptamers shown in Table 16 below were synthesized. The nucleic acid aptamers shown in Table 16 are: nucleic acid aptamers that bind to TrfR1 as a target, (in Table below, the aptamers are represented by "MB45-Ds"); and the same nucleic acid aptamers as these aptamers except that Ds is substituted with a Ds alternative or a natural adenine base. The MB45-Ds aptamer is based on a sequence isolated using ExSELEX in the past literature (Futami et al., Molecular Therapy Nucleic Acids. 2019 Mar 1: 14:158-170.).

**[Table 16]**

| Table 16: TrfR1-targeting nucleic acid aptamer | | |
|---|---|---|
| Name of nucleic acid aptamer | Base sequence of nucleic acid aptamer (5'-3') | SEQ ID NO: |
| MB45-Ds | GGCGCCA GGGGTGTTTGTGCCGTGAGDsTGTGGCGCC CGCGLAGCG | 159 |
| MB45-Ys | GGCGCCA GGGGTGTTTGTGCCGTGAGYsTGTGGCGCC CGCGLAGCG | 159 |
| MB45-Yo | GGCGCCA GGGGTGTTTGTGCCGTGAGYoTGTGGCGCC CGCGLAGCG | 159 |
| MB45-Bs | GGCGCCA GGGGTGTTTGTGCCGTGAGBsTGTGGCGCC CGCGLAGCG | 159 |
| MB45-Bo | GGCGCCA GGGGTGTTTGTGCCGTGAGBoTGTGGCGCC CGCGLAGCG | 159 |
| MB45-A | GGCGCCA GGGGTGTTTGTGCCGTGAGA TGTGGCGCC CGCGLAGCG | 160 |

| | | |
|---|---|---|
| * The underlines denote sequences complementary to each other, and capable of forming a stem structure; the black frame denotes a hairpin sequence; "L" in the sequence represents Biotin-dT; "Ds" represents a Ds base; and "Bs", "Bo", "Ys", and "Yo" represent a Ds alternative. | | |

### (2) Binding assay

The affinity of the nucleic acid aptamers shown in Table 16 above to human TrfR1 was analyzed using EMSA. In a binding buffer (20 mM Tris-HCl, a pH of 7.5, 150 mM NaCl, 2.7 mM KCl, 1 mM MgCl2, and 0.05% Tween 20), the nucleic acid aptamer was incubated, at 25°C for 30 minutes, with human TrfR1 manufactured by AcroBiosystems. The resulting sample solution incubated was mixed with glycerol (at a final concentration of 5%), and the resulting solution was analyzed with native 6% PAGE at 25°C. The other conditions were the same as in Example 3.

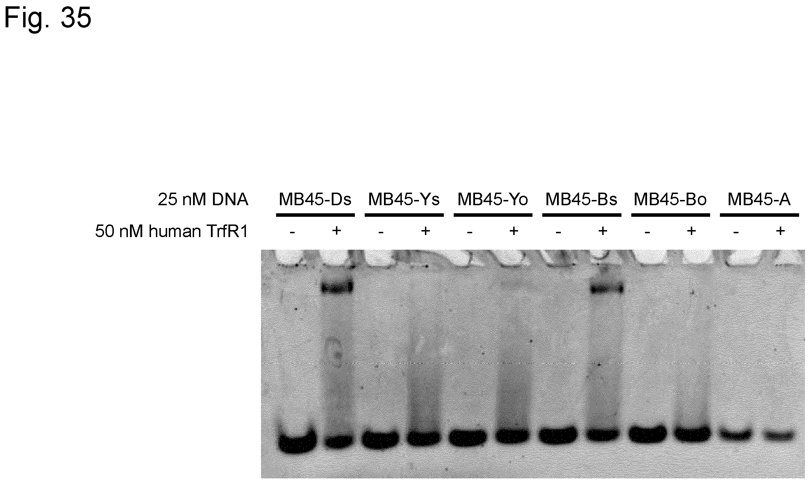

The results are shown in Figure 35. Only MB45-Bs exhibited a binding activity the same as or higher than MB45-Ds. On the other hand, the other nucleic acid aptamers exhibited no band shift. MB45-A having a natural adenine base substituted for the Ds base was markedly decreased in the binding activity.

Next, the K_{D} value was determined using the SPR method. The SPR method was performed at a flow rate of 30 µL/minute at 25°C, using a running buffer (1 × D-PBS supplemented with 0.05% Nonidet P-40 and 1 mM MgCl₂), in which method, 50 mM NaOH was injected for 5 seconds, and the running buffer was used for equilibration to thereby perform regeneration. The K_{D} values for human TrfR1 expressed in a HEK293 cell were 42 pM for MB45-Ds and 51 pM for MB45-Bs. The K_{D} values for Mouse TrfR1 expressed in a HEK293 cell were 1 nM for MB45-Ds and 363 pM for MB45-Bs. MB45-Bs exhibited increased affinity to TrfR1 than MB45-Ds.

All publications, patents, and patent applications cited herein are incorporated herein by reference in their entirety.

## Claims

1. A nucleic acid aptamer comprising unnatural base(s) represented by the following general formula(s) (I) and/or (II), 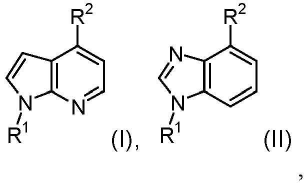 wherein R¹ represents a sugar portion in a nucleoside, and R² represents any one formula represented by the following formulas (III) to (IX),

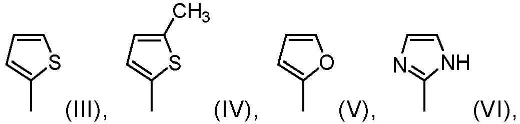

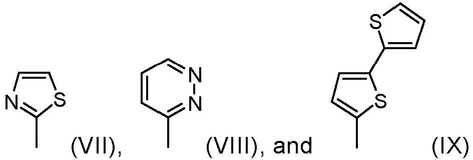

2. The nucleic acid aptamer of claim 1, comprising one to three said unnatural base(s).

3. The nucleic acid aptamer of claim 1 or 2, comprising the unnatural bases represented by said general formulas (I) and (II).

4. The nucleic acid aptamer of any one of claims 1 to 3, wherein the unnatural base is any base represented by the following formulas (X) to (XVII) and formulas (XXI) to (XXII),

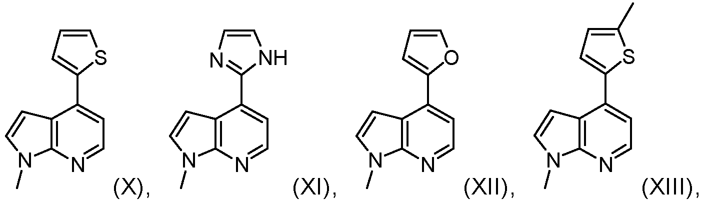

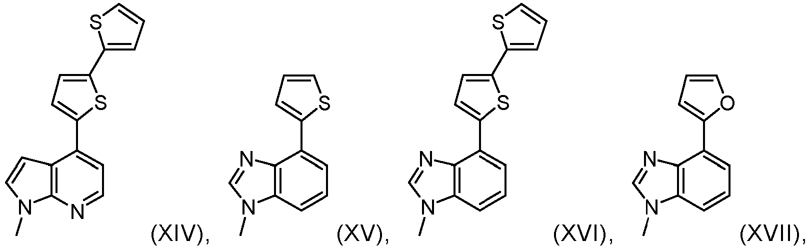

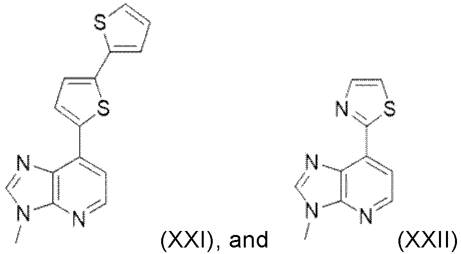

5. The nucleic acid aptamer of any one of claims 1 to 4, comprising an unnatural base represented by the following formula (XVIII),

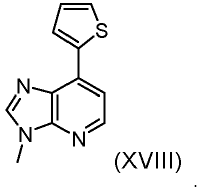

6. The nucleic acid aptamer of any one of claims 1 to 5 which is a DNA aptamer.

7. The nucleic acid aptamer of claim 1,
(1) comprising the base sequence of SEQ ID NO: 205, 206, or 187, or a sequence in which a pair of base sequences complementary to each other and capable of forming a stem structure are added to the 5' end side and 3' end side of the base sequence of SEQ ID NO: 205, 206, or 187, wherein the nucleic acid aptamer binds to von Willebrand factor (vWF protein),
(2) comprising the base sequence of SEQ ID NO: 207, 208, 188, 56, 59, 61, 65, 67, 189, or 213, or a sequence in which a pair of base sequences complementary to each other and capable of forming a stem structure are added to the 5' end side and 3' end side of the base sequence of SEQ ID NO: 207, 208, 188, 56, 59, 61, 65, 67, 189, or 213, wherein the nucleic acid aptamer binds to Interferon gamma (IFN-γ),
(3) comprising the base sequence of SEQ ID NO: 190, or a sequence in which a pair of base sequences complementary to each other and capable of forming a stem structure are added to the 5' end side and 3' end side of the base sequence of SEQ ID NO: 190, wherein the nucleic acid aptamer binds to Thrombin,
(4) comprising the base sequence of SEQ ID NO: 209 or 192, or a sequence in which a pair of base sequences complementary to each other and capable of forming a stem structure are added to the 5' end side and 3' end side of the base sequence of SEQ ID NO: 209 or 192, wherein the nucleic acid aptamer binds to vascular endothelial growth factor (VEGF),
(5) comprising the base sequence of SEQ ID NO: 194, or a sequence in which a pair of base sequences complementary to each other and capable of forming a stem structure are added to the 5' end side and 3' end side of the base sequence of SEQ ID NO: 194, wherein the nucleic acid aptamer binds to HMGB1 protein,
(6) comprising the base sequence of SEQ ID NO: 196, or a sequence in which a pair of base sequences complementary to each other and capable of forming a stem structure are added to the 5' end side and 3' end side of the base sequence of SEQ ID NO: 196, wherein the nucleic acid aptamer binds to Transferrin receptor 1,
(7) comprising the base sequence of SEQ ID NO: 210, 197, or 199, or a sequence in which a pair of base sequences complementary to each other and capable of forming a stem structure are added to the 5' end side and 3' end side of the base sequence of SEQ ID NO: 210, 197, or 199, wherein the nucleic acid aptamer binds to Dengue Virus NS1 Protein Serotype 1 (DEN1 protein),
(8) comprising the base sequence of SEQ ID NO: 201, or a sequence in which a pair of base sequences complementary to each other and capable of forming a stem structure are added to the 5' end side and 3' end side of the base sequence of SEQ ID NO: 201, wherein the nucleic acid aptamer binds to Dengue Virus NS1 Protein Serotype 2 (DEN2 protein),
(9) comprising the base sequence of SEQ ID NO: 39 or 202, or a sequence in which a pair of base sequences complementary to each other and capable of forming a stem structure are added to the 5' end side and 3' end side of the base sequence of SEQ ID NO: 39 or 202, wherein the nucleic acid aptamer binds to Dengue Virus NS1 Protein Serotype 3 (DEN3 protein), or
(10) comprising the base sequence of SEQ ID NO: 204, or a sequence in which a pair of base sequences complementary to each other and capable of forming a stem structure are added to the 5' end side and 3' end side of the base sequence of SEQ ID NO: 204, wherein the nucleic acid aptamer binds to Dengue Virus NS1 Protein Serotype 4 (DEN4 protein),
wherein at least one of the bases shown as n in said base sequence are unnatural base(s) represented by the general formula(s) (I) and/or (II).

8. The nucleic acid aptamer of claim 7, comprising one to three said unnatural bases as the bases shown as n in said base sequence.

9. The nucleic acid aptamer of claim 7 or 8, comprising the unnatural bases represented by said general formulas (I) and (II).

10. The nucleic acid aptamer of claim 7, comprising 7-(2-thienyl)-3H-imidazo[4,5-b]pyridine-3-yl as the base shown as n in said base sequence.

11. The nucleic acid aptamer of claim 7, wherein said unnatural base is any of the bases represented by the following formulas (X) to (XVII) and formulas (XXI) to (XXII),

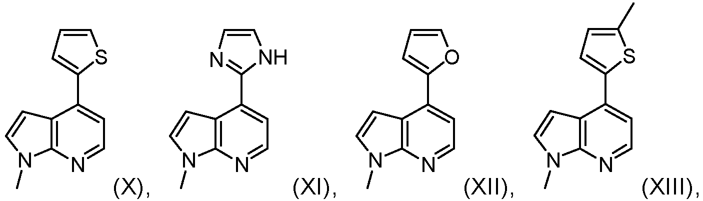

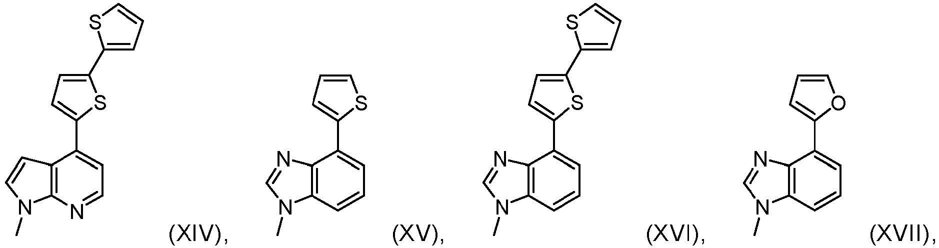

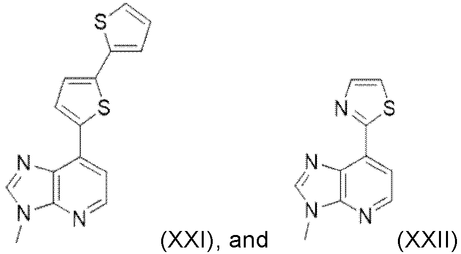

12. The nucleic acid aptamer of claim 7, comprising an unnatural base represented by the following formula (XVIII),

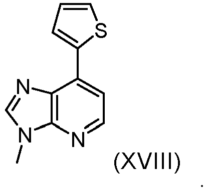

13. The nucleic acid aptamer of claim 7 which is selected from the group consisting of the following (1) to (10):
(1) a nucleic acid aptamer that binds to vWF protein selected from the group consisting of the following (1-a) to (1-d):
(1-a) a nucleic acid aptamer comprising the base sequence of SEQ ID NO: 1 or 3 wherein the bases shown as n at position 10 and position 31 in said base sequence are, respectively, (I) base Bs and base Bs, (II) base Ys and base Ys, (III) base Yo and base Yo, (IV) base Bs and base Ys, (V) base Bs and base Yo, or (VI) base Ys and base Bs;
(1-b) a nucleic acid aptamer comprising a base sequence in which one or multiple bases are deleted, substituted, or added in the base sequence of SEQ ID NO: 1 or 3 except position 10 and position 31, wherein the positions corresponding to the bases shown as n at position 10 and position 31 of SEQ ID NO: 1 or 3 in said base sequence are, respectively, (I) base Bs and base Bs, (II) base Ys and base Ys, (III) base Yo and base Yo, (IV) base Bs and base Ys, (V) base Bs and base Yo, or (VI) base Ys and base Bs;
(1-c) a nucleic acid aptamer comprising a 5' region, a central region, and a 3' region in this order in this order from 5' side, wherein said 5' region and 3' region comprise a pair of base sequences complementary to each other and capable of forming a stem structure, said central region comprises a base sequence of SEQ ID NO: 205 or 187, and the bases shown as n at position 3 and position 24 in said base sequence of SEQ ID NO: 205 or 187 are, respectively, (I) base Bs and base Bs, (II) base Ys and base Ys, (III) base Yo and base Yo, (IV) base Bs and base Ys, (V) base Bs and base Yo, or (VI) base Ys and base Bs;
(1-d) a nucleic acid aptamer comprising a 5' region, a central region, and a 3' region in this order in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 205 or 187 except position 3 and position 24, wherein the positions corresponding to the bases shown as n at position 3 and position 24 of SEQ ID NO: 205 or 187 in said base sequence in said central region are, respectively, (I) base Bs and base Bs, (II) base Ys and base Ys, (III) base Yo and base Yo, (IV) base Bs and base Ys, (V) base Bs and base Yo, or (VI) base Ys and base Bs;
(2) a nucleic acid aptamer that binds to IFNγ selected from the group consisting of the following (2-i-a) to (2-iii-d):
(2-i-a) a nucleic acid aptamer comprising the base sequence of SEQ ID NO: 15 or 17 wherein the bases shown as n at position 28 and position 39 in said base sequence are, respectively, (I) base Ys and base Ys, or (II) base Yo and base Yo;
(2-i-b) a nucleic acid aptamer comprising a base sequence in which one or multiple bases are deleted, substituted, or added in the base sequence of SEQ ID NO: 15 or 17 except position 28 and position 39, wherein the positions corresponding to the bases shown as n at position 28 and position 39 of SEQ ID NO: 15 or 17 in said base sequence are, respectively, (I) base Ys and base Ys, or (II) base Yo and base Yo;
(2-i-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 207 or 188, and the bases shown as n at position 22 and position 33 in said base sequence of SEQ ID NO: 207 or 188 are, respectively, (I) base Ys and base Ys, or (II) base Yo and base Yo;
(2-i-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 207 or 188 except position 22 and position 33, wherein the positions corresponding to the bases shown as n at position 22 and position 33 of SEQ ID NO: 207 or 188 in said base sequence in said central region are, respectively, (I) base Ys and base Ys, or (II) base Yo and base Yo;
(2-ii-a) a nucleic acid aptamer comprising the base sequence of SEQ ID NO: 62 wherein the bases shown as n at position 24 and position 37 in said base sequence are, respectively, (I) base Ys and base Ds, or (II) base Bs and base Ds;
(2-ii-b) a nucleic acid aptamer comprising a base sequence in which one or multiple bases are deleted, substituted, or added in the base sequence of SEQ ID NO: 62 except position 24 and position 37, wherein the positions corresponding to the bases shown as n at position 24 and position 37 of SEQ ID NO: 62 in said base sequence are, respectively, (I) base Ys and base Ds, or (II) base Bs and base Ds;
(2-ii-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 189, and the bases shown as n at position 15 and position 28 in said base sequence of SEQ ID NO: 189 are, respectively, (I) base Ys and base Ds, or (II) base Bs and base Ds;
(2-ii-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 289 except position 15 and position 28, wherein the positions corresponding to the bases shown as n at position 15 and position 28 of SEQ ID NO: 189 in said base sequence in said central region are, respectively, (I) base Ys and base Ds, or (II) base Bs and base Ds;
(2-iii-a) a nucleic acid aptamer comprising the base sequence of SEQ ID NO: 212 wherein the base shown as n at position 37 in said base sequence is base Ys or base Bs;
(2-iii-b) a nucleic acid aptamer comprising a base sequence in which one or multiple bases are deleted, substituted, or added in the base sequence of SEQ ID NO: 212 except position 37, wherein the position corresponding to the base shown as n at position 37 of SEQ ID NO: 212 in said base sequence is base Ys or base Bs;
(2-iii-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 213, and the base shown as n at position 28 in said base sequence of SEQ ID NO: 213 is base Ys or base Bs;
(2-iii-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 213 except position 28, wherein the position corresponding to the base shown as n at position 28 of SEQ ID NO: 213 in said base sequence in said central region is base Ys or base Bs;
(3) a nucleic acid aptamer that binds to Thrombin selected from the group consisting of the following (3-a) to (3-d):
(3-a) a nucleic acid aptamer comprising the base sequence of SEQ ID NO: 81 wherein the bases shown as n at position 16 and position 22 in said base sequence are, respectively, (I) base Bs and base Bs, (II) base Bs and base Ys, (III) base Ys and base Bs, or (IV) base Ys and base Ys;
(3-b) a nucleic acid aptamer comprising a base sequence in which one or multiple bases are deleted, substituted, or added in the base sequence of SEQ ID NO: 81 except position 16 and position 22, wherein the positions corresponding to the bases shown as n at position 16 and position 22 of SEQ ID NO: 81 in said base sequence are, respectively, (I) base Bs and base Bs, (II) base Bs and base Ys, (III) base Ys and base Bs, or (IV) base Ys and base Ys;
(3-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 190, and the bases shown as n at position 7 and position 13 in said base sequence of SEQ ID NO: 190 are, respectively, (I) base Bs and base Bs, (II) base Bs and base Ys, (III) base Ys and base Bs, or (IV) base Ys and base Ys;
(3-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 190 except position 7 and position 13, wherein the positions corresponding to the bases shown as n at position 7 and position 13 of SEQ ID NO: 190 in said base sequence in said central region are, respectively, (I) base Bs and base Bs, (II) base Bs and base Ys, (III) base Ys and base Bs, or (IV) base Ys and base Ys;
(4) a nucleic acid aptamer that binds to VEGF selected from the group consisting of the following (4-a) to (4-d):
(4-a) a nucleic acid aptamer comprising the base sequence of SEQ ID NO: 191 wherein the bases shown as n at position 25 and position 34 in said base sequence are, respectively, (I) base Ys and base Ys, (II) base Ys and base Bs, (III) base Bs and base Ys, (IV) base Bs and base Bs, or (V) base Yo and base Yo;
(4-b) a nucleic acid aptamer comprising a base sequence in which one or multiple bases are deleted, substituted, or added in the base sequence of SEQ ID NO: 191 except position 25 and position 34, wherein the positions corresponding to the bases shown as n at position 25 and position 34 of SEQ ID NO: 191 in said base sequence are, respectively, (I) base Ys and base Ys, (II) base Ys and base Bs, (III) base Bs and base Ys, (IV) base Bs and base Bs, or (V) base Yo and base Yo;
(4-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 192, and the bases shown as n at position 21 and position 30 in said base sequence of SEQ ID NO: 192 are, respectively, (I) base Ys and base Ys, (II) base Ys and base Bs, (III) base Bs and base Ys, (IV) base Bs and base Bs, or (V) base Yo and base Yo;
(4-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 192 except position 21 and position 30, wherein the positions corresponding to the bases shown as n at position 21 and position 30 of SEQ ID NO: 192 in said base sequence in said central region are, respectively, (I) base Ys and base Ys, (II) base Ys and base Bs, (III) base Bs and base Ys, (IV) base Bs and base Bs, or (V) base Yo and base Yo;
(5) a nucleic acid aptamer that binds to HMGB1 protein selected from the group consisting of the following (5-a) to (5-d):
(5-a) a nucleic acid aptamer comprising the base sequence of SEQ ID NO: 193 wherein the bases shown as n at position 22 and position 33 in said base sequence are, respectively, (I) base Bs and base Bs, (II) base Bs and base Ys, (III) base Ys and base Bs, or (IV) base Ys and base Ys;
(5-b) a nucleic acid aptamer comprising a base sequence in which one or multiple bases are deleted, substituted, or added in the base sequence of SEQ ID NO: 193 except position 22 and position 33, wherein the positions corresponding to the bases shown as n at position 22 and position 33 of SEQ ID NO: 193 in said base sequence are, respectively, (I) base Bs and base Bs, (II) base Bs and base Ys, (III) base Ys and base Bs, or (IV) base Ys and base Ys;
(5-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 194, and the bases shown as n at position 12 and position 23 in said base sequence of SEQ ID NO: 194 are, respectively, (I) base Bs and base Bs, (II) base Bs and base Ys, (III) base Ys and base Bs, or (IV) base Ys and base Ys;
(5-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 194 except position 12 and position 23, wherein the positions corresponding to the bases shown as n at position 12 and position 23 of SEQ ID NO: 194 in said base sequence in said central region are, respectively, (I) base Bs and base Bs, (II) base Bs and base Ys, (III) base Ys and base Bs, or (IV) base Ys and base Ys;
(6) a nucleic acid aptamer that binds to Transferrin receptor 1 selected from the group consisting of the following (6-a) to (6-d):
(6-a) a nucleic acid aptamer comprising the base sequence of SEQ ID NO: 195 wherein the base shown as n at position 27 in said base sequence is base Bs;
(6-b) a nucleic acid aptamer comprising a base sequence in which one or multiple bases are deleted, substituted, or added in the base sequence of SEQ ID NO: 195 except position 27, wherein the positions corresponding to the base shown as n at position 27 of SEQ ID NO: 193 in said base sequence is base Bs;
(6-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 195, and the base shown as n at position 20 in said base sequence of SEQ ID NO: 195 is base Bs;
(6-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 195 except position 20, wherein the position corresponding to the base shown as n at position 20 of SEQ ID NO: 195 in said base sequence in said central region is base Bs;
(7) a nucleic acid aptamer that binds to DEN1 selected from the group consisting of the following (7-i-a) to (7-ii-d):
(7-i-a) a nucleic acid aptamer comprising the base sequence of SEQ ID NO: 27 or 29 wherein the bases shown as n at position 19 and position 25 in said base sequence are, respectively, (I) base Yss and base Ds, (II) base Ds and base Dt, (III) base Ys and base Bs, (IV) base Yo and base Bs, or (V) base Ysm and base Bs;
(7-i-b) a nucleic acid aptamer comprising a base sequence in which one or multiple bases are deleted, substituted, or added in the base sequence of SEQ ID NO: 27 or 29 except position 19 and position 25, wherein the positions corresponding to the bases shown as n at position 19 and position 25 of SEQ ID NO: 27 or 29 in said base sequence are, respectively, (I) base Yss and base Ds, (II) base Ds and base Dt, (III) base Ys and base Bs, (IV) base Yo and base Bs, or (V) base Ysm and base Bs;
(7-i-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 210 or 197, and the bases shown as n at position 9 and position 15 in said base sequence of SEQ ID NO: 210 or 197 are, respectively, (I) base Yss and base Ds, (II) base Ds and base Dt, (III) base Ys and base Bs, (IV) base Yo and base Bs, or (V) base Ysm and base Bs;
(7-i-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 210 or 197 except position 9 and position 15, wherein the positions corresponding to the bases shown as n at position 9 and position 15 of SEQ ID NO: 210 or 197 in said base sequence in said central region are, respectively, (I) base Yss and base Ds, (II) base Ds and base Dt, (III) base Ys and base Bs, (IV) base Yo and base Bs, or (V) base Ysm and base Bs;
(7-ii-a) a nucleic acid aptamer comprising the base sequence of SEQ ID NO: 198 wherein the bases shown as n at position 14 and position 27 in said base sequence are, respectively, (I) base Ys and base Ys, (II) base Ys and base Bs, (III) base Bs and base Ys, or (IV) base Bs and base Bs;
(7-ii-b) a nucleic acid aptamer comprising a base sequence in which one or multiple bases are deleted, substituted, or added in the base sequence of SEQ ID NO: 198 except position 14 and position 27, wherein the positions corresponding to the bases shown as n at position 14 and position 27 of SEQ ID NO: 198 in said base sequence are, respectively, (I) base Ys and base Ys, (II) base Ys and base Bs, (III) base Bs and base Ys, or (IV) base Bs and base Bs;
(7-ii-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 199, and the bases shown as n at position 4 and position 17 in said base sequence of SEQ ID NO: 199 are, respectively, (I) base Ys and base Ys, (II) base Ys and base Bs, (III) base Bs and base Ys, or (IV) base Bs and base Bs;
(7-ii-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 199 except position 4 and position 17, wherein the positions corresponding to the bases shown as n at position 4 and position 17 of SEQ ID NO: 199 in said base sequence in said central region are, respectively, (I) base Ys and base Ys, (II) base Ys and base Bs, (III) base Bs and base Ys, or (IV) base Bs and base Bs;
(8) a nucleic acid aptamer that binds to DEN2 protein selected from the group consisting of the following (8-a) to (8-d):
(8-a) a nucleic acid aptamer comprising the base sequence of SEQ ID NO: 200 wherein the base shown as n at position 13 in said base sequence is base Bs;
(8-b) a nucleic acid aptamer comprising a base sequence in which one or multiple bases are deleted, substituted, or added in the base sequence of SEQ ID NO: 200 except position 13, wherein the positions corresponding to the base shown as n at position 13 of SEQ ID NO: 200 in said base sequence is base Ys or base Bs;
(8-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 201, and the base shown as n at position 3 in said base sequence of SEQ ID NO: 201 is base Ys or base Bs;
(8-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 201 except position 3, wherein the position corresponding to the base shown as n at position 3 of SEQ ID NO: 201 in said base sequence in said central region is base Ys or base Bs;
(9) a nucleic acid aptamer that binds to DEN3 protein selected from the group consisting of the following (9-a) to (9-d):
(9-a) a nucleic acid aptamer comprising the base sequence of SEQ ID NO: 38 or 40 wherein the bases shown as n at position 15 and position 23 in said base sequence are, respectively, (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(9-b) a nucleic acid aptamer comprising a base sequence in which one or multiple bases are deleted, substituted, or added in the base sequence of SEQ ID NO: 38 or 40 except position 15 and position 23, wherein the positions corresponding to the bases shown as n at position 15 and position 23 of SEQ ID NO: 38 or 40 in said base sequence are, respectively, (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(9-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 38 or 40, and the bases shown as n at position 5 and position 13 in said base sequence of SEQ ID NO: 38 or 40 are, respectively, (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(9-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 38 or 40 except position 5 and position 13, wherein the positions corresponding to the bases shown as n at position 5 and position 13 of SEQ ID NO: 38 or 40 in said base sequence in said central region are, respectively, (I) base Dss and base Dss, (II) base Ys and base Dss, (III) base Dss and base Ys, (IV) base Ysm and base Ysm, (V) base Yss and base Ys, (VI) base Yss and base Yo, or (VII) base Yss and base Ysm;
(10) a nucleic acid aptamer that binds to DEN3 protein selected from the group consisting of the following (10-a) to (10-d):
(10-a) a nucleic acid aptamer comprising the base sequence of SEQ ID NO: 203 wherein the bases shown as n at position 18 and position 28 in said base sequence are, respectively, (I) base Ys and base Ys, (II) base Ys and base Bs, (III) base Bs and base Ys, or (IV) base Bs and base Bs;
(10-b) a nucleic acid aptamer comprising a base sequence in which one or multiple bases are deleted, substituted, or added in the base sequence of SEQ ID NO: 203 except 18 and position 28, wherein the positions corresponding to the bases shown as n at position 18 and position 28 of SEQ ID NO: 203 in said base sequence are, respectively, (I) base Ys and base Ys, (II) base Ys and base Bs, (III) base Bs and base Ys, or (IV) base Bs and base Bs;
(10-c) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence of SEQ ID NO: 203, and the bases shown as n at position 11 and position 21 in said base sequence of SEQ ID NO: 203 are, respectively, (I) base Ys and base Ys, (II) base Ys and base Bs, (III) base Bs and base Ys, or (IV) base Bs and base Bs;
(10-d) a nucleic acid aptamer comprising a 5' region, a central region, and a 3' region in this order from 5' side, wherein said 5' region and 3' region comprise a pair of base sequences complementary to each other and capable of forming a stem structure, said central region comprises a base sequence in which one or multiple bases are deleted, substituted, or added in the base sequence of SEQ ID NO: 203 except position 11 and position 21, wherein the positions corresponding to the bases shown as n at position 11 and position 21 of SEQ ID NO: 203 in said base sequence in said central region are, respectively, (I) base Ys and base Ys, (II) base Ys and base Bs, (III) base Bs and base Ys, or (IV) base Bs and base Bs,
wherein said base Ys is an unnatural base represented by the following formula (X), said base Yo is an unnatural base represented by the following formula (XII), said base Ysm is an unnatural base represented by the following formula (XIII), said base Yss is an unnatural base represented by the following formula (XIV), said base Bs is an unnatural base represented by the following formula (XV), said base Ds is an unnatural base represented by the following formula (XVIII), said base Dss is an unnatural base represented by the following formula (XXI), and said base Dt is an unnatural base represented by the following formula (XXII),

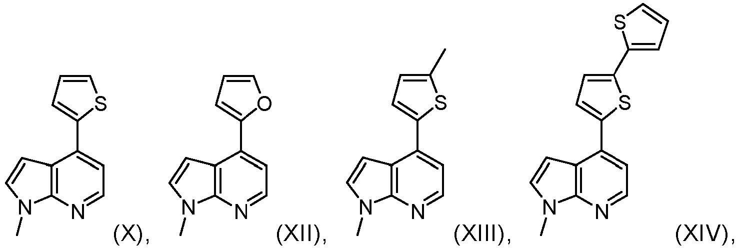

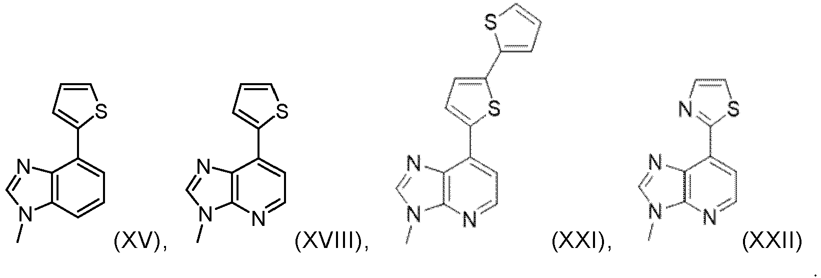

14. The nucleic acid aptamer of claim 7 which is a DNA aptamer.

15. A pharmaceutical composition comprising the nucleic acid aptamer of claim 7.

16. The pharmaceutical composition of claim 15, comprising the nucleic acid aptamer of said (1) as an active ingredient, wherein said pharmaceutical composition is for treating and/or preventing a disease selected from the group consisting of thrombosis, thrombotic thrombocytopenic purpura, intracranial embolism, brain embolism, carotid artery stenosis, thrombotic microangiopathy, and acute myocardial infarction.

17. The pharmaceutical composition of claim 15, comprising the nucleic acid aptamer of said (2) as an active ingredient, wherein said pharmaceutical composition is for treating and/or preventing autoimmune disease or inflammatory disorder.

18. The pharmaceutical composition of claim 15, comprising the nucleic acid aptamer of said (3) as an active ingredient, wherein said pharmaceutical composition is for treating and/or preventing thrombotic disorder.

19. The pharmaceutical composition of claim 15, comprising the nucleic acid aptamer of said (4) as an active ingredient, wherein said pharmaceutical composition is for treating and/or preventing age-related macular degeneration, diabetic retinopathy, rheumatoid arthritis, or cancer.

20. The pharmaceutical composition of claim 15, comprising the nucleic acid aptamer of said (5) as an active ingredient, wherein said pharmaceutical composition is for treating and/or preventing a disease selected from the group consisting of autoimmune disease, cardiovascular disease, rheumatoid arthritis, inflammatory bowel disease, sepsis, cancer, lupus, Sjogren's syndrome, myocardial infarction, arteriosclerosis, stroke, cerebral infarction, cerebral edema, cerebral vasospasm, traumatic brain injury, atherosclerosis, neuropathic pain, arthritis, acute pulmonary trauma, cerebral ischemia, renal ischemia, and hepatic ischemia.

21. The pharmaceutical composition of claim 15, comprising the nucleic acid aptamer of said (6) as an active ingredient, wherein said pharmaceutical composition is for treating and/or preventing iron deficiency anemia, iron overload disorder, cancer, neurodegenerative disease, or infectious disease.

22. The pharmaceutical composition of claim 15, comprising any one or more nucleic acid aptamers of said (7) to (10) as active ingredient(s), wherein said pharmaceutical composition is for treating and/or preventing dengue virus infectious disease.

23. An unnatural base represented by any of the following formulas (XI) to (XIII) and formulas (XV) to (XVII),

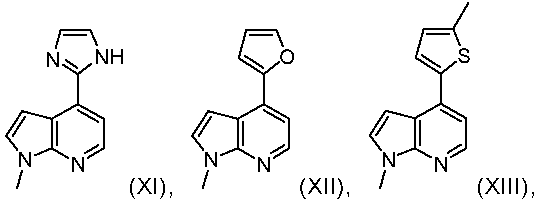

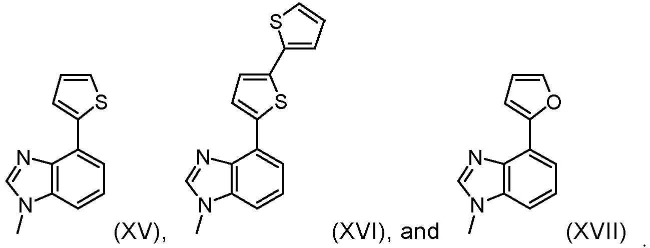

24. A nucleoside comprising the unnatural base of claim 23.

25. A nucleotide comprising the unnatural base of claim 23.

26. The nucleotide of claim 25 which is an amidite.
